# EUROPÄISCHE PATENTSCHRIFT

(11) **EP 3 083 570 B1**
(45) Veröffentlichungstag und Bekanntmachung des Hinweises auf die Patenterteilung: **24.10.2018**
(21) Anmeldenummer: 14811902.7
(22) Anmeldetag: 12.12.2014
(51) Int. Cl.: C07D 239/36, A01N 43/54

(54) **SECHSGLIEDRIGE C-N-VERKNÜPFTE ARYLSULFID- UND ARYLSULFOXID- DERIVATE ALS SCHÄDLINGSBEKÄMPFUNGSMITTEL**
SIX-MEMBERED C-N-LINKED ARYL SULFIDE DERIVATIVES AND ARYL SULFOXIDE DERIVATIVES AS PEST CONTROL AGENTS
DÉRIVÉS DE SULFURE D'ARYLE ET D'OXYDE ARYLSULFONIQUE À LIAISON C-N, À SIX CHAÎNONS, UTILISÉS COMME PESTICIDES

(30) Priorität: 16.12.2013 EP 13197460
(43) Veröffentlichungstag der Anmeldung: 26.10.2016
(73) Patentinhaber: Bayer CropScience Aktiengesellschaft, 40789 Monheim am Rhein (DE)
(72) Erfinder: KÖHLER, Adeline, 40764 Langenfeld (DE); CEREZO-GALVEZ, Silvia, 40764 Langenfeld (DE); ALIG, Bernd, 53639 Königswinter (DE); FISCHER, Reiner, 40789 Monheim (DE); HAHN, Julia Johanna, 40589 Düsseldorf (DE); ILG, Kerstin, 50670 Köln (DE); PORTZ, Daniela, 52391 Vettweiß (DE); MALSAM, Olga, 51503 Rösrath (DE); LÖSEL, Peter, 51371 Leverkusen (DE); WILCKE, David, 40219 Düsseldorf (DE)
(74) Vertreter: BIP Patents
(86) Internationale Anmeldenummer: PCT/EP2014/077576
(87) Internationale Veröffentlichungsnummer: WO 2015/091267

(56) Entgegenhaltungen:
- WO-A1-2010/100189
- US-A1- 2003 069 242

## Beschreibung

Die vorliegende Anmeldung betrifft neue heterocyclische Verbindungen, Verfahren zu Ihrer Herstellung und ihre Verwendung zur Bekämpfung von tierischen Schädlingen, zu denen Arthropoden und insbesondere Insekten zählen.

Arylsulfid- und Arylsulfoxid- Derivate sowie deren insektizide und akarizide Wirkung sind beispielsweise bereits aus WO 1999/055668 A1 bekannt. Arylchinazolinone sowie deren insektizide und akarizide Wirkung sind bereits aus WO 2010/100189 A1 bekannt.

Pflanzenschutzmittel, zu denen auch Schädlingsbekämpfungsmittel gehören, müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert oder verbessert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I) 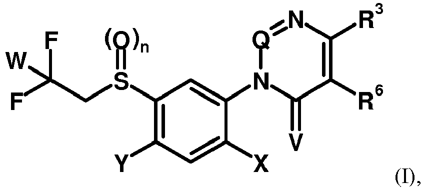 worin (Ausgestaltung 2-1)V für Sauerstoff steht;
- Q: für C-R⁵ oder Stickstoff steht, wobei
- R⁵: für Wasserstoff, Halogen, Amino, Cyano, Nitro, (C₁-C₆)Alkyl, (C₁-C₆)Halogenalkyl, (C₁-C₆)Alkylsulfanyl, (C₁-C₆)Alkylsulfinyl, (C₁-C₆)Alkylsulfonyl, (C₁-C₆)Halogenalkylsulfanyl, (C₁-C₆)Halogenalkylsulfinyl, (C₁-C₆)Halogenalkylsulfonyl, (C₃-C₆)Cycloalkyl oder (C₁-C₆)Alkoxy steht;
- R³ und R⁶: unabhängig voneinander
für Wasserstoff, Halogen, Cyano oder Nitro stehen; oder
für (C₁-C₆)Alkyl, (C₁-C₆)Halogenalkyl, (C₂-C₆)Alkenyl, (C₂-C₆)Alkinyl, (C₁-C₆)Alkoxy, (C₁-C₆)Halogenalkoxy, (C₁-C₆)Halogenalkylsulfanyl, (C₁-C₆)Halogenalkylsulfinyl, (C₁-C₆)Halogenalkylsulfonyl, (C₁-C₆)Alkylsulfanyl, (C₁-C₆)Alkylsulfinyl, (C₁-C₆)Alkylsulfonyl, (C₁-C₅)Alkylcarbonyl, (C₁-C₅)Halogenalkylcarbonyl oder (C₂-C₆)Alkoxycarbonyl stehen; oder
für (C₃-C₆)Cycloalkyl, (C₃-C₆)Cycloalkyl(C₁-C₃)alkyl oder (C₃-C₆)Cycloalkenyl stehen, wobei die vorgenannten Reste gegebenenfalls durch Halogen, Alkyl, Cyano, Nitro, Alkoxy, Halogenalkyl oder Halogenalkoxy substituiert sein können; oder
für Phenyl, Heteroaryl, Phenyl(C₁-C₃)alkyl oder Heteroaryl(C₁-C₃)alkyl stehen, wobei die vorgenannten Reste gegebenenfalls durch Halogen, Alkyl, Cyano, Nitro, Alkoxy, Halogenalkyl oder Halogenalkoxy substituiert sein können; oder
für NR'R" stehen,
wobei R' und R" unabhängig voneinander für Wasserstoff, Cyano, (C₁-C₄)Alkyl, (C₁-C₄)Halogenalkyl, (C₁-C₄)Cyanoalkyl, (C₁-C₄)Hydroxyalkyl, (C₁-C₄)Alkoxy(C₁-C₄)alkyl, (C₁-C₄)Alkylsulfanyl(C₁-C₄)alkyl, (C₁-C₄)Alkylsulfinyl(C₁-C₄)alkyl, (C₁-C₄)Alkylsulfonyl(C₁-C₄)alkyl, (C₃-C₄)Alkenyl, (C₃-C₄)Halogenalkenyl, (C₂-C₄)Cyanoalkenyl, (C₃-C₄)Alkinyl, (C₃-C₄)Halogenalkinyl, (C₃-C₄)Cyanoalkinyl, (C₁-C₄)Alkylcarbonyl oder (C₁-C₄)Alkoxycarbonyl stehen;
oder
wobei R' und R" gemeinsam mit dem N-Atom, an das sie gebunden sind, einen gegebenenfalls durch Halogen, Alkyl, Cyano, Nitro, Alkoxy, Halogenalkyl oder Halogenalkoxy substituierten und gegebenenfalls durch ein oder mehrere Heteroatome, welche unabhängig ausgewählt werden aus der Gruppe bestehend aus O, S und N mit der Maßgabe, dass zwei Sauerstoffatome nicht unmittelbar benachbart zueinander stehen, unterbrochenen gesättigten oder ungesättigten drei- bis achtgliedrigen Ring bilden können;
- W: für Wasserstoff oder Halogen steht;
- n: für die Zahl 0 oder 1 steht;
- Y: für Wasserstoff, Halogen, (C₁-C₄)Alkyl, Trifluoromethyl, (C₂-C₄)Halogenalkyl, (C₁-C₄)Alkoxy, (C₁-C₄)Halogenalkoxy oder Amino steht; oder
für NR'''R'''' steht,
wobei R''' und R'''' unabhängig voneinander für Wasserstoff, (C₁-C₄)Alkyl, oder (C₂-C₄)Halogenalkyl stehen;
- X: für Wasserstoff, Halogen, Cyano, (C₁-C₄)Alkyl, (C₁-C₄)Halogenalkyl oder (C₁-C₄)Alkoxy steht.

Weiterhin wurde gefunden, dass die neuen Verbindungen der Formel (I) eine gute Wirksamkeit als Schädlingsbekämpfungsmittel, beispielsweise gegen Arthropoden und insbesondere Insekten, Nematoden und Akariden, darüber hinaus in der Regel insbesondere gegenüber Kulturpflanzen sehr gut pflanzenverträglich sind und/oder über günstige toxikologische und/oder umweltrelevante Eigenschaften verfügen.

In einer weiteren Ausgestaltung (Ausgestaltung 2-2) sind die bevorzugten Substituenten bzw. Bereiche der in den Verbindungen der Formel (I) aufgeführten Reste wie folgt:
- V: steht für Sauerstoff;
- Q: steht für C-R⁵ oder Stickstoff, wobei
- R⁵: für Wasserstoff, Halogen, Amino, Cyano, Nitro, (C₁-C₆)Alkyl, (C₁-C₆)Halogenalkyl, (C₁-C₆)Alkylsulfanyl, (C₁-C₆)Alkylsulfinyl, (C₁-C₆)Alkylsulfonyl, (C₁-C₆)Halogenalkylsulfanyl, (C₁-C₆)Halogenalkylsulfinyl, (C₁-C₆)Halogenalkylsulfonyl oder (C₁-C₆)Alkoxy steht;
- R³ und R⁶: stehen unabhängig voneinander
für Wasserstoff, Halogen, Cyano oder Nitro; oder
für (C₁-C₆)Alkyl, (C₁-C₆)Halogenalkyl, (C₂-C₆)Alkenyl, (C₂-C₆)Alkinyl, (C₁-C₆)Alkoxy, (C₁-C₆)Halogenalkoxy, (C₁-C₆)Halogenalkylsulfanyl, (C₁-C₆)Halogenalkylsulfinyl, (C₁-C₆)Halogenalkylsulfonyl, (C₁-C₆)Alkylsulfanyl, (C₁-C₆)Alkylsulfinyl, (C₁-C₆)Alkylsulfonyl, (C₁-C₅)Halogenalkylcarbonyl oder (C₂-C₆)Alkoxycarbonyl; oder für NR'R",
wobei R' und R" unabhängig voneinander für Wasserstoff, Cyano, (C₁-C₄)Alkyl, (C₁-C₄)Halogenalkyl, (C₁-C₄)Cyanoalkyl, (C₁-C₄)Hydroxyalkyl, (C₁-C₄)Alkoxy(C₁-C₄)alkyl, (C₁-C₄)Alkylsulfanyl(C₁-C₄)alkyl, (C₁-C₄)Alkylsulfinyl(C₁-C₄)alkyl, (C₁-C₄)Alkylsulfonyl(C₁-C₄)alkyl, (C₃-C₄)Alkenyl, (C₃-C₄)Halogenalkenyl, (C₂-C₄)Cyanoalkenyl, (C₃-C₄)Alkinyl, (C₃-C₄)Halogenalkinyl, (C₃-C₄)Cyanoalkinyl, (C₁-C₄)Alkylcarbonyl oder (C₁-C₄)Alkoxycarbonyl stehen;
- W: steht für Wasserstoff oder Halogen;
- n: steht für die Zahl 0 oder 1;
- Y: steht für Wasserstoff, Halogen, (C₁-C₄)Alkyl, Trifluoromethyl, (C₂-C₄)Halogenalkyl, (C₁-C₄)Alkoxy, (C₁-C₄)Halogenalkoxy oder Amino; oder
für NR'''R'''',
wobei R''' und R'''' unabhängig voneinander für Wasserstoff, (C₁-C₄)Alkyl, oder (C₂-C₄)Halogenalkyl stehen;
- X: steht für Wasserstoff, Halogen, Cyano, (C₁-C₄)Alkyl, (C₁-C₄)Halogenalkyl oder (C₁-C₄)Alkoxy.

In einer weiteren Ausgestaltung (Ausgestaltung 2-3) sind die bevorzugten Substituenten bzw. Bereiche der in den Verbindungen der Formel (I) aufgeführten Reste wie folgt:
- V: steht für Sauerstoff;
- Q: steht für C-R⁵ oder Stickstoff, wobei
- R⁵: steht für Wasserstoff, Halogen, Amino, Cyano, Nitro, (C₁-C₆)Alkyl, (C₁-C₆)Halogenalkyl, (C₁-C₆)Alkylsulfanyl, (C₁-C₆)Alkylsulfinyl, (C₁-C₆)Alkylsulfonyl, (C₁-C₆)Halogenalkylsulfanyl, (C₁-C₆)Halogenalkylsulfinyl, (C₁-C₆)Halogenalkylsulfonyl, (C₃-C₆)Cycloalkyl oder (C₁-C₆)Alkoxy;
- R³ und R⁶: stehen unabhängig voneinander
für (C₁-C₅)Alkylcarbonyl; oder
für (C₃-C₆)Cycloalkyl, (C₃-C₆)Cycloalkyl(C₁-C₃)alkyl oder (C₃-C₆)Cycloalkenyl, wobei die vorgenannten Reste gegebenenfalls durch Halogen, Alkyl, Cyano, Nitro, Alkoxy, Halogenalkyl oder Halogenalkoxy substituiert sein können; oder
für Phenyl, Heteroaryl, Phenyl(C₁-C₃)alkyl oder Heteroaryl(C₁-C₃)alkyl, wobei die vorgenannten Reste gegebenenfalls durch Halogen, Alkyl, Cyano, Nitro, Alkoxy, Halogenalkyl oder Halogenalkoxy substituiert sein können; oder
für NR'R",
wobei R' und R" gemeinsam mit dem N-Atom, an das sie gebunden sind, einen gegebenenfalls durch Halogen, Alkyl, Cyano, Nitro, Alkoxy, Halogenalkyl oder Halogenalkoxy substituierten und gegebenenfalls durch ein oder mehrere Heteroatome, welche unabhängig ausgewählt werden aus der Gruppe bestehend aus O, S und N mit der Maßgabe, dass zwei Sauerstoffatome nicht unmittelbar benachbart zueinander stehen, unterbrochenen gesättigten oder ungesättigten drei- bis achtgliedrigen Ring bilden können;
- W: steht für Wasserstoff oder Halogen;
- n: steht für die Zahl 0 oder 1;
- Y: steht für Wasserstoff, Halogen, (C₁-C₄)Alkyl, Trifluoromethyl, (C₂-C₄)Halogenalkyl, (C₁-C₄)Alkoxy, (C₁-C₄)Halogenalkoxy oder Amino; oder
für NR'''R'''',
wobei R''' und R'''' unabhängig voneinander für Wasserstoff, (C₁-C₄)Alkyl, oder (C₂-C₄)Halogenalkyl stehen;
- X: steht für Wasserstoff, Halogen, Cyano, (C₁-C₄)Alkyl, (C₁-C₄)Halogenalkyl oder (C₁-C₄)Alkoxy.

Weiter bevorzugte Substituenten bzw. Bereiche der in den Verbindungen der Formel (I) aufgeführten Reste werden im Folgenden erläutert (Ausgestaltung 3-1).
- V: steht für Sauerstoff;
- Q: steht für C-R⁵ oder Stickstoff, wobei
- R⁵: für Wasserstoff, Halogen, Amino, Cyano, Nitro, (C₁-C₄)Alkyl, (C₁-C₄)Halogenalkyl, (C₃-C₆)Cycloalkyl oder (C₁-C₄)Alkoxy steht;
- R³ und R⁶: stehen unabhängig voneinander
für Wasserstoff oder Halogen; oder
für (C₁-C₄)Alkyl, (C₁-C₄)Halogenalkyl, (C₁-C₄)Alkoxy, (C₁-C₄)Halogenalkoxy; oder
für (C₃-C₆)Cycloalkyl oder (C₃-C₆)Cycloalkyl(C₁-C₂)alkyl stehen, wobei alle vorgenannten Reste gegebenenfalls durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituiert sein können; oder
für Phenyl oder Phenyl(C₁-C₃)alkyl stehen, wobei die vorgenannten Reste gegebenenfalls einfach bis zweifach durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituiert sein können; oder
für Heteroaryl ausgewählt aus der Gruppe bestehend aus Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Indolyl, Isoindolyl und Indazolyl, wobei die vorgenannten Reste gegebenenfalls einfach bis zweifach durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituiert sein können; oder
für NR'R",
wobei R' und R" unabhängig voneinander für Wasserstoff, (C₁-C₄)Alkyl, (C₁-C₄)Halogenalkyl, (C₁-C₄)Cyanoalkyl, (C₁-C₄)Alkoxy(C₁-C₄)alkyl, (C₁-C₄)Alkylsulfanyl(C₁-C₄)alkyl, (C₁-C₄)Alkylsulfinyl(C₁-C₄)alkyl, (C₁-C₄)Alkylsulfonyl(C₁-C₄)alkyl, (C₁-C₄)Alkylcarbonyl oder (C₁-C₄)Alkoxycarbonyl stehen;
oder
wobei R' und R" gemeinsam mit dem N-Atom, an das sie gebunden sind, einen gegebenenfalls einfach bis zweifach durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituierten und gegebenenfalls durch ein Heteroatom, welches ausgewählt ist aus der Gruppe bestehend aus O, S und N, unterbrochenen gesättigten vier- bis sechsgliedrigen Ring bilden können;
- W: steht für Wasserstoff oder Fluor;
- n: steht für die Zahl 0 oder 1;
- Y: steht für Wasserstoff, Fluor, Chlor, Brom, Methyl, Ethyl, Trifluoromethyl, Methoxy, Ethoxy, Difluoromethoxy, Trifluoromethoxy, Amino, Methylamino oder Dimethylamino;
- X: steht für Wasserstoff, Fluor, Chlor, Brom, Cyano, Methyl, Ethyl, Trifluoromethyl, Methoxy oder Ethoxy.

In einer weiteren Ausgestaltung (Ausgestaltung 3-2) sind die weiter bevorzugten Substituenten bzw. Bereiche der in den Verbindungen der Formel (I) aufgeführten Reste wie folgt:
- V: steht für Sauerstoff;
- Q: steht für C-R⁵ oder Stickstoff, wobei
- R⁵: für Wasserstoff, Halogen, Amino, Cyano, Nitro, (C₁-C₄)Alkyl, (C₁-C₄)Halogenalkyl, (C₃-C₆)Cycloalkyl oder (C₁-C₄)Alkoxy steht;
- R³ und R⁶: stehen unabhängig voneinander
für Wasserstoff oder Halogen; oder
für (C₁-C₄)Alkyl, (C₁-C₄)Halogenalkyl, (C₁-C₄)Alkoxy, (C₁-C₄)Halogenalkoxy; oder für NR'R",
wobei R' und R" unabhängig voneinander für Wasserstoff, (C₁-C₄)Alkyl, (C₁-C₄)Halogenalkyl, (C₁-C₄)Cyanoalkyl, (C₁-C₄)Alkoxy(C₁-C₄)alkyl, (C₁-C₄)Alkylsulfanyl(C₁-C₄)alkyl, (C₁-C₄)Alkylsulfinyl(C₁-C₄)alkyl, (C₁-C₄)Alkylsulfonyl(C₁-C₄)alkyl, (C₁-C₄) Alkylcarbonyl oder (C₁-C₄)Alkoxycarbonyl stehen;
- W: steht für Wasserstoff oder Fluor;
- n: steht für die Zahl 0 oder 1;
- Y: steht für Wasserstoff, Fluor, Chlor, Brom, Methyl, Ethyl, Trifluoromethyl, Methoxy, Ethoxy, Difluoromethoxy, Trifluoromethoxy, Amino, Methylamino oder Dimethylamino;
- X: steht für Wasserstoff, Fluor, Chlor, Brom, Cyano, Methyl, Ethyl, Trifluoromethyl, Methoxy oder Ethoxy.

In einer weiteren Ausgestaltung (Ausgestaltung 3-3) sind die weiter bevorzugten Substituenten bzw. Bereiche der in den Verbindungen der Formel (I) aufgeführten Reste wie folgt:
- V: steht für Sauerstoff;
- Q: steht für C-R⁵ oder Stickstoff, wobei
- R⁵: für Wasserstoff, Halogen, Amino, Cyano, Nitro, (C₁-C₄)Alkyl, (C₁-C₄)Halogenalkyl, (C₃-C₆)Cycloalkyl oder (C₁-C₄)Alkoxy steht;
- R³ und R⁶: stehen unabhängig voneinander
für (C₃-C₆)Cycloalkyl oder (C₃-C₆)Cycloalkyl(C₁-C₂)alkyl stehen, wobei die vorgenannten Reste gegebenenfalls durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituiert sein können; oder
für Phenyl oder Phenyl(C₁-C₃)alkyl stehen, wobei die vorgenannten Reste gegebenenfalls einfach bis zweifach durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituiert sein können; oder
für Heteroaryl ausgewählt aus der Gruppe bestehend aus Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Indolyl, Isoindolyl und Indazolyl, wobei die vorgenannten Reste gegebenenfalls einfach bis zweifach durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituiert sein können; oder
für NR'R",
wobei R' und R" gemeinsam mit dem N-Atom, an das sie gebunden sind, einen gegebenenfalls einfach bis zweifach durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituierten und gegebenenfalls durch ein Heteroatom, welches ausgewählt ist aus der Gruppe bestehend aus O, S und N, unterbrochenen gesättigten vier- bis sechsgliedrigen Ring bilden können;
- W: steht für Wasserstoff oder Fluor;
- n: steht für die Zahl 0 oder 1;
- Y: steht für Wasserstoff, Fluor, Chlor, Brom, Methyl, Ethyl, Trifluoromethyl, Methoxy, Ethoxy, Difluoromethoxy, Trifluoromethoxy, Amino, Methylamino oder Dimethylamino;
- X: steht für Wasserstoff, Fluor, Chlor, Brom, Cyano, Methyl, Ethyl, Trifluoromethyl, Methoxy oder Ethoxy.

Besonders bevorzugte Substituenten bzw. Bereiche der in den Verbindungen der Formel (I) aufgeführten Reste werden im Folgenden erläutert (Ausgestaltung 4-1).
- V: steht für Sauerstoff;
- Q: steht für C-R⁵, wobei
- R⁵: für Wasserstoff, Methyl, Ethyl, Methoxy, Cyclopropyl oder Trifluoromethyl steht;
- R³ und R⁶: stehen unabhängig voneinander für Wasserstoff, Methyl, Ethyl, Trifluoromethyl, Difluoromethyl, Difluorchlormethyl, Cyclopropyl oder Phenyl;
- W: steht für Wasserstoff oder Fluor;
- n: steht für die Zahl 0 oder 1;
- Y: steht für Fluor, Chlor, Brom, Methyl, Trifluoromethyl oder Methoxy;
- X: steht für Wasserstoff, Chlor, Fluor oder Methyl;
insbesondere wobei X und Y für folgende Kombinationen (Y,X) stehen: (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F).

In einer weiteren Ausgestaltung (Ausgestaltung 4-2) sind die besonders bevorzugten Substituenten bzw. Bereiche der in den Verbindungen der Formel (I) aufgeführten Reste wie folgt:
- V: steht für Sauerstoff;
- Q: steht für C-R⁵, wobei
- R⁵: für Wasserstoff, Methyl, Ethyl oder Trifluoromethyl steht;
- R³ und R⁶: stehen unabhängig voneinander für Wasserstoff, Methyl, Ethyl, Trifluoromethyl, Difluoromethyl;
- W: steht für Wasserstoff oder Fluor;
- n: steht für die Zahl 0 oder 1;
- Y: steht für Fluor, Chlor, Brom, Methyl, Trifluoromethyl oder Methoxy;
- X: steht für Wasserstoff, Chlor, Fluor oder Methyl;
insbesondere wobei X und Y für folgende Kombinationen (Y,X) stehen: (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F).

In einer weiteren Ausgestaltung (Ausgestaltung 4-3) sind die besonders bevorzugten Substituenten bzw. Bereiche der in den Verbindungen der Formel (I) aufgeführten Reste wie folgt:
- V: steht für Sauerstoff;
- Q: steht für C-R⁵, wobei
- R⁵: für Wasserstoff, Methyl, Ethyl, Methoxy, Cyclopropyl oder Trifluoromethyl steht;
- R³ und R⁶: stehen unabhängig voneinander für Difluorchlormethyl, Cyclopropyl oder Phenyl;
- W: steht für Wasserstoff oder Fluor;
- n: steht für die Zahl 0 oder 1;
- Y: steht für Fluor, Chlor, Brom, Methyl, Trifluoromethyl oder Methoxy;
- X: steht für Wasserstoff, Chlor, Fluor oder Methyl;
insbesondere wobei X und Y für folgende Kombinationen (Y,X) stehen: (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F).

In einer weiteren Ausgestaltung (Ausgestaltung 4-4) sind die besonders bevorzugten Substituenten bzw. Bereiche der in den Verbindungen der Formel (I) aufgeführten Reste wie folgt:
- V: steht für Sauerstoff;
- Q: steht für C-R⁵, wobei
- R⁵: für Wasserstoff, Methyl, Ethyl, Methoxy, Cyclopropyl oder Trifluoromethyl steht;
- R³ und R⁶: stehen unabhängig voneinander für Wasserstoff, Fluor, Methyl, Ethyl, Isopropyl, Trifluoromethyl, Pentafluoroethyl, Difluoromethyl, 1,1-Difluoroethyl, Difluorchlormethyl, Cyclopropyl oder Phenyl;
- W: steht für Wasserstoff oder Fluor;
- n: steht für die Zahl 0 oder 1;
- Y: steht für Fluor, Chlor, Brom, Methyl, Trifluoromethyl oder Methoxy;
- X: steht für Wasserstoff, Chlor, Fluor oder Methyl;
insbesondere wobei X und Y für folgende Kombinationen (Y,X) stehen: (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F).

In einer weiteren Ausgestaltung (Ausgestaltung 4-5) sind die besonders bevorzugten Substituenten bzw. Bereiche der in den Verbindungen der Formel (I) aufgeführten Reste wie folgt:
- V: steht für Sauerstoff;
- Q: steht für C-R⁵, wobei
- R⁵: für Wasserstoff, Methyl, Ethyl, Methoxy, Cyclopropyl oder Trifluoromethyl steht;
- R³ und R⁶: stehen unabhängig voneinander für Fluor, Isopropyl, Pentafluoroethyl, 1,1-Difluoroethyl, Difluorchlormethyl, Cyclopropyl oder Phenyl;
- W: steht für Wasserstoff oder Fluor;
- n: steht für die Zahl 0 oder 1;
- Y: steht für Fluor, Chlor, Brom, Methyl, Trifluoromethyl oder Methoxy;
- X: steht für Wasserstoff, Chlor, Fluor oder Methyl;
insbesondere wobei X und Y für folgende Kombinationen (Y,X) stehen: (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F).

Ganz besonders bevorzugte Substituenten bzw. Bereiche der in den Verbindungen der Formel (I) aufgeführten Reste werden im Folgenden erläutert (Ausgestaltung 5-1).
- V: steht für Sauerstoff;
- Q: steht für C-R⁵, wobei
- R⁵: für Wasserstoff steht;
- R³: steht für Trifluoromethyl;
- R⁶: steht für Wasserstoff;
- W: steht für Fluor;
- n: steht für die Zahl 0 oder 1;
- Y: steht für Chlor oder Methyl;
- X: steht für Chlor, Fluor oder Methyl;
insbesondere wobei X und Y für folgende Kombinationen (Y,X) stehen: (Me, F), (Me,Me), (Cl,Cl).

In einer weiteren Ausgestaltung (Ausgestaltung 5-2) sind die ganz besonders bevorzugten Substituenten bzw. Bereiche der in den Verbindungen der Formel (I) aufgeführten Reste wie folgt:
- V: steht für Sauerstoff;
- Q: steht für C-R⁵, wobei
- R⁵: für Wasserstoff oder Methyl steht;
- R³: steht für Wasserstoff, Methyl, Ethyl, Cyclopropyl, Isopropyl, Difluoromethyl, Trifluoromethyl, Pentafluoroethyl, 1,1-Difluoroethyl oder Phenyl;
- R⁶: steht für Wasserstoff, Fluor oder Methyl;
- W: steht für Fluor;
- n: steht für die Zahl 0 oder 1;
- Y: steht für Brom, Chlor oder Methyl;
- X: steht für Wasserstoff, Chlor, Fluor oder Methyl;
insbesondere wobei X und Y für folgende Kombinationen (Y,X) stehen: (Me,H), (Me,Cl), (Me, F), (Me,Me), (Cl,Cl), (Cl,F), (Cl,H), (Br,F).

Wenn in obigen Definitionen in Ringen Schwefel und/oder Stickstoff vorkommen, wie beispielsweise in Ausdrücken wie "in welchen die Ringe mindestens ein Heteroatom aus der Reihe Schwefel, Sauerstoff (wobei Sauerstoffatome nicht unmittelbar benachbart sein dürfen) und Stickstoff enthalten können" oder "in welchen ein oder zwei Ringglieder jeweils durch ein Heteroatom aus der Reihe Schwefel, Sauerstoff (wobei Sauerstoffatome nicht unmittelbar benachbart sein dürfen) und Stickstoff ersetzt sein können", dann kann, sofern nichts anderes angegeben ist, der Schwefel auch als SO oder SO₂ vorliegen, der Stickstoff, sofern er nicht als -N= vorliegt, neben NH auch als N-Alkyl (insbesondere N-C₁-C₆-Alkyl) vorliegen.

In den breitesten und den bevorzugten Definitionen ist, sofern nichts anderes angegeben ist,

Halogen ausgewählt aus der Reihe Fluor, Chlor, Brom und Iod, bevorzugt wiederum aus der Reihe Fluor, Chlor und Brom,

Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl.

In den weiter bevorzugten Definitionen ist, sofern nichts anderes angegeben ist,

Halogen ausgewählt aus der Reihe Fluor, Chlor, Brom und Iod, bevorzugt wiederum aus der Reihe Fluor, Chlor und Brom,

Durch Halogen substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können. Sofern Substituenten vorgesehen oder gegebenenfalls vorgesehen sind, handelt es sich bei den Substituenten, sofern nichts anderes angegeben ist, um Halogen, Alkyl, Cycloalkyl, Cyano, Nitro, Alkoxy, Halogenalkyl oder Halogenalkoxy, insbesondere um Fluor, Chlor, (C₁-C₃)Alkyl, (C₃-C₆)Cycloalkyl (insbesondere Cyclopropyl), Cyano, (C₁-C₃)Alkoxy, (C₁-C₃)Halogenalkyl oder (C₁-C₃)Halogenalkoxy.

Die oben aufgeführten allgemeinen oder in Vorzugsbereichen aufgeführten Restedefinitionen bzw. Erläuterungen gelten für die Endprodukte (auch für die Verbindungen der Formel (I) mit der später aufgeführten Substruktur (I-A) und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt, wobei jede vorstehend als bevorzugt beschriebene Ausgestaltung eine eigenständige Kombination darstellt, insbesondere eine Kombination wie beschrieben in Ausgestaltung 2-1 oder in Ausgestaltung 2-2 oder in Ausgestaltung 2-3.

Erfindungsgemäß weiter bevorzugt werden Verbindungen der Formel (I), in welchen eine Kombination der vorstehend als weiter bevorzugt aufgeführten Bedeutungen vorliegt, wobei jede vorstehend als weiter bevorzugt beschriebene Ausgestaltung eine eigenständige Kombination darstellt, insbesondere eine Kombination wie beschrieben in Ausgestaltung 3-1 oder in Ausgestaltung 3-2 oder in Ausgestaltung 3-3.

Erfindungsgemäß besonders bevorzugt werden Verbindungen der Formel (I), in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt, insbesondere eine Kombination wie beschrieben in Ausgestaltung 4-1 oder in Ausgestaltung 4-2 oder in Ausgestaltung 4-3 oder in ausgestaltung 4-4 oder in ausgestaltung 4-5.

Erfindungsgemäß ganz besonders bevorzugt werden Verbindungen der Formel (I), in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt, insbesondere eine Kombination wie beschrieben in Ausgestaltung 5-1 oder in Ausgestaltung 5-2.

In weiteren bevorzugten Ausführungsformen betrifft die Erfindung Verbindungen der Formel (I), in denen Q für C-R⁵ steht. Hieraus ergeben sich Verbindungen der Formel (I-A)

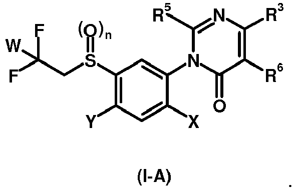

In der durch die Struktur (I-A) definierten Verbindungen der Formel (I) haben die Reste bzw. Strukturelemente R⁵, R³, R⁶, W, n, Y und X die weiter oben genannten Bedeutungen.

Insbesondere stehen X und Y für folgende Kombinationen (Y,X): (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F), wobei die folgenden Kombinationen (Y,X) besonders bevorzugt sind: (Me, F), (Me,Me), (Cl,Cl).

Bevorzugt innerhalb der durch die Struktur (I-A) definierten Verbindungen der Formel (I) werden diejenigen Verbindungen, in welchen eine Kombination der vorstehend als bevorzugt aufgeführten Bedeutungen vorliegt, wobei jede vorstehend als bevorzugt beschriebene Ausgestaltung eine eigenständige Kombination darstellt, insbesondere eine Kombination wie beschrieben in Ausgestaltung 2-1 oder in Ausgestaltung 2-2 oder in Ausgestaltung 2-3.

Weiter bevorzugt innerhalb der durch die Struktur (I-A) definierten Verbindungen der Formel (I) werden diejenigen Verbindungen, in welchen eine Kombination der vorstehend als weiter bevorzugt aufgeführten Bedeutungen vorliegt, wobei jede vorstehend als weiter bevorzugt beschriebene Ausgestaltung eine eigenständige Kombination darstellt, insbesondere eine Kombination wie beschrieben in Ausgestaltung 3-1 oder in Ausgestaltung 3-2 oder in Ausgestaltung 3-3.

Besonders bevorzugt innerhalb der durch die Struktur (I-A) definierten Verbindungen der Formel (I) werden diejenigen Verbindungen, in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt, insbesondere eine Kombination wie beschrieben in Ausgestaltung 4-1 oder in Ausgestaltung 4-2 oder in Ausgestaltung 4-3 oder in ausgestaltung 4-4 oder in ausgestaltung 4-5.

Ganz besonders bevorzugt innerhalb der durch die Struktur (I-A) definierten Verbindungen der Formel (I) werden diejenigen Verbindungen, in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt, insbesondere eine Kombination wie beschrieben in Ausgestaltung 5-1 oder in Ausgestaltung 5-2.

Die Verbindungen der Formel (I) können auch als Salze, insbesondere Säureadditionssalze und Metallsalzkomplexe, vorliegen. Die Verbindungen der Formel (I) und deren Säureadditionssalze und Metallsalzkomplexe besitzen gute Wirksamkeit, insbesondere zur Bekämpfung von tierischen Schädlingen, zu denen Arthropoden und insbesondere Insekten und Akariden zählen.

Als geeignete Salze der Verbindungen der allgemeinen Formel (I) können übliche nicht toxische Salze, d. h. Salze mit entsprechenden Basen und Salze mit zugesetzten Säuren genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calzium- oder Magnesiumsalze, Ammoniumsalze, Salze mit organischen Basen sowie mit anorganischen Aminen, beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren, beispielsweise Hydrochloride, Hydrobromide, Dihydrosulfate, Trihydrosulfate, oder Phosphate, Salze mit organischen Carbonsäuren oder organischen Sulfosäure, beispielsweise Formiate, Acetate, Trifluoracetate, Maleate, Tartrate, Methansulfonate, Benzolsulfonate oder para-Toluolsulfonate, Salze mit basischen Aminosäuren, beispielsweise Arginate, Aspartate oder Glutamate und Ähnliches zu nennen.

Die Verbindungen der Formel (I) können gegebenenfalls auch in Abhängigkeit von der Art der Substituenten als Stereoisomere, d.h. als geometrische und/oder als optische Isomere oder Isomerengemische vorliegen in unterschiedlichen Zusammensetzungen vorliegen. Sowohl die reinen Stereoisomeren als auch beliebige Gemische dieser Isomeren sind Gegenstand dieser Erfindung, auch wenn hier im Allgemeinen nur von Verbindungen der Formel (I) die Rede ist.

Vorzugsweise werden jedoch die optisch aktiven, stereoisomeren Formen der Verbindungen der Formel (I) und deren Salze erfindungsgemäß verwendet.

Die Erfindung betrifft daher sowohl die reinen Enantiomeren und Diastereomeren, als auch deren Gemische zur Bekämpfung von tierischen Schädlingen, zu denen Arthropoden und insbesondere Insekten und Akariden zählen. Eine individuelle Ausgestaltung der Erfindung ist daher auf das Vorliegen des R-Enantiomers gerichtet bzw. auf ein Gemisch, welches mehrheitlich das R-Enantiomer umfasst, vorzugsweise wobei das Verhältnis von R- zu S-Enantiomer mindestens 60:40 und zunehmend bevorzugt mindestens 70:30, 75:25, 80:20, 85:15 und 90:10 beträgt. Eine weitere individuelle Ausgestaltung der Erfindung ist daher auf das Vorliegen des S-Enantiomers gerichtet bzw. auf ein Gemisch, welches mehrheitlich das S-Enantiomer umfasst, vorzugsweise wobei das Verhältnis von S-zu R-Enantiomer mindestens 60:40 und zunehmend bevorzugt mindestens 70:30, 75:25, 80:20, 85:15 und 90:10 beträgt.

Die Verbindungen der Formel (I) können in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Verbindungen sind durch die Formel (I) allgemein definiert und die beinhaltet auch alle möglichen Rotamere und Gemische davon.

Die erfindungsgemässen Verbindungen der Formel (I) können nach üblichen, dem Fachmann bekannten Methoden hergestellt werden. Verschiedene Herstellungsverfahren, die gleichfalls Gegenstand der Erfindung sind, werden nachfolgend beschrieben.

### Herstellverfahren

Die Verbindungen der allgemeinen Formel (I) lassen sich in Verbindungen mit n=0 (Ia), n=1 (Ib) und n=2 (Ic) unterteilen und können nach folgendem Schema hergestellt werden, beispielsweise nach Methoden A und B, wie in der Anmeldung WO 2010/100189 beschrieben. Abweichend von diesen Methoden können die Verbindungen der Formel (I) auch nach Verfahren D und E oder den nachfolgend erläuterten Verfahren F und G oder nach in WO1999/055668 beschriebenen Verfahren hergestellt werden.

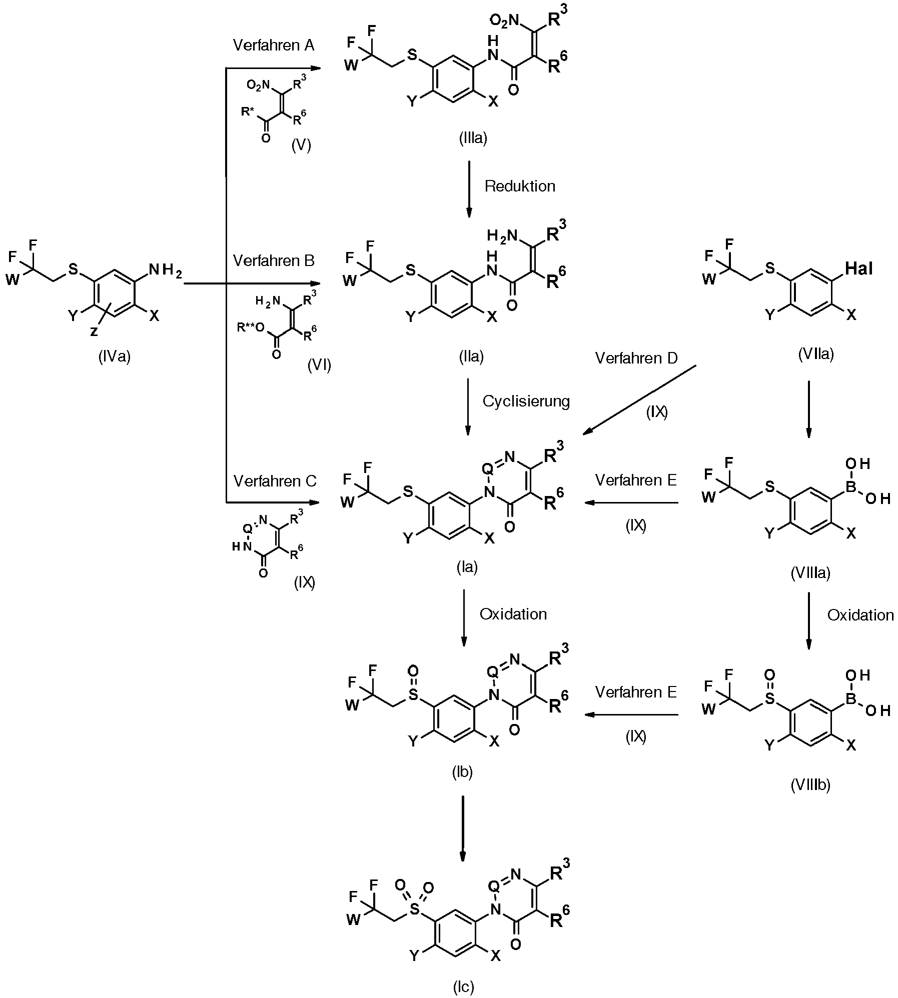

### Verfahren A

Verbindungen der Formel (IIIa) oder Tautomere davon können beispielsweise nach Verfahren A aus der Umsetzung von Anilinen der Formel (IVa) mit Nitroverbindungen der Formel (V), in welchen R* für Hydroxy oder Halogen (bevorzugt Cl und Br) steht, hergestellt werden.

Für die Herstellung von Carbonsäureamiden aus Carbonsäuren (R*=Hydroxy) oder Carbonsäurehalogeniden (R*=Halogen) ist eine breite Vielfalt von Methoden bekannt, z.B. G. Benz in Comprehensive Organic Synthesis, 1st Ed., Pergamon Press, Oxford, 1991, Vol. 6, S. 381-417; P.D. Bailey et al. in Comprehensive Organic Functional Group Transformation, 1st Ed., Elsevier Science Ltd., Oxford, 1995, Vol. 5, S. 257-308 und R.C. Larock in Comprehensive Organic Transformations, 2nd Ed., Wiley-VCH, New York, Weinheim, 1999, S. 1929-1994. Carbonsäurechloride können isoliert oder insitu erzeugt eingesetzt werden.

Die Amidierungsreaktionen erfolgen gegebenenfalls in Gegenwart eines Kondensationsmittels, gegebenenfalls in Gegenwart eines Säureaktivators, gegebenenfalls in Gegenwart eines Säureakzeptors und gegebenenfalls in Gegenwart eines Lösungsmittels. Als Kondensationsmittel kommen alle üblicherweise für derartige Amidierungsreaktionen verwendbaren Kondensationsmittel infrage. Beispielhaft genannt seien Säurehalogenidbildner wir Phosgen, Phosphortrichlorid, Oxalylchlorid oder Thionylchlorid; Carbodiimide, wie *N*,*N*'-Dicyclohexylcarbodiimid (DCC) und 1-(3-Dimethylaminopropyl)-3-ethyl-carbodiimid (EDCI), oder andere übliche Kondensationsmittel, wie Phosphorpentoxid, Polyphosphorsäure, *N*,*N*'-Carbonyldiimidazol, 2-Chlorpyridin 1-Methoiodid (Mukaiyamas Reagenz), 2-Ethoxy-N-ethoxycarbonyl-1,2-dihydrochinolin (EEDQ), Triphenylphosphin/Tetrachlorkohlenstoff, Brom-tripyrrolidino-phosphonium-hexafluorphosphat (BROP), O-(*1H*-Benzotriazol-1-yloxy)tris(dimethylamino)phosphonium-hexafluorphosphat (BOP), *N,N,N',N'*-Bis(tetramethylen)cloruronium-tetrafluorborat, O-(*1H*-Benzotriazol-1-yl)-*N,N,N'*,*N'-*tetramethyluronium-hexafluorphosphat (HBTU), O-(*1H*-Benzotriazol-1-yl)-*N,N,N',N'-*bis(tetramethylen)uronium-hexafluorphosphat, O-(*1H*-Benzotriazol-1-yl)-*N,N,N',N'-*tetramethyluronium-tetrafluorborat (TBTU), O-(*1H*-Benzotriazol-1-yl)-*N,N,N',N'-*bis(tetramethylen)uronium-tetrafluorborat, O-(7-Azabenzotriazol-1-yl)-*N,N,N'*,*N'*-tetramethyluronium-hexafluorphosphat (HATU) und 1-Hydroxybenzotriazol. Diese Reagenzien können separat oder gegebenenfalls in Kombination eingesetzt werden. Als Säureakzeptor kommen alle üblichen anorganischen oder organischen Basen infrage, beispielsweise Triethylamin, Diisopropylethylamin, *N-*Methylmorpholin oder *N,N*-Dimethylaminopyridin. Das erfindungsgemäße Verfahren A wird gegebenenfalls in Gegenwart eines geeigneten Reaktionshilfstoffes wie beispielsweise *N,N-*Dimethylformamid oder *N,N*-Dimethylaminopyridin durchgeführt.

Des Weiteren können auch gemischte Anhydride zur Darstellung von (IIIa) verwendet werden, wie beispielsweise in J. Am. Chem. Soc 1967, 5012, publiziert. Bei diesem Verfahren können verschiedene Chlorameisensäureester zum Einsatz kommen, wie z.B. Chlorameisensäureisobutylester, Chlorameisensäureisopropylester. Ebenfalls können dafür Diethylacetylchlorid, Trimethylacetylchlorid und ähnliche verwendet werden.

Verbindungen der allgemeinen Formel (IIa) oder Tautomere davon können beispielsweise durch Reduktion der Nitroverbindungen der allgemeinen Formel (IIIa) nach literaturbekannten Methoden hergestellt werden. Geeignete Verfahren für solche Reduktionen sind vor allem Metall-vermittelte Reaktionen wie z.B. Zinn(II)chlorid, Eisenpulver, Zinkpulver, Raney-Nickel, Palladium(0) auf Kohle oder Platindioxid (als Hydrat). Die Metall-vermittelten Reduktionen wie z.B. mit Zinn(II)chlorid können nach einem in Organic Syntheses Coll. Vol. (III), 453 beschriebenen Verfahren durchgeführt werden.

### Verfahren B

Alternativ können Verbindungen der allgemeinen Formel (IIa) durch einer Acylierungsreaktion nach Verfahren B hergestellt werden, in dem ein Anilin der allgemeinen Formel (IVa) mit einem geeigneten Carbonsäurederivaten der Formel (VI) umgesetzt wird, wobei R** bevorzugt für Alkyl steht. Dies kann ohne Aktivation erfolgen, wie vom B. M. Trost und I. Fleming in Comprehensive Organic Synthesis, Ed. Pergamon, 1991, Vol. 6, beschrieben. Alternativ sind in der Literatur Aktivierungsmethoden durch die Bildung eines Aluminiumamids bekannt, wie von T. Ooi und K. Marouka in Science of Synthesis, Ed. Georg Thieme, 2003, Vol. 7, 225-246. Diese Aluminiumamide können aus den Anilinen oder deren Salze durch Umsetzung mit Trimethylaluminium oder deren luft-stabilem Addukt mit 1,4-Diazobicyclo[2.2.3]oktan (DABCO), wie vom S. Woodward in Tet. Lett. 2006, 47, 5767-5769, beschrieben.

R** kann auch für Wasserstoff stehen, sodass für die Synthese von Verbindungen der Formel (IIa) alle beschriebenen Synthesenmethoden im Verfahren A geeignet wären.

Für die Herstellung von Thioether der allgemeinen Formel (Ia) eignen sich unterschiedliche Methoden. Beispielsweise seien gennant: ausgehend von Verbindungen der Formel (IIa) durch Ringschluss; ausgehend von Anilinen der Formel (IVa) durch Reaktion mit Verbindungen der Formel (IX) nach Verfahren C oder ausgehend von Halogeniden der Formel (VIIa) bzw. Boronsäuren der Formel (VIIIa) oder (VIIIb) durch metall-katalysierten Reaktionen nach Verfahren D bzw. Verfahren E.

### Cyclisierung

Für Q=C-R⁵, wo R⁵ für H oder Alkyl steht, kann die Herstellung der Thioether der allgemeinen Formel (Ia) nach literaturbekannten Methoden durch Cyclisierung von offenkettigen Vorstufen der Formel (IIa) mit einem Orthoester, wie Orthoameisensäuretriethylester oder Orthoessigsäuretriethylester, gegebenenfalls in Gegenwart eines Lösungs- und Verdünnungsmittel, gegebenenfalls in Gegenwart einer Säure organischer Natur (wie zum Beispiel *para*-Toluolsulfonsäure) oder anorganischen Natur (wie Salzsäure oder Schwefelsäure) in katalytischen oder stöchiometrischen Mengen oder in Überschuss oder anstelle des Lösungs- oder Verdünnungsmittels erfolgen. Alternativ kann der Thioether der allgemeinen Formel (Ia) nach literaturbekannten Methoden durch Reaktion mit DMF-DMA und anschließende Reaktion mit Ameisensäure hergestellt werden.

Für Q=C-R⁵, wo R⁵ für Alkyl oder Haloalkyl steht, kann die Herstellung der Thioether der allgemeinen Formel (Ia) auch durch Umsetzung mit den entsprechenden Carbonsäurenanhydriden oder Säurechloriden nach literaturbekannten Methoden erfolgen, zum Beispiel wie für R⁵ = CF₃ im Patent WO 2008/039489 beschrieben.

Für Q=N können die Verbindungen der Formel (Ia) durch Azodiazotierung der Verbindungen der Formel (IIa) nach literaturbekannten Methoden hergestellt werden. Beispielsweise werden Verbindungen der Formel (IIa) mit einer Nitritquelle, wie Natriumnitrit oder Isobutylnitrit, typischerweise in Wasser, Alkohol oder einem polaren, inerten Lösungsmittel, bei 0 bis 5 °C in Anwesenheit einer organischen oder anorganischen Säure versetzt. Beispiele für Reaktionsbedingungen können zum Beispiel vom Patent WO 2004/242572 oder aus J. Amer. Chem. Soc. Perkin Trans. 1, 1980, 633-638, entnommen werden.

### Verfahren C

Alternativ können die Verbindungen der Formel (Ia) ausgehend von Anilinen der Formel (IVa) durch Reaktion mit Verbindungen der Formel (IX) oder deren tautomerischen Hydroxypyrimidinonen nach Verfahren C hergestellt werden. Wie von Yang et al. in Org. Lett. 2009, 11, 6, 1421-1424 beschrieben, erfolgt die *N*-Arylierung der Hydroxypyrimidinonen unter milden Reaktionsbedingungen, zum Beispiel durch HATU-vermittelte Kupplung mit primären Aminen mit DBU als Base in Acetonitril als Lösungsmittel, meistens bei Raumtemperatur oder bis zu 70°C.

### Verfahren D

Eine alternative Herstellung der Verbindungen der Formel (Ia) stellt die Umsetzung von Halogeniden der Formel (VIIa) mit Verbindungen der Formel (IX) unter metall-katalysierten Reaktionsbedingungen dar. In der Literatur sind zahlreichen Methoden bekannt, zum Beispiel in Chem. Pharm. Bull. 1997, 45, 4, 719-721; in Tet. Lett. 2006, 47, 7677-7680; oder Synlett 2008, 9, 1335-1340, in denen Kupferiodid als Metallquelle in Anwesenheit einer Base und gegebenenfalls eines Ligandes bei höheren Temperaturen (beispielsweise 120 bis 150 °C) eingesetzt wird.

### Verfahren E

Ebenfalls wurde gefunden, dass die Umsetzung von Boronsäuren der Formel (VIIIa) mit Verbindungen der Formel (IX) durch metall-katalysierten Reaktionen zur Herstellung der Verbindungen der Formel (Ia) dienen kann. Eine Übersicht solcher Reaktionen findet man in Synthesis 2011, 6, 829-856. Eine geeignete Metallquelle ist Kupfer(II)acetat, wie in Synlett 2010, 5 ,721-724; Tetrahedron 2006, 62, 8, 1764-1771; Tetrahedron Lett. 2005, 46, 34, 5699-5702 oder WO 2010/104818.

Die Oxidation der Boronsäuren der Formel (VIIIa) oder deren Boronsäureester nach literaturbekannten Methoden, wie zum Beispiel mit Natriumperiodat, führt zu Sulfoxiden der Formel (VIIIb), die ebenfalls mit Verbindungen der Formel (IX) unter metall-katalysierten Reaktionsbedingungen umgesetzt werden können, sodass man zu den Zielverbindungen (Ib) gelangen kann.

Bei der Durchführung der erfindungsgemäßen Verfahren D und E kann gegebenenfalls jede für diese Reaktionen geeignete, handelsübliche Mikrowellenapparatur verwendet werden (z.B. Anton Paar Monowave 300, CEM Discover S, Biotage Initiator 60).

Alternativ können Verbindungen der allgemeinen Formel (Ib) durch ähnliche wie die hier genannten Methoden in einer anderen Reihenfolge hergestellt werden, zum Beispiel durch Oxidation der Aniline der Formel (IVa) zu Sulfoxide der Formel (IVb) und deren weiteren Umsetzung nach Verfahren A, B oder C.

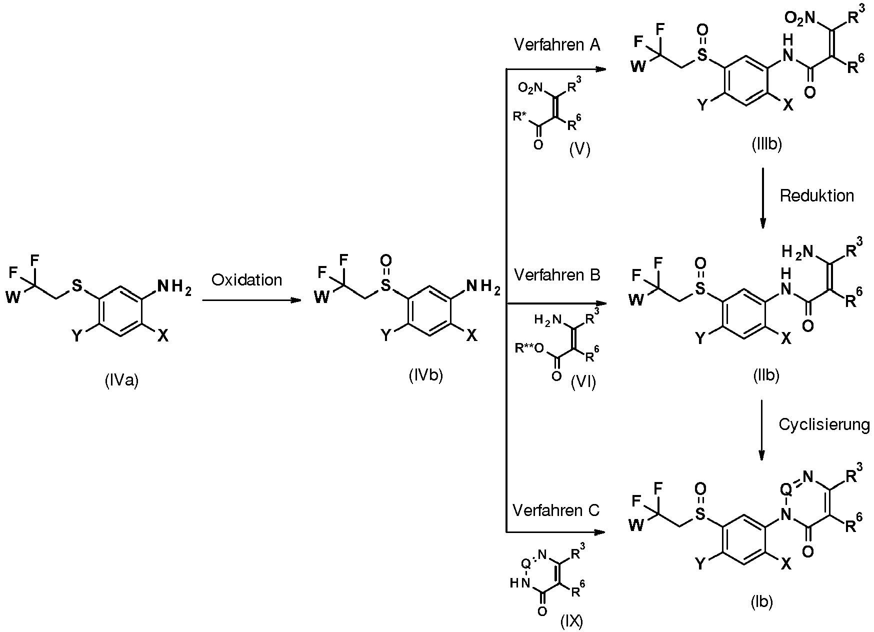

Abweichend von diesen Methoden können die Verbindungen der Formel (I) mit Q=C-R⁵ auch nach Verfahren F hergestellt werden.

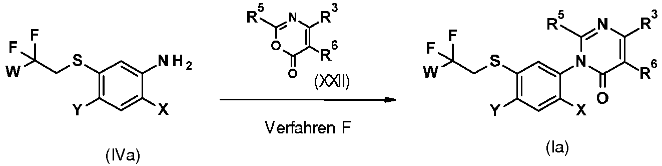

Aniline der Formel (IVa) können mit Oxazinonen der Formel (XXII) zu Zielverbindungen der Formel (Ia) führen, zum Beispiel nach Assiut Univ. J. of Chemistry 2006, 45-63. Oxazinone der Formel (XXII) sind bekannt oder durch litteraturbekannte Methoden herstellbar, wie zum Beispiel in Journal of the Chemical Society 1965, 4240-4246.

Abweichend von diesen Methoden können die Verbindungen der Formel (I) auch nach Verfahren G hergestellt werden.

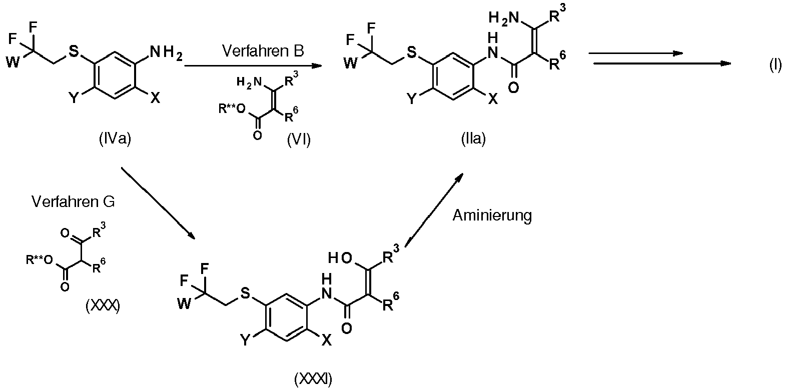

Aniline der Formel (IVa) können mit β-Ketoestern der Formel (XXX) zu Enolen der Formel (XXXI) führen, zum Beispiel nach WO 2004/056785. Die Aminierung der Enole der Formel (XXXI) mit einem Aminierungsmittel wie Ammoniak oder Ammoniumacetat liefert die offenkettige Verbindung der Formel (IIa). Die Herstellung der Verbindungen der Formel (I) aus (IIa) ist bereits weiter oben im Verfahren B beschrieben.

### Reaktionen in der Mikrowelle

Bei der Durchführung der erfindungsgemäßen Verfahren kann gegebenenfalls jede für diese Reaktionen geeignete, handelsübliche Mikrowellenapparatur verwendet werden (z.B. Anton Paar Monowave 300, CEM Discover S, Biotage Initiator 60).

### Thionierung

Ein weiteres allgemeines Verfahren zur Herstellung der erfindungsgemäßen Verbindungen der allgemeinen Formel (Ia) oder (Ib), in denen V für Schwefel steht, besteht in der Umwandlung der Carbonylgruppe entsprechender Vorstufen in die Thiocarbonylgruppe mit Hilfe geeigneter Schwefelungsreagenzien wie beispielsweise Phosphorpentasulfid oder Lawessons Reagenz in einem geeigneten Lösungsmittel, beispielsweise Pyridin, Xylol oder Cumol. Diese Variante ist in zahlreichen Publikationen beschrieben, z.B. in J. Amer. Chem. Soc. 1956, 1938-1941, Chem. Pharm. Bull. 1962, 10, 647-652, US 3007927, DE 2554866 oder WO 2000026194.

### Allgemeine Herstellverfahren für die Oxidation von Thioethern zu Sulfoxiden

Verbindungen der allgemeinen Formel (Ib) lassen sich durch Oxidation nach literaturbekannten Verfahren aus Verbindungen der allgemeinen Formel (Ia) herstellen, beispielsweise durch ein Oxidationsmittel in einem geeignetem Lösungs- und Verdünnungsmittel. Als Oxidationsmittel eignen sich zum Beispiel verdünnte Salpetersäure, Wasserstoffperoxid und Peroxycarbonsäuren, wie etwa meta-Chlorperbenzoesäure. Als Lösungsmittel eignen sich inerte organische Lösungsmittel, typischerweise Acetonitril und halogenierte Lösungsmittel wie Dichlormethan, Chloroform oder Dichlorethan.

Zur Erzeugung enantiomeren angereicherten Sulfoxide eignen sich eine Vielfalt von Methoden, wie von A.R. Maguire in ARKIVOC, 2011(i), 1-110, beschrieben: metall-katalysierte asymmetrische Oxidationen von Thioethern, zum Beispiel mit Titanium oder Vanadium als meistbenutzten Katalysatorquellen, in Form von Ti(OⁱPr₄) oder VO(acac)₂, zusammen mit einem chiralen Liganden und einem Oxidationsmittel wie tert-Butylwasserstoffperoxid (TBHP), 2-Phenylpropan-2-ylhydroperoxid (CHP) oder Wasserstoffperoxid; nicht-metall katalysierte asymmetrische Oxidationen durch Verwendung von chiralen Oxidationsmitteln oder chiralen Katalysatoren; elektrochemische oder biologische asymmetrische Oxidationen sowie kinetische Resolution von Sulfoxiden und nukleophilische Versetzung (nach Andersens Methode).

Die Enantiomere können auch aus dem Racemat gewonnen werden, in dem sie zum Beispiel präparativ auf einer chiralen HPLC getrennt werden.

### Erläuterung der Ausgangsstoffe und Zwischenprodukte

Aniline der Formel (IV), Halogenide der Formel (VII) und Boronsäuren der Formel (VIII) sind zentrale Bausteine, um die Verbindungen der Formel (I) herzustellen.

Die *Aniline der allgemeinen Formel* (*IV*) lassen sich in Verbindungen mit n=0 (IVa) und n=1 (IVb) unterteilen.

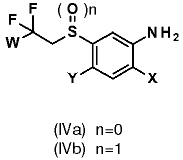

Aniline der Formel (IVa) sind literaturbekannt, z.B. aus JP 2007/284356, oder können anhand literaturbekannter Verfahren synthetisiert werden.

Die Aniline der allgemeinen Formel (IVa) können beispielsweise wie im folgenden Schema hergestellt werden. 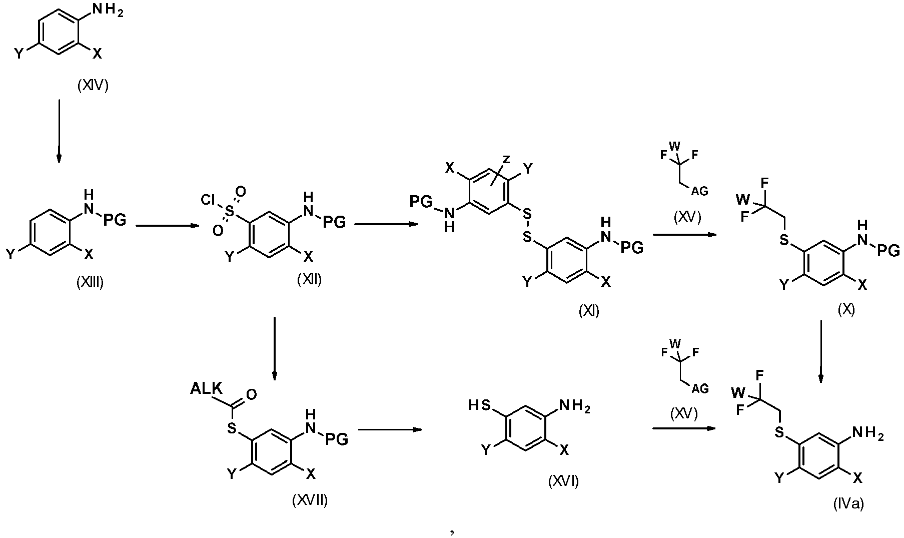 wobei X, Y und W die oben angegebenen Bedeutungen haben, AG für eine Abgangsgruppe und PG für eine Schutzgruppe steht.

Aniline der Formel (XIV) sind entweder kommerziell erhältlich oder können durch bekannte Methoden hergestellt werden. Sie können mit einer geeigneten Schutzgruppe, wie z.B. einer Acetylgruppe, zu Verbindungen der Formel (XIII) geschützt werden. Beispielsweise in Gegenwart von Säuren, Säureanhydriden oder Säurechloriden können die Aniline (XIV) in die entsprechenden Anilide (XIII) überführt werden. Die Chlorsulfonierung der geschützen Aniline (XIII) mit Chlorsulfonsäure liefert die entsprechenden Sulfonylchloride (XII). Die Reduktion der Sulfonylchloride (XII) in die Disulfide (XI) ist mit literaturbekannten Methoden wie zum Beispiel Eisen in Salzsäure oder Iodid möglich. Die Umsetzung der Disulfide (XI) mit Haloalkylelektrophilen der Formel (XV), wobei AG für eine Abgangsgruppe wie zum Beispiel Chlor, Brom, Tosylat, Mesylat oder Triflat steht, liefert die Sulfide (X). Die Schutzgruppe kann durch geeignete literaturbekannte Methoden abgespalten werden, so dass man Aniline der Formel (IVa) erhält.

Anstatt der Reduktion zum Disulfid (XI), kann das Sulfonylchlorid (XII) mit einem geeigneten Reduktionsmittel wie zum Beispiel Iod/Phosphor zum Alkylthioat (XVII) reduziert und anschließend durch eine geeignete Methode, wie zum Beispiel die Umsetzung mit Kaliumhydroxidlösung, zu Thiolen der Formel (XVI) entschützt werden. Die Umsetzung der Thiole (XVI) mit Haloalkylelektrophilen der Formel (XV), wobei AG für eine Abgangsgruppe wie zum Beispiel Chlor, Brom, Tosylat, Mesylat oder Triflat steht, liefert die Sulfide (IVa).

Ebenfalls bevorzugt können die Thioether der Formel (IVa) alternativ nach folgendem Schema hergestellt werden, 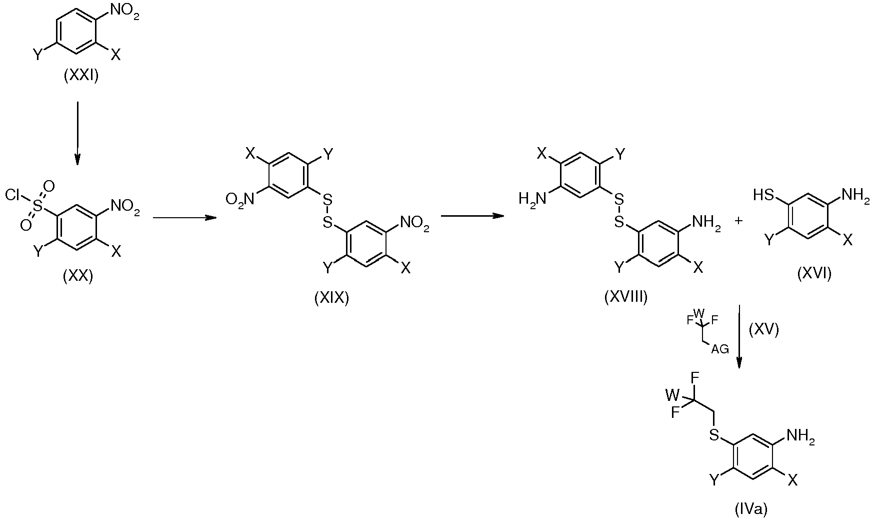 wobei X, Y und W die oben angegebenen Bedeutungen haben, AG für eine Abgangsgruppe und PG für eine Schutzgruppe steht.

Die Chlorsulfonierung der Nitroaromaten der Formel (XXI) mit Chlorsulfonsäure liefert die entsprechenden Sulfonylchloride (XX). Die Reduktion der Sulfonylchloride (XX) in die Bis(nitroaryl)disulfide (XIX) ist mit literaturbekannten Methoden, wie zum Beispiel Iodid, möglich. Die Reduktion der Disulfide (XIX) in die Disulfanediyldianiline (XVIII), die teilweise als Gemisch mit den entsprechenden Aminoarylthiolen (XVI) entstehen, ist mit allgemein bekannten Reduktionsmittel, wie zum Beispiel Wasserstoff, gegebenenfalls mit Hilfe heterogener Katalysatoren, wie zum Beispiel Raney-Nickel, Platin auf Aktivkohle oder Palladium auf Aktivkohle, möglich. Die Umsetzung der Disulfide (XVIII) bzw. Thiophenole (XVI) mit Haloalkylelektrophilen der Formel (XV), wobei AG für eine Abgangsgruppe wie zum Beispiel Chlor, Brom, Iod, Tosylat, Mesylat oder Triflat steht, liefert die 3-[(2,2,2-Trifluorethyl)sulfanyl]aniline der Formel (IVa).

Halogenide der allgemeinen Formel (VIIa) 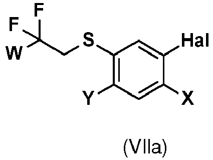 in welcher X, Y und W die oben angegebenen Bedeutungen haben und Hal für Chlor, Brom oder Iod steht, sind literaturbekannt aus WO 2007/034755, JP 2007/081019, JP 2007/284385, JP 2008/260706, JP 2008/308448, JP 2009/023910 oder WO 2012/176856 oder können anhand literaturbekannter Verfahren synthetisiert werden, die gegebenenfalls geringfügig modifiziert sein können.

Als Ausgangsstoffe für die Synthese der Iodide der allgemeinen Formel (VIIa) eignen sich Bromide derselben Formel, zum Beispiel in Halogenaustauschreaktionen nach literaturbekannten Methoden gegebenenfalls unter Metallkatalyse (s. H. Suzuki, Chem. Let. 1985, 3, 411-412; S. L. Buchwald, J. Amer. Chem. Soc. 2002, 124 (50), 14844-14845) . Ebenso ist die Synthese möglich ausgehend von Anilinen der Formel (IVa) unter Sandmeyers Reaktionsbedingungen, wie von E. B. Merkushev in Synthesis 1988, 12, 923-937, beschrieben.

### Boronsäuren der allgemeinen Formel (VIIIa) und (VIIIb)

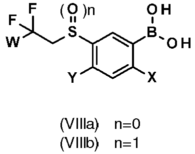

in welcher X, Y und W die oben angegebenen Bedeutungen haben, sind literaturbekannt, z.B. aus WO2007/034755, JP2007/284385, JP2009/023910 und WO2012/176856 oder können anhand literaturbekannter Verfahren synthetisiert werden.

Von besonderem Interesse sind ferner Zwischenprodukte, die in den beschriebenen Verfahren und Methoden dargestellt sind. Diese Zwischenprodukte stellen weitere Gegenstände der Erfindung dar. Zusätzlich zu den vorstehend beschriebenen Zwischenprodukten sind weitere Zwischenprodukte im folgenden beschrieben.

Ein weiterer Gegenstand der Erfindung ist eine Verbindung der Formel (IIa) 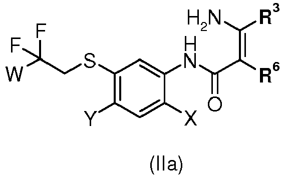 worin R³, R⁶, W, X und Y die vorstehend angegebenen Bedeutungen haben.

Bevorzugte Verbindungen der Formel (IIa) sind diejenigen, in welchen eine Kombination der vorstehend als bevorzugt aufgeführten Bedeutungen vorliegt, wobei jede vorstehend als bevorzugt beschriebene Ausgestaltung eine eigenständige Kombination darstellt.

Weiter bevorzugte Verbindungen der Formel (IIa) sind diejenigen, in welchen eine Kombination der vorstehend als weiter bevorzugt aufgeführten Bedeutungen vorliegt, wobei jede vorstehend als weiter bevorzugt beschriebene Ausgestaltung eine eigenständige Kombination darstellt.

Besonders bevorzugte Verbindungen der Formel (IIa) sind diejenigen, in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt.

Ganz besonders bevorzugte Verbindungen der Formel (IIa) sind diejenigen, in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt.

Die Verbindungen der Formel (IIa) können in verschiedenen tautomeren Formen vorkommen. Diese Formen sind folglich mit umfasst, auch wenn sie nicht explizit dargestellt wurden.

Ein weiterer Gegenstand der Erfindung ist eine Verbindung der Formel (XXXI) 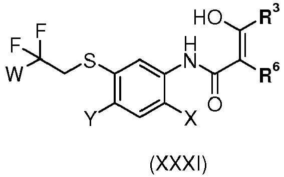 worin R³, R⁶, W, X und Y die vorstehend angegebenen Bedeutungen haben.

Bevorzugte Verbindungen der Formel (XXXI) sind diejenigen, in welchen eine Kombination der vorstehend als bevorzugt aufgeführten Bedeutungen vorliegt, wobei jede vorstehend als bevorzugt beschriebene Ausgestaltung eine eigenständige Kombination darstellt.

Weiter bevorzugte Verbindungen der Formel (XXXI) sind diejenigen, in welchen eine Kombination der vorstehend als weiter bevorzugt aufgeführten Bedeutungen vorliegt, wobei jede vorstehend als weiter bevorzugt beschriebene Ausgestaltung eine eigenständige Kombination darstellt.

Besonders bevorzugte Verbindungen der Formel (XXXI) sind diejenigen, in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt.

Ganz besonders bevorzugte Verbindungen der Formel (XXXI) sind diejenigen, in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt.

Die Verbindungen der Formel (XXXI) können in verschiedenen tautomeren Formen vorkommen. Diese Formen sind folglich mit umfasst, auch wenn sie nicht explizit dargestellt wurden.

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Panchlora spp., Parcoblatta spp., Periplaneta spp., z.B. Periplaneta americana, Periplaneta australasiae, Supella longipalpa;
aus der Ordnung der Coleoptera z.B. Acalymma vittatum, Acanthoscelides obtectus, Adoretus spp., Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex;
aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Nephotettix spp., z.B. Nephotettix cincticeps" Nephotettix nigropictus, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Monalonion atratum, Nezara spp., z.B. Nezara viridula, Oebalus spp., Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Diprion spp., z.B. Diprion similis, Hoplocampa spp., z.B. Hoplocampa cookei, Hoplocampa testudinea, Lasius spp., Monomorium pharaonis, Sirex spp., Solenopsis invicta, Tapinoma spp., Urocerus spp., Vespa spp., z.B. Vespa crabro, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Cornitermes cumulans, Cryptotermes spp., Incisitermes spp., Microtermes obesi, Odontotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Etiella spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia z.B. Eimeria spp. bekämpfen.

### Nematoden

Der Begriff "Nematoden" umfasst im vorliegenden Zusammenhang alle Arten des Stammes Nematoda und hierbei insbesondere Arten, die Pflanzen oder Pilze (zum Beispiel Arten der Ordnung Aphelenchida, Meloidogyne, Tylenchida und andere) oder auch Menschen und Tiere (zum Beispiel Arten der Ordnungen Trichinellida, Tylenchida, Rhabditida und Spirurida) parasitieren oder in bzw. an diesen Lebewesen Schädigungen verursachen, sowie andere parasitäre Helminthen.

Ein Nematizid im Pflanzenschutz, wie hier beschrieben, besitzt die Fähigkeit, Nematoden zu kontrollieren.

Der Begriff "Nematoden kontrollieren" bedeutet das Abtöten der Nematoden oder das Verhindern oder Erschweren ihrer Entwicklung bzw. ihres Wachstums oder das Verhindern oder Erschweren ihres Eindringens in oder ihres Saugens am pflanzlichen Gewebe.

Dabei wird die Wirksamkeit der Verbindungen durch einen Vergleich von Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden zwischen einer mit der Verbindung der Formel (I) behandelten Pflanze, Pflanzenteil oder dem behandelten Boden und einer unbehandelten Pflanze, Pflanzenteil oder unbehandelten Boden (100 %) ermittelt. Vorzugsweise wird eine Verringerung um 25-50 % im Vergleich mit einer unbehandelten Pflanze, Pflanzenteil oder unbehandelten Boden, besonders bevorzugt eine Verringerung um 51 - 79 % und ganz besonders bevorzugt das vollständige Abtöten oder die vollständige Verhinderung von Entwicklung und Wachstum der Nematoden durch eine Verringerung um 80 bis 100 % erreicht. Die Kontrolle von Nematoden, wie hier beschreiben, beinhaltet ebenso die Kontrolle der Nematoden-Vermehrung (Entwicklung von Zysten und/oder Eier). Verbindungen der Formel (I) können ebenso verwendet werden, um die Pflanzen oder Tiere gesund zu erhalten und können kurativ, präventiv oder systemisch zur Nematoden-Kontrolle eingesetzt werden.

Dem Fachmann sind Methoden bekannt, wie Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden bestimmt werden.

Die Verwendung einer Verbindung der Formel (I) kann die Pflanze gesund erhalten und beinhaltet ebenso eine Reduktion der von Nematoden hervorgerufenen Schäden sowie eine Erhöhung der Erntemenge.

Der Begriff "Nematoden" bezieht sich im vorliegenden Zusammenhang auf Pflanzennematoden, unter die man alle Nematoden zusammenfasst, die Pflanzen schädigen. Pflanzennematoden umfassen pflanzenparasitäre Nematoden und im Boden lebende Nematoden. Zu den pflanzenparasitären Nematoden zählen Ektoparasiten wie Xiphinema spp., Longidorus spp. und Trichodorus spp.; Halbparasiten wie Tylenchulus spp.; migratorische Endoparasiten wie Pratylenchus spp., Radopholus spp. und Scutellonema spp.; ortsgebundene Parasiten wie Heterodera spp., Globodera spp. und Meloidogyne spp., sowie Stängel- und Blattendoparasiten wie Ditylenchus spp., Aphelenchoides spp. und Hirschmaniella spp.. Besonders schädliche wurzelparasitäre Bodennematoden sind zum Beispiel zystenbildende Nematoden der Gattungen Heterodera oder Globodera, und/oder Wurzelgallennematoden der Gattung Meloidogyne. Schädliche Arten dieser Gattungen sind zum Beispiel Meloidogyne incognita, Heterodera glycines (Sojabohnenzystennematode), Globodera pallida und Globodera rostochiensis (Kartoffelzystennematode), wobei diese Arten wirksam mit dem im vorliegenden Text beschriebenen Verbindungen bekämpft werden. Die Verwendung der im vorliegenden Text beschriebenen Verbindungen ist jedoch keineswegs auf diese Gattungen oder Arten beschränkt, sondern erstreckt sich in gleicher Weise auch auf andere Nematoden.

Zu den Pflanzennematoden zählen z.B. Aglenchus agricola, Anguina tritici, Aphelenchoides arachidis, Aphelenchoides fragaria und die Stängel- und Blattendoparasiten Aphelenchoides spp., Belonolaimus gracilis, Belonolaimus longicaudatus, Belonolaimus nortoni, Bursaphelenchus cocophilus, Bursaphelenchus eremus, Bursaphelenchus xylophilus und Bursaphelenchus spp., Cacopaurus pestis, Criconemella curvata, Criconemella onoensis, Criconemella ornata, Criconemella rusium, Criconemella xenoplax (= Mesocriconema xenoplax) und Criconemella spp.,

Criconemoides ferniae, Criconemoides onoense, Criconemoides ornatum und Criconemoides spp., Ditylenchus destructor, Ditylenchus dipsaci, Ditylenchus myceliophagus sowie die Stängel- und Blattendoparasiten Ditylenchus spp., Dolichodorus heterocephalus, Globodera pallida (=Heterodera pallida), Globodera rostochiensis (Kartoffelzystennematode), Globodera solanacearum, Globodera tabacum, Globodera virginia und die ortsgebundenen zystenbildenden Parasiten Globodera spp., Helicotylenchus digonicus, Helicotylenchus dihystera, Helicotylenchus erythrine, Helicotylenchus multicinctus, Helicotylenchus nannus, Helicotylenchus pseudorobustus und Helicotylenchus spp., Hemicriconemoides, Hemicycliophora arenaria, Hemicycliophora nudata, Hemicycliophora parvana, Heterodera avenae, Heterodera cruciferae, Heterodera glycines (Sojabohnenzystennematode), Heterodera oryzae, Heterodera schachtii, Heterodera zeae und die ortsgebundenen zystenbildenden Parasiten Heterodera spp., Hirschmaniella gracilis, Hirschmaniella oryzae, Hirschmaniella spinicaudata und die Stängel- und Blattendoparasiten Hirschmaniella spp., Hoplolaimus aegyptii, Hoplolaimus californicus, Hoplolaimus columbus, Hoplolaimus galeatus, Hoplolaimus indicus, Hoplolaimus magnistylus, Hoplolaimus pararobustus, Longidorus africanus, Longidorus breviannulatus, Longidorus elongatus, Longidorus laevicapitatus, Longidorus vineacola und die Ektoparasiten Longidorus spp., Meloidogyne acronea, Meloidogyne africana, Meloidogyne arenaria, Meloidogyne arenaria thamesi, Meloidogyne artiella, Meloidogyne chitwoodi, Meloidogyne coffeicola, Meloidogyne ethiopica, Meloidogyne exigua, Meloidogyne fallax, Meloidogyne graminicola, Meloidogyne graminis, Meloidogyne hapla, Meloidogyne incognita, Meloidogyne incognita acrita, Meloidogyne javanica, Meloidogyne kikuyensis, Meloidogyne minor, Meloidogyne naasi, Meloidogyne paranaensis, Meloidogyne thamesi und die ortsgebundenen Parasiten Meloidogyne spp., Meloinema spp., Nacobbus aberrans, Neotylenchus vigissi, Paraphelenchus pseudoparietinus, Paratrichodorus allius, Paratrichodorus lobatus, Paratrichodorus minor, Paratrichodorus nanus, Paratrichodorus porosus, Paratrichodorus teres und Paratrichodorus spp., Paratylenchus hamatus, Paratylenchus minutus, Paratylenchus projectus und Paratylenchus spp., Pratylenchus agilis, Pratylenchus alleni, Pratylenchus andinus, Pratylenchus brachyurus, Pratylenchus cerealis, Pratylenchus coffeae, Pratylenchus crenatus, Pratylenchus delattrei, Pratylenchus giibbicaudatus, Pratylenchus goodeyi, Pratylenchus hamatus, Pratylenchus hexincisus, Pratylenchus loosi, Pratylenchus neglectus, Pratylenchus penetrans,Pratylenchus pratensis, Pratylenchus scribneri, Pratylenchus teres, Pratylenchus thornei, Pratylenchus vulnus, Pratylenchus zeae und die migratorischen Endoparasiten Pratylenchus spp., Pseudohalenchus minutus, Psilenchus magnidens, Psilenchus tumidus, Punctodera chalcoensis, Quinisulcius acutus, Radopholus citrophilus, Radopholus similis, die migratorischen Endoparasiten Radopholus spp., Rotylenchulus borealis, Rotylenchulus parvus, Rotylenchulus reniformis und Rotylenchulus spp., Rotylenchus laurentinus, Rotylenchus macrodoratus, Rotylenchus robustus, Rotylenchus uniformis und Rotylenchus spp., Scutellonema brachyurum, Scutellonema bradys, Scutellonema clathricaudatum und die migratorischen Endoparasiten Scutellonema spp., Subanguina radiciola, Tetylenchus nicotianae, Trichodorus cylindricus, Trichodorus minor, Trichodorus primitivus, Trichodorus proximus, Trichodorus similis, Trichodorus sparsus und die Ektoparasiten Trichodorus spp., Tylenchorhynchus agri, Tylenchorhynchus brassicae, Tylenchorhynchus clarus, Tylenchorhynchus claytoni, Tylenchorhynchus digitatus, Tylenchorhynchus ebriensis, Tylenchorhynchus maximus, Tylenchorhynchus nudus, Tylenchorhynchus vulgaris und Tylenchorhynchus spp., Tylenchulus semipenetrans und die Semiparasiten Tylenchulus spp., Xiphinema americanum, Xiphinema brevicolle, Xiphinema dimorphicaudatum, Xiphinema index und die Ektoparasiten Xiphinema spp.

Zu den Nematoden, zu deren Bekämpfung eine Verbindung der Formel (I) eingesetzt werden kann, zählen Nematoden der Gattung Meloidogyne wie der Southern Root-Knot Nematode (Meloidogyne incognita), der Javanese Root-Knot Nematode (Meloidogyne javanica, der Northern Root-Knot Nematode (Meloidogyne hapla) und der Peanut Root-Knot Nematode (Meloidogyne arenaria); Nematoden der Gattung Ditylenchus wie das Kartoffelkrätzeälchen (Ditylenchus destructor) und das Stock- und Stängelälchen (Ditylenchus dipsaci); Nematoden der Gattung Pratylenchus wie der Cob Root-Lesion Nematode (Pratylenchus penetrans), der Chrysanthemum Root-Lesion Nematode (Pratylenchus fallax), der Kaffeewurzelnematode (Pratylenchus coffeae), der Teewurzelnematode (Pratylenchus loosi) und der Walnut Root-Lesion Nematode (Pratylenchus vulnus); Nematoden der Gattung Globodera wie das Goldfarbene Kartoffelzystenälchen (Globodera rostochiensis) und das Weiße Kartoffelzystenälchen (Globodera pallida); Nematoden der Gattung Heterodera wie der Sojabohnenzystennematode (Heterodera glycines) und das Rübenzystenälchen (Heterodera schachtii); Nematoden der Gattung Aphelenchoides wie der Rice White-tip Nematode (Aphelenchoides besseyi), das Chrysanthemenälchen (Aphelenchoides ritzemabosi) und das Erdbeerälchen (Aphelenchoides fragariae); Nematoden der Gattung Aphelenchus wie der fungivore Nematode (Aphelenchus avenae); Nematoden der Gattung Radopholus, wie der Burrowing-Nematode (Radopholus similis); Nematoden der Gattung Tylenchulus wie der Orangenwurzelnematode (Tylenchulus semipenetrans); Nematoden der Gattung Rotylenchulus wie der reniforme Nematode (Rotylenchulus reniformis); in Bäumen lebende Nematoden, wie der Kiefernholznematode (Bursaphelenchus xylophilus) und der Red Ring Nematode (Bursaphelenchus cocophilus) und dergleichen.

Zu den Pflanzen, zu deren Schutz eine Verbindung der Formel (I) verwendet werden kann, zählen Pflanzen wie Getreide (zum Beispiel Reis, Gerste, Weizen, Roggen, Hafer, Mais, und dergleichen), Bohnen (Sojabohne, Azukibohne, Bohne, Dicke Bohne, Erbsen, Erdnüsse und dergleichen), Obstbäume/Früchte (Äpfel, Zitrusarten, Birnen, Trauben, Pfirsiche, japanische Aprikosen, Kirschen, Walnüsse, Mandeln, Bananen, Erdbeeren und dergleichen), Gemüsearten (Kohl, Tomate, Spinat, Brokkoli, Salat, Zwiebel, Röhrenlauch, Pfeffer und dergleichen), Hackfrüchte (Karotte, Kartoffel, Süßkartoffel, Rettich, Lotuswurzel, Steckrübe und dergleichen), Pflanzen für industrielle Rohstoffe (Baumwolle, Hanf, Papiermaulbeere, Mitsumata, Raps, Rübe, Hopfen, Zuckerrohr, Zuckerrübe, Olive, Gummi, Palmen, Kaffee, Tabak, Tee und dergleichen), Kürbisgewächse (Kürbis, Gurke, Wassermelone, Melone und dergleichen), Weidepflanzen (Knaulgras, Sorgum, Wiesenlieschgras, Klee, Luzerne und dergleichen), Rasengräser (Maskarenengras, Straußgras und dergleichen), Gewürzpflanzen usw. (Lavendel, Rosmarin, Thymian, Petersilie, Pfeffer, Ingwer und dergleichen) und Blumenpflanzen (Chrysantheme, Rose, Orchidee und dergleichen) erwähnt werden.

Die Verbindungen der Formel (I) eignen sich besonders für die Bekämpfung von Nematoden des Kaffees, insbesondere von Pratylenchus brachyurus, Pratylenchus coffeae, Meloidogyne exigua, Meloidogyne incognita, Meloidogyne coffeicola, Helicotylenchus spp. sowie aus Meloidogyne paranaensis, Rotylenchus spp., Xiphinema spp., Tylenchorhynchus spp. und Scutellonema spp..

Die Verbindungen der Formel (I) eignen sich besonders für die Bekämpfung von Nematoden der Kartoffel, insbesondere von Pratylenchus brachyurus, Pratylenchus pratensis, Pratylenchus scribneri, Pratylenchus penetrans, Pratylenchus coffeae, Ditylenchus dipsaci sowie aus Pratylenchus alleni, Pratylenchus andinus, Pratylenchus cerealis, Pratylenchus crenatus, Pratylenchus hexincisus, Pratylenchus loosi, Pratylenchus neglectus, Pratylenchus teres, Pratylenchus thornei, Pratylenchus vulnus, Belonolaimus longicaudatus, Trichodorus cylindricus, Trichodorus primitivus, Trichodorus proximus, Trichodorus similis, Trichodorus sparsus, Paratrichodorus minor, Paratrichodorus allius, Paratrichodorus nanus, Paratrichodorus teres, Meloidogyne arenaria, Meloidogyne fallax, Meloidogyne hapla, Meloidogyne thamesi, Meloidogyne incognita, Meloidogyne chitwoodi, Meloidogyne javanica, Nacobbus aberrans, Globodera rostochiensis, Globodera pallida, Ditylenchus destructor, Radopholus similis, Rotylenchulus reniformis, Neotylenchus vigissi, Paraphelenchus pseudoparietinus, Aphelenchoides fragariae und Meloinema spp.

Die Verbindungen der Formel (I) eignen sich besonders für die Bekämpfung von Nematoden der Tomate, insbesondere von Meloidogyne arenaria, Meloidogyne hapla, Meloidogyne javanica, Meloidogyne incognita, Pratylenchus penetrans und aus Pratylenchus brachyurus, Pratylenchus coffeae, Pratylenchus scribneri, Pratylenchus vulnus, Paratrichodorus minor, Meloidogyne exigua, Nacobbus aberrans, Globodera solanacearum, Dolichodorus heterocephalus und Rotylenchulus reniformis.

Die Verbindungen der Formel (I) eignen sich besonders für die Bekämpfung von Nematoden von Gurkengewächsen, insbesondere von Meloidogyne arenaria, Meloidogyne hapla, Meloidogyne javanica, Meloidogyne incognita, Rotylenchulus reniformis und Pratylenchus thornei.

Die Verbindungen der Formel (I) eignen sich besonders für die Bekämpfung von Nematoden der Baumwolle, insbesondere von Belonolaimus longicaudatus, Meloidogyne incognita, Hoplolaimus columbus, Hoplolaimus galeatus und Rotylenchulus reniformis.

Die Verbindungen der Formel (I) eignen sich besonders für die Bekämpfung von Nematoden des Maises, insbesondere von Belonolaimus longicaudatus, Paratrichodorus minor und aus Pratylenchus brachyurus, Pratylenchus delattrei, Pratylenchus hexincisus, Pratylenchus penetrans, Pratylenchus zeae, (Belonolaimus gracilis), Belonolaimus nortoni, Longidorus breviannulatus, Meloidogyne arenaria, Meloidogyne arenaria thamesi, Meloidogyne graminis, Meloidogyne incognita, Meloidogyne incognita acrita, Meloidogyne javanica, Meloidogyne naasi, Heterodera avenae, Heterodera oryzae, Heterodera zeae, Punctodera chalcoensis, Ditylenchus dipsaci, Hoplolaimus aegyptii, Hoplolaimus magnistylus, Hoplolaimus galeatus, Hoplolaimus indicus, Helicotylenchus digonicus, Helicotylenchus dihystera, Helicotylenchus pseudorobustus, Xiphinema americanum, Dolichodorus heterocephalus, Criconemella ornata, Criconemella onoensis, Radopholus similis, Rotylenchulus borealis, Rotylenchulus parvus, Tylenchorhynchus agri, Tylenchorhynchus clarus, Tylenchorhynchus claytoni, Tylenchorhynchus maximus, Tylenchorhynchus nudus, Tylenchorhynchus vulgaris, Quinisulcius acutus, Paratylenchus minutus, Hemicycliophora parvana, Aglenchus agricola, Anguina tritici, Aphelenchoides arachidis, Scutellonema brachyurum und Subanguina radiciola.

Die Verbindungen der Formel (I) eignen sich besonders für die Bekämpfung von Nematoden der Sojabohne, insbesondere vonPratylenchus brachyurus, Pratylenchus pratensis, Pratylenchus penetrans, Pratylenchus scribneri, Belonolaimus longicaudatus, Heterodera glycines, Hoplolaimus columbus und aus Pratylenchus coffeae, Pratylenchus hexincisus, Pratylenchus neglectus, Pratylenchus crenatus, Pratylenchus alleni, Pratylenchus agilis, Pratylenchus zeae, Pratylenchus vulnus, (Belonolaimus gracilis), Meloidogyne arenaria, Meloidogyne incognita, Meloidogyne javanica, Meloidogyne hapla, Hoplolaimus columbus, Hoplolaimus galeatus und Rotylenchulus reniformis.

Die Verbindungen der Formel (I) eignen sich besonders für die Bekämpfung von Nematoden des Tabaks, insbesondere von Meloidogyne incognita, Meloidogyne javanica und aus Pratylenchus brachyurus, Pratylenchus pratensis, Pratylenchus hexincisus, Pratylenchus penetrans, Pratylenchus neglectus, Pratylenchus crenatus, Pratylenchus thornei, Pratylenchus vulnus, Pratylenchus zeae, Longidorus elongatu, Paratrichodorus lobatus, Trichodorus spp., Meloidogyne arenaria, Meloidogyne hapla, Globodera tabacum, Globodera solanacearum, Globodera virginiae, Ditylenchus dipsaci, Rotylenchus spp., Helicotylenchus spp., Xiphinema americanum, Criconemella spp., Rotylenchulus reniformis, Tylenchorhynchus claytoni, Paratylenchus spp. und Tetylenchus nicotianae.

Die Verbindungen der Formel (I) eignen sich besonders für die Bekämpfung von Nematoden von Zitrusgewächsen, insbesondere von Pratylenchus coffeae und aus Pratylenchus brachyurus, Pratylenchus vulnus, Belonolaimus longicaudatus, Paratrichodorus minor, Paratrichodorus porosus, Trichodorus , Meloidogyne incognita, Meloidogyne incognita acrita, Meloidogyne javanica, Rotylenchus macrodoratus, Xiphinema americanum, Xiphinema brevicolle, Xiphinema index, Criconemella spp., Hemicriconemoides, Radopholus similis bzw. Radopholus citrophilus, Hemicycliophora arenaria, Hemicycliophora nudata und Tylenchulus semipenetrans .

Die Verbindungen der Formel (I) eignen sich besonders für die Bekämpfung von Nematoden der Banane, insbesondere von Pratylenchus coffeae, Radopholus similis und aus Pratylenchus giibbicaudatus, Pratylenchus loosi, Meloidogyne spp., Helicotylenchus multicinctus, Helicotylenchus dihystera und Rotylenchulus spp..

Die Verbindungen der Formel (I) eignen sich besonders für die Bekämpfung von Nematoden der Ananas, insbesondere von Pratylenchus zeae, Pratylenchus pratensis, Pratylenchus brachyurus, Pratylenchus goodeyi., Meloidogyne spp., Rotylenchulus reniformis und aus Longidorus elongatus, Longidorus laevicapitatus, Trichodorus primitivus, Trichodorus minor, Heterodera spp., Ditylenchus myceliophagus, Hoplolaimus californicus, Hoplolaimus pararobustus, Hoplolaimus indicus, Helicotylenchus dihystera, Helicotylenchus nannus, Helicotylenchus multicinctus, Helicotylenchus erythrine, Xiphinema dimorphicaudatum, Radopholus similis, Tylenchorhynchus digitatus, Tylenchorhynchus ebriensis, Paratylenchus minutus, Scutellonema clathricaudatum, Scutellonema bradys, Psilenchus tumidus, Psilenchus magnidens, Pseudohalenchus minutus, Criconemoides ferniae, Criconemoides onoense und Criconemoides ornatum .

Die Verbindungen der Formel (I) eignen sich besonders für die Bekämpfung von Nematoden von Trauben, insbesondere von Pratylenchus vulnus, Meloidogyne arenaria, Meloidogyne incognita, Meloidogyne javanica, Xiphinema americanum, Xiphinema index und aus Pratylenchus pratensis, Pratylenchus scribneri, Pratylenchus neglectus, Pratylenchus brachyurus, Pratylenchus thornei und Tylenchulus semipenetrans.

Die Verbindungen der Formel (I) eignen sich besonders für die Bekämpfung von Nematoden von Baumkulturen - Kernobst, insbesondere von Pratylenchus penetrans und aus Pratylenchus vulnus, Longidorus elongatus, Meloidogyne incognita und Meloidogyne hapla.

Die Verbindungen der Formel (I) eignen sich besonders für die Bekämpfung von Nematoden von Baumkulturen - Steinfrüchten, insbesondere von Pratylenchus penetrans, Pratylenchus vulnus, Meloidogyne arenaria, Meloidogyne hapla, Meloidogyne javanica, Meloidogyne incognita, Criconemella xenoplax und aus Pratylenchus brachyurus, Pratylenchus coffeae, Pratylenchus scribneri, Pratylenchus zeae, Belonolaimus longicaudatus, Helicotylenchus dihystera, Xiphinema americanum, Criconemella curvata, Tylenchorhynchus claytoni, Paratylenchus hamatus, Paratylenchus projectus, Scutellonema brachyurum und Hoplolaimus galeatus.

Die Verbindungen der Formel (I) eignen sich besonders für die Bekämpfung von Nematoden in Baumkulturen, Zuckerrohr und Reis, insbesondere von Trichodorus spp., Criconemella spp. und aus Pratylenchus spp. , Paratrichodorus spp., Meloidogyne spp. , Helicotylenchus spp., Tylenchorhynchus spp., Aphelenchoides spp. Heterodera spp, Xiphinema spp. und Cacopaurus pestis.

Ebenso bezieht sich der Begriff "Nematoden" im vorliegenden Zusammenhang auf Nematoden, die Menschen oder Tiere schädigen.

Spezifische Nematodenarten, die für den Menschen oder für Tiere schädlich sind, sind:
Trichinellida, zum Beispiel: Trichuris spp., Capillaria spp., Paracapillaria spp., Eucoleus spp., Trichomosoides spp., Trichinella spp.

Aus der Ordnung der Tylenchida zum Beispiel: Micronema spp., Strongyloides spp.

Aus der Ordnung der Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.

Aus der Odnung der Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;

Viele bekannte Nematizide wirken gleichsam gegen andere parasitäre Helminthen und werden daher für die Bekämpfung von human- und tierparasitären Würmern, die nicht unbedingt zu der Gruppe Nematoda gehören, verwendet. Die vorliegende Erfindung bezieht sich auch auf die Verwendung der Verbindungen der Formel (I) als anthelmintische Arzneimittel. Zu den pathogenen endoparasitären Helminthen zählen Platyhelmintha (z.B. Monogenea, Cestodes und Trematodes), Acanthocephala und Pentastoma. Die folgenden Helminthen sind als bevorzugt zu erwähnen:
Monogenea: z.B.: Gyrodactylus spp., Dactylogyrus spp., Polystoma spp.

Cestodes: aus der Ordnung der Pseudophyllidea zum Beispiel: Diphyllobothrium spp., Spirometra spp., Schistocephalus spp., Ligula spp., Bothridium spp., Diplogonoporus spp.

Aus der Ordnung der Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.

Trematodes: aus der Klasse der Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonimus spp.

Acanthocephala: aus der Ordnung der Oligacanthorhynchida z.B.: Macracanthorhynchus spp., Prosthenorchis spp.; aus der Ordnung der Polymorphida zum Beispiel: Filicollis spp.; aus der Ordnung der Moniliformida zum Beispiel: Moniliformis spp.,

Aus der Ordnung der Echinorhynchida zum Beispiel Acanthocephalus spp., Echinorhynchus spp., Leptorhynchoides spp.

Pentastoma: aus der Ordnung der Porocephalida zum Beispiel Linguatula spp.

Auf dem Gebiet der Tiermedizin und in der Tierhaltung erfolgt die Verabreichung der Verbindungen der Formel (I) auf bekannte Weise direkt oder enteral, parenteral, dermal oder nasal in Form von geeigneten Anwendungsformen. Die Verabreichung kann prophylaktisch oder therapeutisch erfolgen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)-Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, CrylAc, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide,
weitere Wirkstoffe wie beispielsweise Afidopyropen, Azadirachtin, Benclothiaz, Benzoximate, Bifenazate, Bromopropylate, Chinomethionat, Cryolite,

Dicofol, Diflovidazin, Fluensulfone, Flometoquin, Flufenerim, Flufenoxystrobin, Flufiprole, Fluopyram, Flupyradifurone, Fufenozide, Heptafluthrin, Imidaclothiz, Iprodione, Meperfluthrin, Paichongding, Pyflubumide, Pyrifluquinazon, Pyriminostrobin, Tetramethylfluthrin und Iodmethan; desweiteren Präparate auf Basis von Bacillus firmus (I-1582, BioNeem, Votivo), sowie folgende Verbindungen:3-Brom-N- {2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) und 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{5-[3-Chlor-5-(trifluormethyl)phenyl]-5-(trifluormethyl)-4,5-dihydro-1,2-oxazol-3-yl}-N-{2-oxo-2-[(2,2,2-trifluorethyl)amino]ethyl}-1-naphthamid (bekannt aus WO2009/002809), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 3-Chlor-N-(2-cyanpropan-2-yl)-N-[4-(1,1,1,2,3,3,3-heptafluorpropan-2-yl)-2-methylphenyl]phthalamid (bekannt aus WO2012/034472), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-*N*-(1-oxidothietan-3-yl)benzamid (bekannt aus WO2009/080250), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), (5S,8R)-1-[(6-Chlorpyridin-3-yl)methyl]-9-nitro-2,3,5,6,7,8-hexahydro-1H-5,8-epoxyimidazo[1,2-a]azepin (bekannt aus WO2010/069266), (2E)-1-[(6-Chlorpyridin-3-yl)methyl]-N'-nitro-2-pentylidenhydrazincarboximidamid (bekannt aus WO2010/060231), 4-(3-{2,6-Dichlor-4-[(3,3-dichlorprop-2-en-1-yl)oxylphenoxylpropoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).
(1) Inhibitoren der Ergosterol-Biosynthese, wie beispielsweise (1.1) Aldimorph, (1.2) Azaconazol, (1.3) Bitertanol, (1.4) Bromuconazol, (1.5) Cyproconazol, (1.6) Diclobutrazol, (1.7) Difenoconazol, (1.8) Diniconazol, (1.9) Diniconazol-M, (1.10) Dodemorph, (1.11) Dodemorph Acetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-Cis, (1.25) Hexaconazol, (1.26) Imazalil, (1.27) Imazalil Sulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) Methyl-1-(2,2-dimethyl-2,3-dihydro-1H-inden-1-yl)-1H-imidazol-5-carboxylat, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat, (1.65) Pyrisoxazole.
(2) Inhibitoren der Respiration (Atmungsketten-Inhibitoren), wie beispielsweise (2.1) Bixafen, (2.2) Boscalid, (2.3) Carboxin, (2.4) Diflumetorim, (2.5) Fenfuram, (2.6) Fluopyram, (2.7) Flutolanil, (2.8) Fluxapyroxad, (2.9) Furametpyr, (2.10) Furmecyclox, (2.11) Isopyrazam Mischung des syn-epimeren Razemates 1RS,4SR,9RS und des anti-empimeren Razemates 1RS,4SR,9SR, (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxane, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]quinazolin-4-amin, (2.29) Benzovindiflupyr, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridine-3-carboxamid, (2.43) Isofetamid
(3) Inhibitoren der Respiration (Atmungsketten-Inhibitoren) am Komplex III der Atumungskette, wie beispielsweise (3.1) Ametoctradin, (3.2) Amisulbrom, (3.3) Azoxystrobin, (3.4) Cyazofamid, (3.5) Coumethoxystrobin, (3.6) Coumoxystrobin, (3.5) Dimoxystrobin, (3.8) Enestroburin, (3.9) Famoxadon, (3.10) Fenamidon, (3.11) Flufenoxystrobin, (3.12) Fluoxastrobin, (3.13) Kresoxim-Methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylethanamid, (3.24) (2E)-2-(Methoxyimino)-N-methyl-2-(2-{[({(1E)-1-[3-(trifluormethyl)phenyl]ethyliden}amino)oxy]methyl}phenyl)ethanamid, (3.25) (2E)-2-(Methoxyimino)-N-methyl-2-{2-[(E)-({1-[3-(trifluormethyl)phenyl]ethoxy}imino)methyl]phenyl}ethanamid, (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid, (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat, (3.31) N-(3-Ethyl-3,5,5-trimethylcyclohexyl)-3-(formylamino)-2-hydroxybenzamid, (3.32) 2-{2-[(2,5-Dimethylphenoxy)methyl]phenyl}-2-methoxy-N-methylacetamid,
(4) Inhibitoren der Mitose und Zellteilung, wie beispielsweise (4.1) Benomyl, (4.2) Carbendazim, (4.3) Chlorfenazol, (4.4) Diethofencarb, (4.5) Ethaboxam, (4.6) Fluopicolid, (4.7) Fuberidazol, (4.8) Pencycuron, (4.9) Thiabendazol, (4.10) Thiophanat-Methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin und (4.14) 3-Chlor-5-(6-chlorpyridin-3-yl)-6-methyl-4-(2,4,6-trifluorphenyl)pyridazin.
(5) Verbindungen mit Multisite-Aktivität, wie beispielsweise (5.1) Bordeauxmischung, (5.2) Captafol, (5.3) Captan, (5.4) Chlorthalonil, (5.5) Kupferzubereitungen wie Kupferhydroxid, (5.6) Kupfernaphthenat, (5.7) Kupferoxid, (5.8) Kupferoxychlorid, (5.9) Kupfersulfat, (5.10) Dichlofluanid, (5.11) Dithianon, (5.12) Dodine, (5.13) Dodine freie Base, (5.14) Ferbam, (5.15) Fluorfolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mankupfer, (5.23) Mancozeb, (5.24) Maneb, (5.25) Metiram, (5.26) Zinkmetiram, (5.27) Kupfer-Oxin, (5.28) Propamidin, (5.29) Propineb, (5.30) Schwefel und Schwefelzubereitungen wie beispielsweise Calciumpolysulfid, (5.31) Thiram, (5.32) Tolylfluanid, (5.33) Zineb, (5.34) Ziram und (5,35) Anilazin.
(6) Resistenzinduktoren, wie beispielsweise (6.1) Acibenzolar-S-Methyl, (6.2) Isotianil, (6.3) Probenazol, (6.4) Tiadinil und (6.5) Laminarin.
(7) Inhibitoren der Aminosäure- und Protein-Biosynthese, wie beispielsweise (7.1) Andoprim, (7.2) Blasticidin-S, (7.3) Cyprodinil, (7.4) Kasugamycin, (7.5) Kasugamycin Hydrochlorid Hydrat, (7.6) Mepanipyrim, (7.7) Pyrimethanil, (7.8) 3-(5-Fluor-3,3,4,4-tetramethyl-3,4-dihydroisochinolin-1-yl)chinolin und (7.9) Oxytetracyclin und (7.10) Streptomycin.
(8) Inhibitoren der ATP Produktion, wie beispielsweise (8.1) Fentin Acetat, (8.2) Fentin Chlorid, (8.3) Fentin Hydroxid und (8.4) Silthiofam.
(9) Inhibitoren der Zellwandsynthese, wie beispielsweise (9.1) Benthiavalicarb, (9.2) Dimethomorph, (9.3) Flumorph, (9.4) Iprovalicarb, (9.5) Mandipropamid, (9.6) Polyoxins, (9.7) Polyoxorim, (9.8) Validamycin A, (9.9) Valifenalat und (9.10) Polyoxin B.
(10) Inhibitoren der Lipid- und Membran-Synthese, wie beispielsweise (10.1) Biphenyl, (10.2) Chlorneb, (10.3) Dicloran, (10.4) Edifenphos, (10.5) Etridiazol, (10.6) Iodocarb, (10.7) Iprobenfos, (10.8) Isoprothiolan, (10.9) Propamocarb, (10.10) Propamocarb Hydrochlorid, (10.11) Prothiocarb,, (10.12) Pyrazophos, (10.13) Quintozen, (10.14) Tecnazene und (10.15) Tolclofos-Methyl.
(11) Inhibitoren der Melanin-Biosynthese, wie beispielsweise (11.1) Carpropamid, (11.2) Diclocymet, (11.3) Fenoxanil, (11.4) Fthalid, (11.5) Pyroquilon, (11.6) Tricyclazol, und (11.7) 2,2,2-Trifluorethyl {3-methyl-1-[(4-methylbenzoyl)amino]butan-2-yl}carbamat.
(12) Inhibitoren der Nukleinsäuresynthese, wie beispielsweise (12.1) Benalaxyl, (12.2) Benalaxyl-M (Kiralaxyl), (12.3) Bupirimat, (12.4) Clozylacon, (12.5) Dimethirimol, (12.6) Ethirimol, (12.7) Furalaxyl, (12.8) Hymexazol, (12.9) Metalaxyl, (12.10) Metalaxyl-M (Mefenoxam), (12.11) Ofurace, (12.12) Oxadixyl, (12.13) Oxolinsäure und (12.14) Octhilinon.
(13) Inhibitoren der Signaltransduktion, wie beispielsweise (13.1) Chlozolinat, (13.2) Fenpiclonil, (13.3) Fludioxonil, (13.4) Iprodion, (13.5) Procymidon, (13.6) Quinoxyfen, (13.7) Vinclozolin und (13.8) Proquinazid.
(14) Entkoppler, wie beispielsweise (14.1) Binapacryl, (14.2) Dinocap, (14.3) Ferimzon, (14.4) Fluazinam und (14.5) Meptyldinocap.
(15) Weitere Verbindungen, wie beispielsweise (15.1) Benthiazol, (15.2) Bethoxazin, (15.3) Capsimycin, (15.4) Carvon, (15.5) Chinomethionat, (15.6) Pyriofenon (Chlazafenon), (15.7) Cufraneb, (15.8) Cyflufenamid, (15.9) Cymoxanil, (15.10) Cyprosulfamid, (15.11) Dazomet, (15.12) Debacarb, (15.13) Dichlorphen, (15.14) Diclomezin, (15.15) Difenzoquat, (15.16) Difenzoquat Methylsulphat, (15.17) Diphenylamin, (15.18) Ecomat, (15.19) Fenpyrazamin, (15.20) Flumetover, (15.21) Fluorimid, (15.22) Flusulfamid, (15.23) Flutianil, (15.24) Fosetyl-Aluminium, (15.25) Fosetyl-Calcium, (15.26) Fosetyl-Natrium, (15.27) Hexachlorbenzol, (15.28) Irumamycin, (15.29) Methasulfocarb, (15.30) Methylisothiocyanat, (15.31) Metrafenon, (15.32) Mildiomycin, (15.33) Natamycin, (15.34) Nickel Dimethyldithiocarbamat, (15.35) Nitrothal-Isopropyl, (15.36) Octhilinone, (15.37) Oxamocarb, (15.38) Oxyfenthiin, (15.39) Pentachlorphenol und dessen Salze, (15.40) Phenothrin, (15.41) Phosphorsäure und deren Salze, (15.42) Propamocarb-Fosetylat, (15.43) Propanosin-Natrium, (15.44) Pyrimorph, (15.45) (2E)-3-(4-Tert-butylphenyl)-3-(2-chlorpyridin-4-yl)-1-(morpholin-4-yl)prop-2-en-1-on, (15.46) (2Z)-3-(4-Tert-butylphenyl)-3-(2-chlorpyridin-4-yl)-1-(morpholin-4-yl)prop-2-en-1-on, (15.47) Pyrrolnitrin, (15.48) Tebufloquin, (15.49) Tecloftalam, (15.50) Tolnifanide, (15.51) Triazoxid, (15.52) Trichlamid, (15.53) Zarilamid, (15.54) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, (15.55) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.56) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.57) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.58) 1-(4-Methoxyphenoxy)-3,3-dimethylbutan-2-yl 1H-imidazole-1-carboxylat, (15.59) 2,3,5,6-Tetrachlor-4-(methylsulfonyl)pyridin, (15.60) 2,3-Dibutyl-6-chlorthieno[2,3-d]pyrimidin-4(3H)-on, (15.61) 2,6-Dimethyl-1H,5H-[1,4]dithiino[2,3-c:5,6-c']dipyrrole-1,3,5,7(2H,6H)-tetron, (15.62) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.63) 2-[5-Methyl-3-(trifluormethyl)-1H-pyrazol-1-yl]-1-(4-14-[(5S)-5-phenyl-4,5-dihydro-1,2-oxazol-3-yl]-1,3-thiazol-2-yl}piperidin-1-yl)ethanon, (15.64) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.65) 2-Butoxy-6-iodo-3-propyl-4H-chromen-4-on, (15.66) 2-Chlor-5-[2-chlor-1-(2,6-difluor-4-methoxyphenyl)-4-methyl-1H-imidazo1-5-yl]pyridin, (15.67) 2-Phenylphenol und Salze, (15.68) 3-(4,4,5-Trifluor-3,3-dimethyl-3,4-dihydroisochinolin-1-yl)chinolin, (15.69) 3,4,5-Trichlorpyridine-2,6-dicarbonitril, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazole-2-thiol, (15.73) 5-Chlor-N'-phenyl-N'-(prop-2-yn-1-yl)thiophene-2-sulfonohydrazid, (15.74) 5-Fluor-2-[(4-fluorbenzyl)oxy]pyrimidin-4-amin, (15.75) 5-Fluor-2-[(4-methylbenzyl)oxy]pyrimidin-4-amin, (15.76) 5-Methyl-6-octyl[1,2,4]triazolo[1,5-a]pyrimidin-7-amin, (15.77) Ethyl (2Z)-3-amino-2-cyano-3-phenylacrylat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-yn-1-yloxy)phenyl]propanamid, (15.80) N-[(4-Chlorphenyl)(cyano)methyl]-3-[3-methoxy-4-(prop-2-yn-1-yloxy)phenyl]propanamid, (15.81) N-[(5-Brom-3-chlorpyridin-2-yl)methyl]-2,4-dichlornicotinamid, (15.82) N-[1-(5-Brom-3-chlorpyridin-2-yl)ethyl]-2,4-dichlornicotinamid, (15.83) N-[1-(5-Brom-3-chlorpyridin-2-yl)ethyl]-2-fluor-4-iodonicotinamid, (15.84) N-{(E)-[(Cyclopropylmethoxy)imino][6-(difluormethoxy)-2,3-difluorphenyl]methyl}-2-phenylacetamid, (15.85) N-{(Z)-[(Cyclopropylmethoxy)imino][6-(difluormethoxy)-2,3-difluorphenyl]methyl}-2-phenylacetamid, (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazole-4-carboxamid, (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazole-4-carboxamid, (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazole-4-carboxamid, (15.90) Pentyl {6-[({[(1-methyl-1H-tetrazol-5-yl)(phenyl)methylene] amino}oxy)methyl]pyridin-2-yl}carbamat, (15.91) Phenazine-1-carbonsäure, (15.92) Chinolin-8-ol, (15.93) Chinolin-8-ol sulfate (2:1), (15.94) Tert-butyl {6-[({[(1-methyl-1H-tetrazol-5-yl)(phenyl)methylene]amino}oxy)methyl]pyridin-2-yl}carbamat, (15.95) 1-Methyl-3-(trifluormethyl)-N-[2'-(trifluormethyl)biphenyl-2-yl]-1H-pyrazol-4-carboxamid, (15.96) N-(4'-Chlorbiphenyl-2-yl)-3-(difluormethyl)-1-methyl-1H-pyrazol-4-carboxamid, (15.97) N-(2',4'-Dichlorbiphenyl-2-yl)-3-(difluormethyl)-1-methyl-1H-pyrazol-4-carboxamid, (15.98) 3-(Difluormethyl)-1-methyl-N-[4'-(trifluormethyl)biphenyl-2-yl]-1H-pyrazol-4-carboxamid, (15.99) N-(2',5'-Difluorbiphenyl-2-yl)-1-methyl-3-(trifluormethyl)-1H-pyrazol-4-carboxamid, (15.100) 3-(Difluormethyl)-1-methyl-N-[4'-(prop-1-yn-1-yl)biphenyl-2-yl]-1H-pyrazol-4-carboxamid, (15.101) 5-Fluor-1,3-dimethyl-N-[4'-(prop-1-yn-1-yl)biphenyl-2-yl]-1H-pyrazol-4-carboxamid, (15.102) 2-Chlor-N-[4'-(prop-1-yn-1-yl)biphenyl-2-yl]nicotinamid, (15.103) 3-(Difluormethyl)-N-[4'-(3,3-dimethylbut-1-yn-1-yl)biphenyl-2-yl]-1-methyl-1H-pyrazol-4-carboxamid, (15.104) N-[4'-(3,3-dimethylbut-1-yn-1-yl)biphenyl-2-yl]-5-fluor-1,3-dimethyl-1H-pyrazol-4-carboxamid, (15.105) 3-(Difluormethyl)-N-(4'-ethynylbiphenyl-2-yl)-1-methyl-1H-pyrazol-4-carboxamid, (15.106) N-(4'-Ethynylbiphenyl-2-yl)-5-fluor-1,3-dimethyl-1H-pyrazol-4-carboxamid, (15.107) 2-Chlor-N-(4'-ethynylbiphenyl-2-yl)nicotinamid, (15.108) 2-Chlor-N-[4'-(3,3-dimethylbut-1-yn-1-yl)biphenyl-2-yl]nicotinamid, (15.109) 4-(Difluormethyl)-2-methyl-N-[4'-(trifluormethyl)biphenyl-2-yl]-1,3-thiazole-5-carboxamid, (15.110) 5-Fluor-N-[4'-(3-hydroxy-3-methylbut-1-yn-1-yl)biphenyl-2-yl]-1,3-dimethyl-1H-pyrazol-4-carboxamid, (15.111) 2-Chlor-N-[4'-(3-hydroxy-3-methylbut-1-yn-1-yl)biphenyl-2-yl]nicotinamid, (15.112) 3-(Difluormethyl)-N-[4'-(3-methoxy-3-methylbut-1-yn-1-yl)biphenyl-2-yl]-1-methyl-1H-pyrazol-4-carboxamid, (15.113) 5-Fluor-N-[4'-(3-methoxy-3-methylbut-1-yn-1-yl)biphenyl-2-yl]-1,3-dimethyl-1H-pyrazol-4-carboxamid, (15.114) 2-Chlor-N-[4'-(3-methoxy-3-methylbut-1-yn-1-yl)biphenyl-2-yl]nicotinamid, (15.115) (5-Brom-2-methoxy-4-methylpyridin-3-yl)(2,3,4-trimethoxy-6-methylphenyl)methanon, (15.116) N-[2-(4-{[3-(4-Chlorphenyl)prop-2-yn-1-yl]oxy}-3-methoxyphenyl)ethyl]-N2-(methylsulfonyl)valinamid, (15.117) 4-Oxo-4-[(2-phenylethyl)amino]butansäure, (15.118) But-3-yn-1-yl {6-[({[(Z)-(1-methyl-1H-tetrazol-5-yl)(phenyl)methylene]amino}oxy)methyl]pyridin-2-yl}carbamat, (15.119) 4-Amino-5-fluorpyrimidin-2-ol (Tautomere Form: 4-Amino-5-fluorpyrimidin-2(1H)-on), (15.120) Propyl 3,4,5-trihydroxybenzoat, (15.121) 1,3-Dimethyl-N-(1,1,3-trimethyl-2,3-dihydro-1H-inden-4-yl)-1H-pyrazol-4-carboxamid, (15.122) 1,3-Dimethyl-N-[(3R)-1,1,3-trimethyl-2,3-dihydro-1H-inden-4-yl]-1H-pyrazol-4-carboxamid, (15.123) 1,3-Dimethyl-N-[(3S)-1,1,3-trimethyl-2,3-dihydro-1H-inden-4-yl]-1H-pyrazol-4-carboxamid, (15.124) [3-(4-Chlor-2-fluorphenyl)-5-(2,4-difluorphenyl)-1,2-oxazol-4-yl](pyridin-3-yl)methanol, (15.125) (S)-[3-(4-Chlor-2-fluorphenyl)-5-(2,4-difluorphenyl)-1,2-oxazol-4-yl](pyridin-3-yl)methanol, (15.126) (R)-[3-(4-Chlor-2-fluorphenyl)-5-(2,4-difluorphenyl)-1,2-oxazol-4-yl](pyridin-3-yl)methanol, (15.127) 2-{[3-(2-Chlorphenyl)-2-(2,4-difluorphenyl)oxiran-2-yl]methyl}-2,4-dihydro-3H-1,2,4-triazol-3-thion, (15.128) 1-{[3-(2-Chlorphenyl)-2-(2,4-difluorphenyl)oxiran-2-yl]methyl}-1H-1,2,4-triazol-5-yl thiocyanat, (15.129) 5-(Allylsulfanyl)-1-{[3-(2-chlorphenyl)-2-(2,4-difluorphenyl)oxiran-2-yl]methyl}-1H-1,2,4-triazol, (15.130) 2-[1-(2,4-Dichlorphenyl)-5-hydroxy-2,6,6-trimethylheptan-4-yl] -2,4-dihydro-3H-1,2,4-triazol-3-thion, (15.131) 2-{[rel(2R,3S)-3-(2-Chlorphenyl)-2-(2,4-difluorphenyl)oxiran-2-yl]methyl}-2,4-dihydro-3H-1,2,4-triazol-3-thion, (15.132) 2-{[rel(2R,3R)-3-(2-Chlorphenyl)-2-(2,4-difluorphenyl)oxiran-2-yl]methyl}-2,4-dihydro-3H-1,2,4-triazol-3-thion, (15.133) 1-{[rel(2R,3S)-3-(2-Chlorphenyl)-2-(2,4-difluorphenyl)oxiran-2-yl]methyl} -1H-1,2,4-triazol-5-yl thiocyanat, (15.134) 1-{[rel(2R,3R)-3-(2-Chlorphenyl)-2-(2,4-difluorphenyl)oxiran-2-yl]methyl}-1H-1,2,4-triazol-5-yl thiocyanat, (15.135) 5-(Allylsulfanyl)-1-{[rel(2R,3S)-3-(2-chlorphenyl)-2-(2,4-difluorphenyl)oxiran-2-yl]methyl}-1H-1,2,4-triazol, (15.136) 5-(Allylsulfanyl)-1-{[rel(2R,3R)-3-(2-chlorphenyl)-2-(2,4-difluorphenyl)oxiran-2-yl]methyl}-1H-1,2,4-triazol, (15.137) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (15.138) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (15.139) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (15.140) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (15.141) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (15.142) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (15.143) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (15.144) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (15.145) 2-Fluor-6-(trifluormethyl)-N-(1,1,3-trimethyl-2,3-dihydro-1H-inden-4-yl)benzamid, (15.146) 2-(6-Benzylpyridin-2-yl)quinazolin, (15.147) 2-[6-(3-Fluor-4-methoxyphenyl)-5-methylpyridin-2-yl]quinazolin, (15.148) 3-(4,4-Difluor-3,3-dimethyl-3,4-dihydroisochinolin-1-yl)chinolin, (15.149) Abscisinsäure, (15.150) 3-(Difluormethyl)-N-methoxy-1-methyl-N-[1-(2,4,6-trichlorphenyl)propan-2-yl]-1H-pyrazol-4-carboxamid, (15.151) N'-[5-Brom-6-(2,3-dihydro-1H-inden-2-yloxy)-2-methylpyridin-3-yl]-N-ethyl-N-methylimidoformamid, (15.152) N'-{5-Brom-6-[1-(3,5-difluorphenyl)ethoxy]-2-methylpyridin-3-yl}-N-ethyl-N-methylimidoformamid, (15.153) N'-{5-Brom-6-[(1R)-1-(3,5-difluorphenyl)ethoxy]-2-methylpyridin-3-yl}-N-ethyl-N-methylimidoformamid, (15.154) N'-{5-Brom-6-[(1S)-1-(3,5-difluorphenyl)ethoxy]-2-methylpyridin-3-yl}-N-ethyl-N-methylimidoformamid, (15.155) N'-{5-Brom-6-[(cis-4-isopropylcyclohexyl)oxy] -2-methylpyridin-3-yl}-N-ethyl-N-methylimidoformamid, (15.156) N'-{5-Brom-6-[(trans-4-isopropylcyclohexyl)oxy]-2-methylpyridin-3-yl}-N-ethyl-N-methylimidoformamid, (15.157) N-Cyclopropyl-3-(difluormethyl)-5-fluor-N-(2-isopropylbenzyl)-1-methyl-1H-pyrazol-4-carboxamid, (15.158) N-Cyclopropyl-N-(2-cyclopropylbenzyl)-3-(difluormethyl)-5-fluor-1-methyl-1H-pyrazol-4-carboxamid, (15.159) N-(2-Tert-butylbenzyl)-N-cyclopropyl-3-(difluormethyl)-5-fluor-1-methyl-1H-pyrazol-4-carboxamid, (15.160) N-(5-Chlor-2-ethylbenzyl)-N-cyclopropyl-3-(difluormethyl)-5-fluor-1-methyl-1H-pyrazol-4-carboxamid, (15.161) N-(5-Chlor-2-isopropylbenzyl)-N-cyclopropyl-3-(difluormethyl)-5-fluor-1-methyl-1H-pyrazol-4-carboxamid, (15.162) N-Cyclopropyl-3-(difluormethyl)-N-(2-ethyl-5-fluorbenzyl)-5-fluor-1-methyl-1H-pyrazol-4-carboxamid, (15.163) N-Cyclopropyl-3-(difluormethyl)-5-fluor-N-(5-fluor-2-isopropylbenzyl)-1-methyl-1H-pyrazol-4-carboxamid, (15.164) N-Cyclopropyl-N-(2-cyclopropyl-5-fluorbenzyl)-3-(difluormethyl)-5-fluor-1-methyl-1H-pyrazol-4-carboxamid, (15.165) N-(2-Cyclopentyl-5-fluorbenzyl)-N-cyclopropyl-3-(difluormethyl)-5-fluor-1-methyl-1H-pyrazol-4-carboxamid, (15.166) N-Cyclopropyl-3-(difluormethyl)-5-fluor-N-(2-fluor-6-isopropylbenzyl)-1-methyl-1H-pyrazol-4-carboxamid, (15.167) N-Cyclopropyl-3-(difluormethyl)-N-(2-ethyl-5-methylbenzyl)-5-fluor-1-methyl-1H-pyrazol-4-carboxamid, (15.168) N-Cyclopropyl-3-(difluormethyl)-5-fluor-N-(2-isopropyl-5-methylbenzyl)-1-methyl-1H-pyrazol-4-carboxamid, (15.169) N-Cyclopropyl-N-(2-cyclopropyl-5-methylbenzyl)-3-(difluormethyl)-5-fluor-1-methyl-1H-pyrazol-4-carboxamid, (15.170) N-(2-Tert-butyl-5-methylbenzyl)-N-cyclopropyl-3-(difluormethyl)-5-fluor-1-methyl-1H-pyrazol-4-carboxamid, (15.171) N-[5-Chlor-2-(trifluormethyl)benzyl]-N-cyclopropyl-3-(difluormethyl)-5-fluor-1-methyl-1H-pyrazol-4-carboxamid, (15.172) N-Cyclopropyl-3-(difluormethyl)-5-fluor-1-methyl-N-[5-methyl-2-(trifluormethyl)benzyl]-1H-pyrazol-4-carboxamid, (15.173) N-[2-Chlor-6-(trifluormethyl)benzyl]-N-cyclopropyl-3-(difluormethyl)-5-fluor-1-methyl-1H-pyrazol-4-carboxamid, (15.174) N-[3-Chlor-2-fluor-6-(trifluormethyl)benzyl]-N-cyclopropyl-3-(difluormethyl)-5-fluor-1-methyl-1H-pyrazol-4-carboxamid, (15.175) N-Cyclopropyl-3-(difluormethyl)-N-(2-ethyl-4,5-dimethylbenzyl)-5-fluor-1-methyl-1H-pyrazol-4-carboxamid, (15.176) N-Cyclopropyl-3-(difluormethyl)-5-fluor-N-(2-isopropylbenzyl)-1-methyl-1H-pyrazol-4-carbothioamid, (15.177) 3-(Difluormethyl)-N-(7-fluor-1,1,3-trimethyl-2,3-dihydro-1H-inden-4-yl)-1-methyl-1H-pyrazol-4-carboxamid, (15.178) 3-(Difluormethyl)-N-[(3R)-7-fluor-1,1,3-trimethyl-2,3-dihydro-1H-inden-4-yl]-1-methyl-1H-pyrazol-4-carboxamid, (15.179) 3-(Difluormethyl)-N-[(3S)-7-fluor-1,1,3-trimethyl-2,3-dihydro-1H-inden-4-yl]-1-methyl-1H-pyrazol-4-carboxamid, (15.180) N'-(2,5-Dimethyl-4-phenoxyphenyl)-N-ethyl-N-methylimidoformamid, (15.181) N'-{4-[(4,5-Dichlor-1,3-thiazol-2-yl)oxy]-2,5-dimethylphenyl}-N-ethyl-N-methylimidoformamid, (15.182) N-(4-Chlor-2,6-difluorphenyl)-4-(2-chlor-4-fluorphenyl)-1,3-dimethyl-1H-pyrazol-5-amin. Alle genannten Mischpartner der Klassen (1) bis (15) können, wenn sie auf Grund ihrer funktionellen Gruppen dazu imstande sind, gegebenenfalls mit geeigneten Basen oder Säuren Salze bilden.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens,* Stamm FZB42 (DSM 231179), oder *Bacillus cereus,* insbesondere *B. cereus* Stamm CNCM 1-1562 oder *Bacillus firmus,* Stamm 1-1582 (Accession number CNCM 1-1582) oder *Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421) *Bacillus thuringiensis,* insbesondere *B. thuringiensis* subspecies *israelensis* (serotype H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii,* (ehemals bekannt als *Verticillium lecanii*), insbesondere Stamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus* (neu: *Isaria fumosorosea*), insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM 1-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia*), *Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia, "Requiem™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie-Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (I) behandelt wurde. Die Erfindung bezieht sich auch auf Saatgut, welches zur gleichen Zeit mit einer Verbindung der Formel (I) und Mischungspartner behandelt wurde. Die Erfindung bezieht sich weiterhin auf Saatgut, welches zu unterschiedlichen Zeiten mit einer Verbindung der Formel (I) und Mischungspartner behandelt wurde. Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein. Die Erfindung bezieht sich auch auf Saatgut, bei dem eine Verbindung der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating - Verfahren unterzogen wird, um Staubabrieb am Saatgut zu vermeiden.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu den Arthropoden zählen:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.;
aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin zählen zu den Arthropoden:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

### Zu parasitären Protozoen zählen:

Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;

Sarcomastigophora (Rhizopoda), wie Entamoebidae, zum Beispiel Entamoeba histolytica, Hartmanellidae, zum Beispiel Acanthamoeba sp., Harmanella sp.;

Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..
Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf die Verwendung einer Verbindung der Formel (I) als Arzneimittel.

Ein weiterer Aspekt bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt wiederum betrifft die Verwendung einer Verbindung der Formel (I) als Antiektoparasitikum, insbesondere ein Arthropodizid wie ein Insektizid oder ein Akarizid Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

### Anthelminthische Mischungspartner

Beispielhaft seien folgende anthelmintische Mischungspartner genannt:
Anthelminthische Wirkstoffe, einschließlich trematicide und cestocide Wirkstoffe:
aus der Klasse der macrocyclischen Lactone, z. B.: Abamectin, Doramectin, Emamectin, Eprinomectin, Ivermectin, Milbemycin, Moxidectin, Nemadectin, Selamectin;
aus der Klasse der Benzimidazole und Probenzimidazole, z. B.: Albendazol, Albendazol- Sulfoxid, Cambendazol, Cyclobendazol, Febantel, Fenbendazol, Flubendazol, Mebendazol, Netobimin, Oxfendazol, Oxibendazol, Parbendazol, Thiabendazol, Thiophanat, Triclabendazol;
aus der Klasse der Cyclooctadepsipeptide, z. B.: Emodepsid, PF1022;
aus der Klasse der Aminoacetonitril-Derivate, z. B.: Monepantel;
aus der Klasse der Tetrahydropyrimidine, z. B.: Morantel, Pyrantel, Oxantel;
aus der Klasse der Imidazothiazole, z. B.: Butamisol, Levamisol, Tetramisol;
aus der Klasse der Salicylanilide, z. B.: Bromoxanid, Brotianid, Clioxanid, Closantel, Niclosamid, Oxyclozanid, Rafoxanid, Tribromsalan;
aus der Klasse der Paraherquamide, z. B.: Derquantel, Paraherquamid;
aus der Klasse der Aminophenylamidine, z. B.: Amidantel, deacyliertes Amidantel (dAMD), Tribendimidin;
aus der Klasse der Organophosphate, z. B.: Coumaphos, Crufomat, Dichlorvos, Haloxon, Naphthalofos, Trichlorfon;
aus der Klasse der substituierten Phenole, z. B.: Bithionol, Disophenol, Hexachlorophen, Niclofolan, Meniclopholan, Nitroxynil;
aus der Klasse der Piperazinone, z. B.: Praziquantel, Epsiprantel;
aus anderen diversen Klassen, z. B.: Amoscanat, Bephenium, Bunamidin, Clonazepam, Clorsulon, Diamfenetid, Dichlorophen, Diethylcarbamazin, Emetin, Hetolin, Hycanthon, Lucanthon, Miracil, Mirasan, Niclosamid, Niridazol, Nitroxynil, Nitroscanat, Oltipraz, Omphalotin, Oxamniquin, Paromomycin, Piperazin, Resorantel.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenz-brechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Beispiele:

### Erläuterung der Verfahren und Zwischenprodukte

Die folgenden Herstellungs- und Verwendungsbeispiele illustrieren die Erfindung, ohne sie zu beschränken. Die Produkte wurden mittels 1H-NMR Spektroskopie und/oder LC-MS (Liquid Chromatography Mass Spectrometry) und/oder GC-MS (Gas Chromatography-Mass Spectrometry) charakterisiert.

Die Bestimmung der logP Werte erfolgte analog OECD Guideline 117 (EC Directive 92/69/EEC) durch HPLC (High Performance Liquid Chromatography) an reversed-phase Säulen (C 18), mit nachfolgenden Methoden:
[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril. LogP^{[a]} wird auch logP(HCOOH) genannt.
[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. LogP^{[b]} wird auch logP(neutral) genannt.

Die Eichung erfolgt mit Lösungen einer homologen Reihe unverzweigter Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit einem Bruker II Avance 400, ausgestattet mit einem 1,7 mm TCI-Probenkopf, gemessen. In Einzelfällen wurden die NMR Spektren mit einem Bruker Avance II 600 bestimmt.

Die NMR-Daten ausgewählter Beispiele werden in klassischer Form (δ-Werte, Multiplettaufspaltung, Anzahl der H-Atome) aufgeführt. Die Aufspaltung der Signale wurde wie folgt beschrieben: s (Singulett), d (Duplett), t (Triplett), q (Quartett), m (Multiplett), breit (für breite Signale). Als Lösungsmittel wurden CD₃CN, CDCl₃ oder D6-DMSO verwendet, wobei als Referenz Tetramethylsilan (0.00 ppm) eingesetzt wurde.

Die GC-MS-Spektren werden mit einem Agilent 6890 GC, HP 5973 MSD an Dimethylsilikonphase bestimmt, mit einem Temperaturgradient von 50 °C bis 320 °C. GC-MS-Indices werden als Kovats-Indices mit Lösung einer homologen Reihe von n-Alkanen (mit geradzahliger Anzahl von 8 bis 38 Kohlenstoffatomen) bestimmt.

### Herstellbeispiel 1: 3-{2-Fluor-4-methyl-5-[(2,2,2-trifluorethyl)sulfinyl]phenyl}-6-(trifluormethyl)pyrimidin-4(3H)-on (Bsp. Nr. 2)

### Stufe 1: 4,4,4-Trifluor-N-{2-fluor-4-methyl-5-[(2,2,2-trifluorethyl)sulfanyl]phenyl}-3-hydroxybut-2-enamid (Bsp. Nr. XXXI-1)

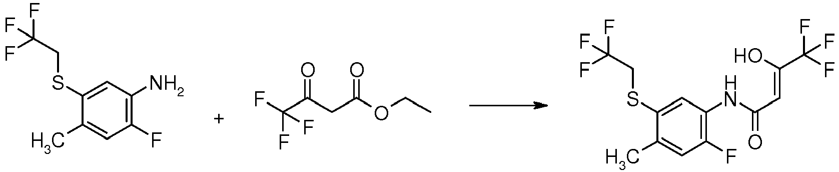

2 g (8,36 mmol) 2-Fluor-4-methyl-5-[(2,2,2-trifluorethyl)sulfanyl]anilin wurden in 42 ml Toluol vorgelegt. 2,48 ml (16,7 mmol) 4,4,4-Trifluoracetessigesterethylester und 20,4 mg (0,16 mmol) 4-DMAP wurden zugegeben und das Reaktionsgemisch wurde 14 h refluxiert. Nach Abkühlen der Reaktionsmischung wurde das Lösungsmittel unter vermindertem Druck am Rotationsverdampfer entfernt und der Rückstand (3,45 g) wurde ohne weitere Aufreinigung zu Stufe 2 umgesetzt.

### logP(HCOOH): 2,77

### Stufe 2: 3-Amino-4,4,4-trifluor-N-{2-fluor-4-methyl-5-[(2,2,2-trifluorethyl)sulfanyl]phenyl}but-2-enamid (Bsp. Nr. IIa-1)

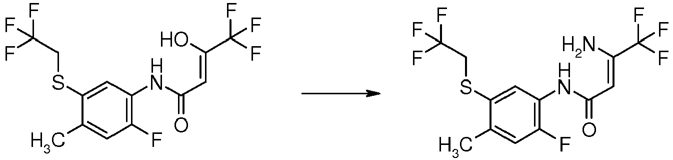

Der Rückstand (3,45 g) wurde in Ethylacetat (37,5 mL) aufgenommen und Ammoniumacetat (4,51 g, 58,5 mmol) wurde zugegeben. Die Reaktionsmischung wurde 7h refluxiert. Nach Abkühlen der Reaktionsmischung wurde Ethylacetat zugegeben (64 mL) und die organische Phase wurde erst mit Wasser und dann mit einer gesättigten NaCl-Lösung gewaschen. Die organische Phase wurde mit MgSO₄ getrocknet und das Lösungsmittel wurde unter vermindertem Druck am Rotationsverdampfer entfernt. Der Rückstand (2,78 g) wurde ohne weitere Aufreinigung zu Stufe 3 umgesetzt.
logP(HCOOH): 3,61

### Stufe 3: 3-{2-Fluor-4-methyl-5-[(2,2,2-trifluorethyl)sulfanyl]phenyl}-6-(trifluormethyl)pyrimidin-4(3H)-on (Bsp. Nr. 1)

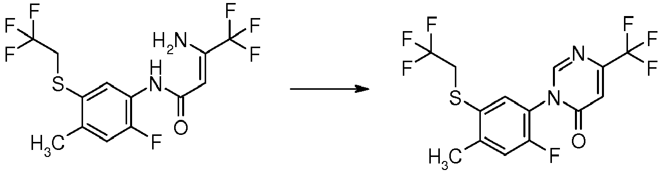

Der Rückstand (2,78 g) wurde in DMF-DMA (6,2 g) aufgenommen und 7h bei 100 °C gerührt. Nach Abkühlen der Reaktionsmischung wurde das Lösungsmittel unter vermindertem Druck am Rotationsverdampfer entfernt und der Rückstand wurde in Ameisensäure (17 mL) aufgenommen. Die Reaktionsmischung wurde 2h refluxiert. Nach Abkühlen der Reaktionsmischung wurde das Lösungsmittel unter vermindertem Druck am Rotationsverdampfer entfernt und der Rückstand wurde mit Ethylacetat versetzt. Die organische Phase wurde mit Wasser gewaschen und mit MgSO₄ getrocknet. Das Lösungsmittel wurde unter vermindertem Druck am Rotationsverdampfer entfernt und der Rückstand wurde mit Hilfe einer MPLC gesäult (Cyclohexan/Aceton). 670 mg der Titelverbindunge wurden isoliert (27% der Theorie über die 3 Stufen, Reinheit 80% nach LC-MS). Eine weitere Aufreinigung mit einer MPLC (Cyclohexan/Essigester) lieferte 157 mg der Titelverbindung mit einer besseren Reinheit (100% nach LC-MS).
¹H-NMR(D6-DMSO) δ ppm: 8,72(s,1H), 7,89(d,1H), 7,48(d,1H), 7,17(s,1H), 4,01(q,2H), 2,44(s,3H)
logP(HCOOH): 3,42 logP(neutral): 3,4

### Stufe 4: 3-{2-Fluor-4-methyl-5-[(2,2,2-trifluorethyl)sulfinyl]phenyl}-6-(trifluormethyl)pyrimidin-4(3H)-on (Bsp. Nr. 2)

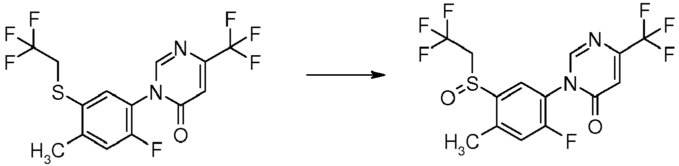

190 mg (0,492 mmol) 3-{2-Fluor-4-methyl-5-[(2,2,2-trifluorethyl)sulfanyl]phenyl}-6-(trifluormethyl)pyrimidin-4(3H)-on wurden in Methylenchlorid vorgelegt, 124 mg (0,541 mmol) meta-Chlorperbenzoesäure wurden zugegeben und das Reaktionsgemisch wurde 3h bei 0 °C gerührt und anschließend mit Natriumthiosulfat- und Natriumhydrogencarbonatlösung ausgeschüttelt. Die organische Phase wurde über Magnesiumsulfat getrocknet und filtriert. Nach Entfernen des Lösungsmittels unter vermindertem Druck wurden 170 mg der Titelverbindung (84% der Theorie, Reinheit 97% nach LC/MS) isoliert.
¹H-NMR(D6-DMSO) δ ppm: 8,75(s,1H), 8,17(d,1H), 7,59(d,1H), 7,17(s,1H), 4,29-4,22(m,1H), 4,07-4,01(m,1H), 2,48(s,3H)
logP(HCOOH): 2,4 logP(neutral): 2,38

### Herstellbeispiel 2: 3-[2,4-dimethyl-5-(2,2,2-trifluoroethylsulfinyl)phenyl]-6-(trifluoromethyl)-pyrimidin-4-on (Bsp. Nr. 6)

### Stufe 1: N-[2,4-dimethyl-5-(2,2,2-trifluoroethylsulfanyl)phenyl]-4,4,4-trifluoro-3-hydroxy-but-2-enamid (Bsp. Nr. XXXI-2)

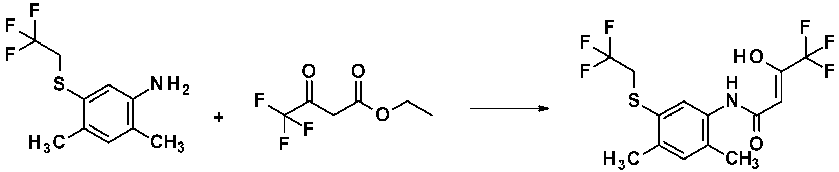

5 g (21,2 mmol) 2,4-Dimethyl-5-[(2,2,2-trifluorethyl)sulfanyl]anilin wurden in 42 ml Toluol vorgelegt. 6,21 ml (42,5 mmol) 4,4,4-Trifluoracetessigesterethylester und 51,9 mg (0,42 mmol) 4-DMAP wurden zugegeben und das Reaktionsgemisch wurde 14 h refluxiert. Nach Abkühlen der Reaktionsmischung wurde das Lösungsmittel unter vermindertem Druck am Rotationsverdampfer entfernt und der Rückstand (7,93 g) wurde ohne weitere Aufreinigung zu Stufe 2 umgesetzt.
logP(HCOOH): 2,87

### Stufe 2: 3-Amino-N-[2,4-dimethyl-5-(2,2,2-trifluoroethylsulfanyl)phenyl}-4,4,4-trifluoro-but-2-enamid (Bsp.-Nr. IIa-2)

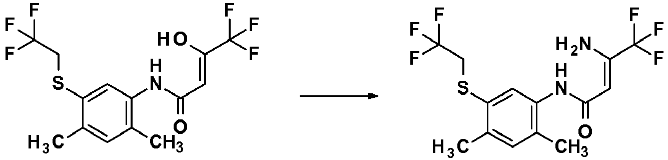

Der Rückstand (7,93 g) wurde in Ethylacetat (82,8 mL) aufgenommen und Ammoniumacetat (6,68 g, 86,6 mmol) wurde zugegeben. Die Reaktionsmischung wurde 7h refluxiert. Nach Abkühlen der Reaktionsmischung wurde das Lösungsmittel unter vermindertem Druck am Rotationsverdampfer entfernt. Der Rückstand (7,91 g) wurde ohne weitere Aufreinigung zu Stufe 3 umgesetzt.
logP(HCOOH): 3,63

### Stufe 3: 3-[2,4-dimethyl-5-(2,2,2-trifluoroethylsulfanyl)phenyl]-6-(trifluoromethyl)pyrimidin-4-on (Bsp. Nr. 5)

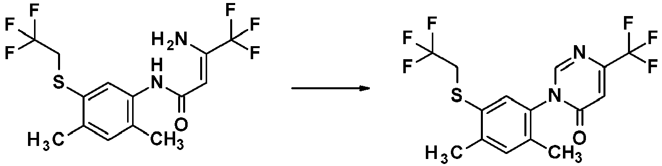

Der Rückstand (7,91 g) wurde in Orthoameisensäuretriethylester (53 ml) aufgenommen und mit Schwefelsäure (0,33 mL) versetzt. Die Mischung wurde 3h bei 140 °C gerührt. Nach Abkühlen der Reaktionsmischung wurde das Lösungsmittel unter vermindertem Druck am Rotationsverdampfer entfernt. Der Rückstand wurde mit Wasser versetzt und mit Ethylacetat extrahiert. Die organische Phase wurde über Na₂SO₄ getrocknet und filtriert. Das Lösungsmittel wurde unter vermindertem Druck am Rotationsverdampfer entfernt und der Rückstand wurde mit Hilfe einer MPLC gesäult (Cyclohexan/Aceton). 2,99 g der Titelverbindung wurden isoliert (37% der Theorie über die 3 Stufen, Reinheit 96% nach LC-MS).
¹H-NMR(D6-DMSO) δ ppm: 8,59(s,1H), 7,64(s,1H), 7,33(s,1H), 7,13(s,1H), 4.00(q,2H), 2,38(s,3H), 2,04(s,3H)
logP(HCOOH): 3,56 logP(neutral): 3,5

### Stufe 4: 3-[2,4-dimethyl-5-(2,2,2-trifluoroethylsulfinyl)phenyl]-6-(trifluoromethyl)pyrimidin-4-on (Bsp. Nr. 6)

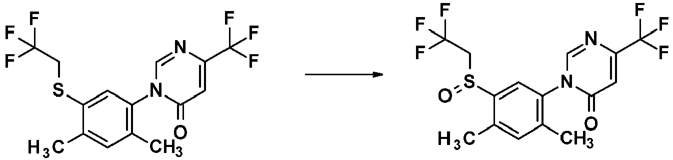

500 mg (1,30 mmol) 3-[2,4-dimethyl-5-(2,2,2-trifluoroethylsulfanyl)phenyl]-6-(trifluoromethyl)-pyrimidin-4-on wurden in Methylenchlorid (25 mL) vorgelegt, 331 mg (1,43 mmol) meta-Chlorperbenzoesäure wurden zugegeben und das Reaktionsgemisch wurde 1h bei 0 °C gerührt und anschließend mit Natriumthiosulfat- und Natriumhydrogencarbonatlösung gewaschen. Die organische Phase wurde über Natriumsulfat getrocknet und filtriert. Nach Entfernen des Lösungsmittels unter vermindertem Druck wurden 490 mg der Titelverbindung (92% der Theorie, Reinheit 98% nach LC/MS) isoliert.
¹H-NMR(D6-DMSO) δ ppm: 8,64-8,62(m,1H), 7,92(s,1H), 7,47-7,44(m,1H), 7,13(s,1H), 4,30-3,88(m,2H), 2,43(s,3H), 2,15(s,3H)
logP(HCOOH): 2,43 logP(neutral): 2,39

Die Enantiomere wurden aus dem Racemat gewonnen, in dem sie präparativ mittels HPLC über einer chiralen Säule (ChiralCel OJ-H z.B. 5nm 250 x4.6 mm) mit Laufmittel Heptan / Methanol / Ethanol getrennt wurden.

Die Bestimmung der Drehwerte erfolgte an einem Perkin Elmer 341, Seriennummer 9123, bei einer Wellenlänge von 589 nm und einer Temperatur von 20 °C.

Die unten stehenden spezifischen Drehwerte sind als Durchschnitt aus 5 unterschiedlichen Messungen zu verstehen:
Enantiomer 1 (Bsp. Nr. 56): 89,5 in CHCl₃ (c=0,022)
Enantiomer 2 (Bsp Nr. 49): - 87,8 in CHCl₃ (c=0,019)

Gemäß den zuvor beschriebenen Verfahren wurden die folgenden Verbindungen der Formel (I) hergestellt.

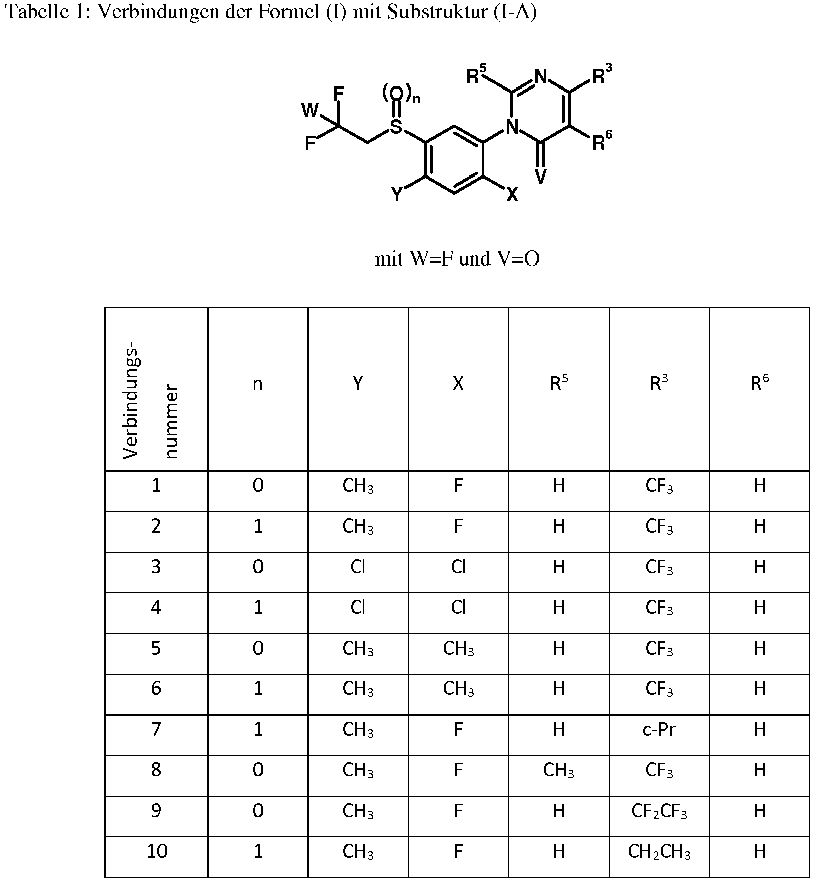

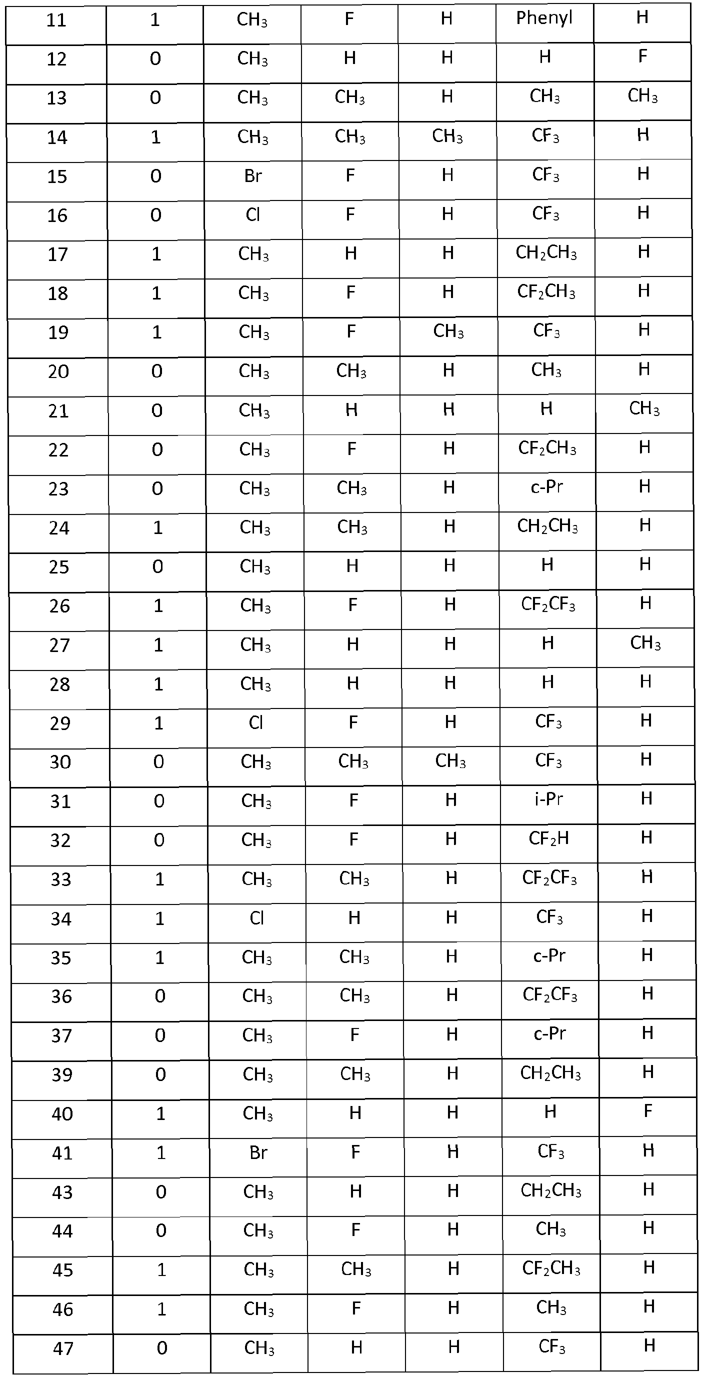

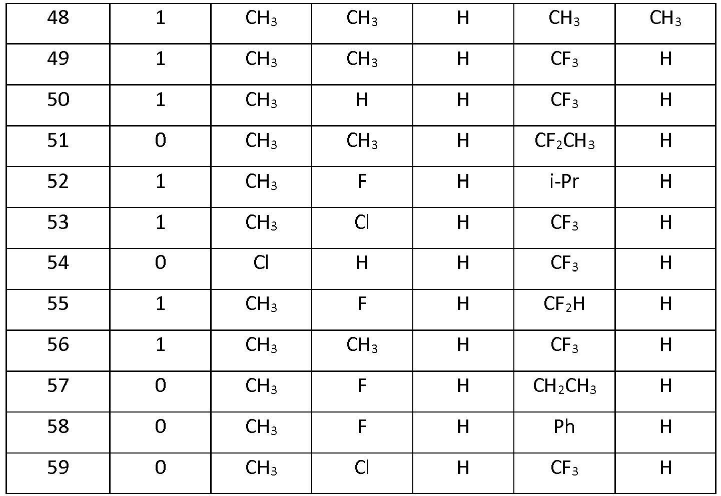

Ferner wurden gemäß den zuvor beschriebenen Verfahren die folgenden Verbindungen der Formel (IIa) (vgl. Tabelle 2) sowie der Formel (XXXI) (vgl. Tabelle 3) hergestellt.

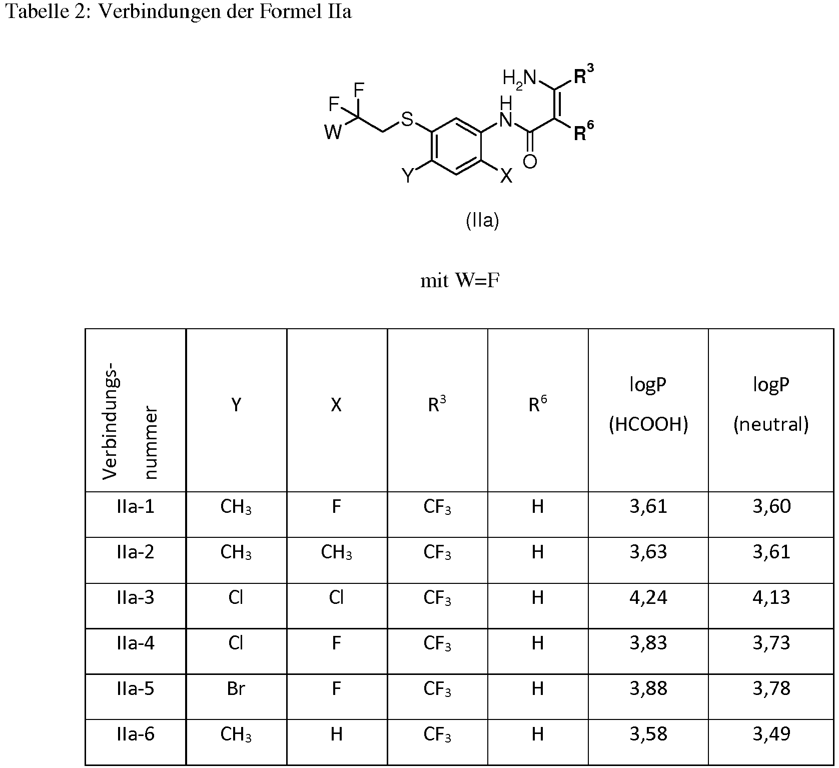

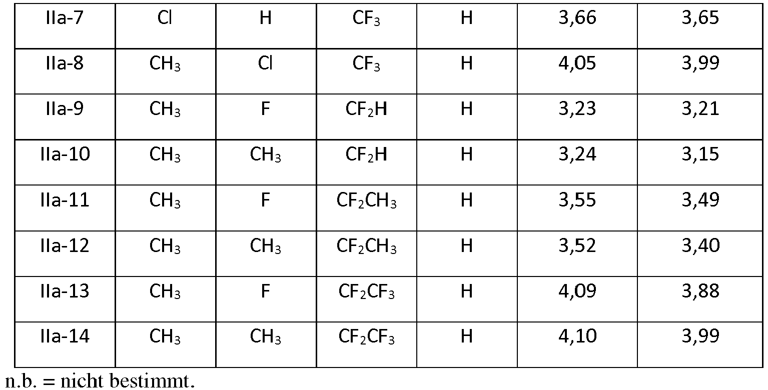

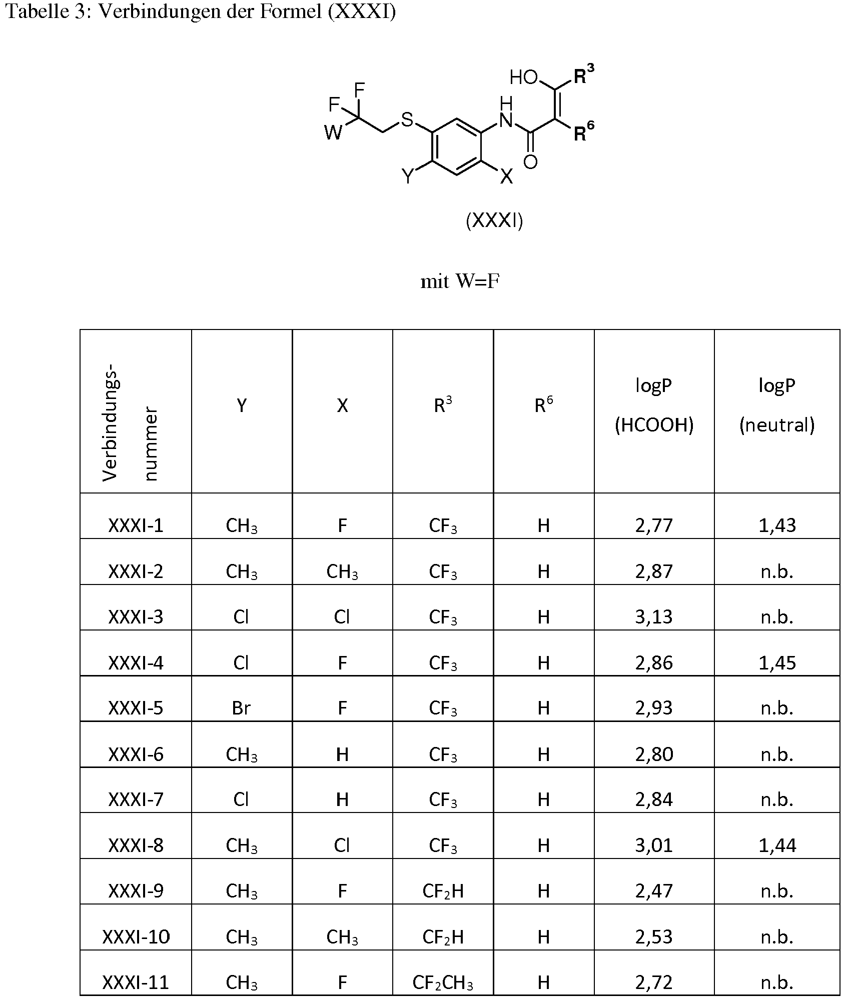

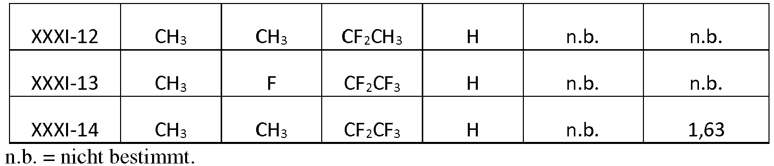

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁); δ₂ (Intensität₂);........; δᵢ (Intensitätᵢ); ......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

**Tabelle 4: NMR-Daten der Verbindungen gemäß Tabelle 1:**

| |
|---|
| Beispiel 1: ¹H-NMR(400,0 MHz, DMSO): |
| δ = 9,238(0,6); 8,720(4,4); 8,292(0,7); 8,290(0,7); 7,905(2,9); 7,886(3,0); 7,493(2,4); 7,465(2,4); 7,175(4,8); 5,758(0,7); 4,047(1,2); 4,022(4,0); 3,996(4,2); 3,970(1,4); 3,322(24,9); 2,670(0,4); 2,524(1,3); 2,519(2,1); 2,510(23,9); 2,506(47,6); 2,501(62,4); 2,497(44,8); 2,492(21,0); 2,443(16,0); 2,328(0,4); 0,008(0,4); 0,000(10,6) |
| Beispiel 2: ¹H-NMR(400,0 MHz, DMSO): |
| δ = 9,238(0,7); 8,753(4,7); 8,291(0,8); 8,185(3,1); 8,167(3,1); 7,609(2,3); 7,582(2,2); 7,173(5,1); 5,757(4,4); 4,286(0,4); 4,259(0,5); 4,250(0,6); 4,223(0,6); 4,070(0,6); 4,044(0,6); 4,034(0,5); 4,008(0,4); 3,321(27,5); 2,745(0,7); 2,675(0,4); 2,670(0,6); 2,666(0,4); 2,524(1,8); 2,510(33,0); 2,506(64,8); 2,501(84,2); 2,497(60,6); 2,492(29,0); 2,479(16,0); 2,333(0,4); 2,328(0,6); 2,324(0,4); 1,236(0,4); 0,000(0,4) |
| Beispiel 3: ¹H-NMR(400,0 MHz, DMSO): |
| δ = 9,237(0,7); 9,172(0,6); 9,155(1,9); 9,125(0,8); 8,742(2,3); 8,720(9,5); 8,704(0,6); 8,318(4,6); 8,312(4,3); 8,290(1,1); 8,131(2,4); 8,129(2,3); 8,093(13,2); 8,070(16,0); 8,006(0,8); 7,988(0,4); 7,850(0,5); 7,840(0,6); 7,764(0,4); 7,620(0,6); 7,522(0,3); 7,430(0,4); 7,344(0,6); 7,325(0,5); 7,244(11,7); 7,224(0,5); 7,185(0,7); 6,781(0,5); 5,958(0,3); 5,537(0,4); 4,246(0,5); 4,231(1,1); 4,220(1,8); 4,206(3,4); 4,195(3,5); 4,181(3,6); 4,170(3,5); 4,156(1,9); 4,144(1,3); 4,130(0,8); 3,380(0,4); 3,324(311,3); 2,680(2,8); 2,675(5,7); 2,671(7,8); 2,666(5,6); 2,662(2,7); 2,639(0,3); 2,620(0,6); 2,606(0,4); 2,600(0,4); 2,541(4,7); 2,524(21,0); 2,511(436,6); 2,506(875,2); 2,502(1147,4); 2,497(818,8); 2,493(387,3); 2,408(0,4); 2,337(2,6); 2,333(5,5); 2,328(7,5); 2,324(5,4); 1,259(0,4); 1,235(1,3); 0,146(4,7); 0,027(0,3); 0,008(37,7); 0,000(1067,8); -0,009(37,2); -0,030(1,3); -0,054(0,6); - 0,068(0,5); -0,087(0,4); -0,092(0,4); -0,150(4,9) |
| Beispiel 4: ¹H-NMR(400,0 MHz, DMSO): |
| δ = 19,997(1,1); 8,714(1,2); 8,663(1,3); 8,316(4,9); 8,303(3,2); 8,290(2,2); 8,269(2,1); 7,224(1,5); 7,210(1,4); 3,323(2222,9); 2,675(11,9); 2,671(16,0); 2,666(11,6); 2,541(10,5); 2,524(46,3); 2,510(902,8); 2,506(1775,9); 2,501(2306,4); 2,497(1634,6); 2,493(766,6); 2,333(10,8); 2,328(14,7); 2,324(10,5); 1,234(2,8); 0,838(0,7); 0,146(15,4); 0,008(154,0); 0,000(3106,4); -0,009(92,9); - 0,150(13,7) |
| Beispiel 5: ¹H-NMR(400,0 MHz, DMSO): |
| δ = 8,588(4,6); 8,316(0,4); 7,641(5,6); 7,328(4,3); 7,126(5,0); 5,756(0,5); 4,031(1,2); 4,005(3,9); 3,979(4,0); 3,953(1,4); 3,321(66,0); 2,675(0,9); 2,670(1,3); 2,666(0,9); 2,662(0,5); 2,541(0,9); 2,524(3,2); 2,510(68,6); 2,506(139,2); 2,501(183,6); 2,497(131,5); 2,492(62,1); 2,380(15,2); 2,337(0,4); 2,333(0,9); 2,328(1,2); 2,324(0,9); 2,319(0,4); 2,041(16,0); 0,146(0,8); 0,008(6,8); 0,000(196,8); -0,009(6,8); -0,150(0,8) |
| Beispiel 6: ¹H-NMR(400,0 MHz, DMSO): |
| δ = 20,011(0,5); 8,637(1,5); 8,620(2,1); 8,316(4,9); 7,918(3,2); 7,464(1,4); 7,439(2,0); 7,131(3,1); 5,756(0,4); 4,300(0,5); 4,271(0,5); 4,263(0,5); 4,235(0,5); 4,129(0,8); 4,103(0,7); 4,081(0,8); 4,055(0,7); 4,018(0,5); 3,976(0,5); 3,945(0,5); 3,919(0,5); 3,883(0,6); 3,399(1,1); 3,391(1,2); 3,323(2085,6); 3,268(0,5); 2,980(0,4); 2,937(0,5); 2,675(12,0); 2,671(16,0); 2,666(11,5); 2,541(11,4); 2,524(49,6); 2,510(913,2); 2,506(1787,3); 2,502(2314,4); 2,497(1649,1); 2,493(779,4); 2,428(10,1); 2,337(5,2); 2,333(10,7); 2,328(14,5); 2,324(10,4); 2,153(7,3); 2,145(5,1); 1,235(1,6); 0,146(14,5); 0,116(0,6); 0,077(1,1); 0,069(1,0); 0,059(1,1); 0,008(138,4); 0,000(3114,1); -0,009(108,4); -0,056(0,6); -0,150(14,2) |
| Beispiel 7: ¹H-NMR(400,0 MHz, DMSO): |
| δ = 8,372 (5,6); 8,009 (3,2); 7,991 (3,2); 7,560 (2,5); 7,533 (2,5); 6,493 (6,1); 6,492 (5,9); 5,757 (7,9); 4,255 (0,5); 4,247 (0,4); 4,228 (0,6); 4,219 (0,9); 4,192 (0,8); 4,165 (0,3); 4,115 (1,0); 4,106 (0,4); 4,088 (1,1); 4,078 (0,7); 4,060 (0,4); 4,051 (0,7); 3,329 (25,0); 2,506 (26,1); 2,502 (33,7); 2,497 (24,9); 2,464 (16,0); 2,411 (0,5); 2,329 (0,3); 2,012 (0,3); 1,996 (1,0); 1,980 (1,5); 1,964 (1,1); 1,948 (0,4); 1,030 (0,4); 1,007 (10,9); 0,993 (5,3); 0,988 (5,4); 0,000 (6,4) |
| Beispiel 8: ¹H-NMR(400,0 MHz, DMSO): |
| δ = 7,895 (2,5); 7,876 (2,5); 7,519 (2,2); 7,492 (2,2); 7,058 (4,9); 4,063 (0,4); 4,049 (0,5); 4,037 (0,5); 4,023 (1,7); 3,997 (2,4); 3,970 (1,7); 3,957 (0,5); 3,944 (0,5); 3,931 (0,4); 3,333 (20,7); 2,671 (0,4); 2,525 (1,1); 2,511 (23,8); 2,507 (47,9); 2,502 (62,7); 2,498 (46,0); 2,493 (22,8); 2,444 (14,2); 2,375 (1,0); 2,329 (0,4); 2,212 (16,0); 0,146 (0,4); 0,008 (3,0); 0,000 (83,1); -0,009 (3,3); -0,150 (0,4) |
| Beispiel 9: ¹H-NMR(400,0 MHz, DMSO): |
| δ = 8,734 (4,9); 7,923 (2,9); 7,905 (2,9); 7,495 (2,5); 7,468 (2,5); 7,228 (5,6); 7,226 (5,7); 4,050 (1,2); 4,024 (3,9); 3,998 (4,1); 3,973 (1,4); 3,322 (31,9); 2,675 (0,4); 2,671 (0,5); 2,666 (0,4); 2,524 (1,3); 2,510 (29,4); 2,506 (59,6); 2,502 (79,5); 2,497 (59,1); 2,493 (29,8); 2,445 (16,0); 2,333 (0,4); 2,328 (0,5); 2,324 (0,4); 0,008 (0,3); 0,000 (10,2); -0,008 (0,4) |
| Beispiel 10: ¹H-NMR(400,0 MHz, DMSO): |
| δ = 8,446 (4,8); 8,413 (0,4); 8,316 (0,9); 8,029 (2,8); 8,011 (2,8); 7,569 (2,2); 7,542 (2,2); 6,389 (4,3); 6,387 (4,3); 4,260 (0,5); 4,251 (0,3); 4,233 (0,6); 4,224 (0,8); 4,196 (0,8); 4,128 (0,9); 4,101 (1,0); 4,091 (0,7); 4,074 (0,4); 4,064 (0,6); 3,322 (149,5); 2,730 (1,1); 2,675 (1,4); 2,671 (1,9); 2,666 (1,4); 2,586 (1,3); 2,567 (3,8); 2,548 (4,2); 2,524 (5,3); 2,510 (104,6); 2,506 (208,9); 2,502 (276,9); 2,497 (208,3); 2,470 (16,0); 2,333 (1,4); 2,328 (2,1); 2,324 (1,5); 1,235 (0,4); 1,217 (5,4); 1,198 (11,4); 1,179 (5,2); 0,146 (1,2); 0,008 (10,9); 0,000 (280,5); -0,008 (14,3); -0,150 (1,3) |
| Beispiel 11: ¹H-NMR(400,0 MHz, DMSO): |
| δ = 8,645 (13,2); 8,316 (0,4); 8,167 (0,9); 8,157 (6,6); 8,152 (7,7); 8,144 (7,2); 8,141 (6,1); 8,133 (7,5); 8,121 (8,0); 8,103 (7,6); 7,603 (5,8); 7,576 (5,8); 7,560 (2,1); 7,550 (16,0); 7,545 (14,9); 7,537 (9,2); 7,533 (9,0); 7,523 (2,4); 7,509 (0,6); 7,157 (14,4); 7,155 (13,7); 5,756 (10,1); 4,312 (0,4); 4,285 (1,3); 4,276 (0,8); 4,258 (1,6); 4,249 (2,1); 4,230 (0,8); 4,221 (2,1); 4,194 (0,7); 4,169 (0,6); 4,142 (2,1); 4,115 (2,3); 4,106 (1,6); 4,088 (1,0); 4,079 (1,4); 4,052 (0,5); 3,325 (90,4); 2,676 (0,6); 2,671 (0,8); 2,666 (0,6); 2,524 (2,5); 2,511 (46,0); 2,506 (91,8); 2,502 (120,2); 2,497 (88,0); 2,492 (56,7); 2,333 (0,7); 2,329 (1,0); 2,324 (0,7); 0,146 (0,8); 0,008 (7,3); 0,000 (188,1); -0,009 (6,7); -0,150 (0,8) |
| Beispiel 12: ¹H-NMR(400,0 MHz, DMSO): |
| δ = 8,339 (6,7); 8,191 (3,5); 8,184 (3,4); 7,704 (3,5); 7,700 (3,5); 7,445 (2,2); 7,425 (3,3); 7,353 (2,3); 7,349 (2,2); 7,333 (1,5); 7,328 (1,4); 5,757 (1,0); 4,106 (1,3); 4,080 (3,9); 4,054 (4,0); 4,029 (1,3); 3,324 (49,3); 2,670 (0,6); 2,502 (92,9); 2,403 (16,0); 2,328 (0,6); 1,235 (0,7); 0,000 (5,1) |
| Beispiel 13: ¹H-NMR(400,0 MHz, DMSO): |
| δ = 8,089 (5,3); 7,990 (0,4); 7,489 (5,6); 7,293 (4,3); 6,792 (0,4); 5,758 (0,4); 4,752 (0,3); 4,037 (0,8); 4,035 (0,8); 4,012 (2,4); 4,009 (2,4); 3,986 (2,4); 3,983 (2,5); 3,960 (0,9); 3,957 (0,9); 3,694 (0,4); 3,668 (0,4); 3,337 (15,5); 3,336 (16,9); 3,330 (19,8); 2,525 (0,5); 2,520 (0,7); 2,511 (11,3); 2,507 (23,2); 2,502 (30,8); 2,498 (22,6); 2,493 (11,3); 2,372 (15,1); 2,346 (0,6); 2,338 (1,2); 2,329 (0,4); 2,291 (12,6); 2,204 (1,5); 2,154 (0,3); 2,145 (1,1); 2,055 (0,5); 1,998 (14,2); 1,989 (16,0); 1,949 (0,4); 0,987 (0,4); 0,969 (0,6); 0,008 (1,1); 0,000 (33,6); -0,009 (1,3) |
| Beispiel 14: ¹H-NMR(400,0 MHz, DMSO): |
| δ = 9,266 (0,3); 8,317 (0,9); 7,910 (3,2); 7,901 (2,2); 7,485 (2,3); 7,474 (1,5); 7,189 (0,5); 7,013 (1,9); 6,999 (2,8); 4,310 (0,5); 4,282 (0,5); 4,273 (0,6); 4,246 (0,6); 4,219 (0,3); 4,193 (0,6); 4,184 (0,6); 4,166 (0,6); 4,157 (0,6); 3,913 (0,5); 3,886 (0,6); 3,876 (0,5); 3,859 (0,3); 3,849 (0,5); 3,331 (519,1); 2,676 (1,9); 2,671 (2,7); 2,667 (1,9); 2,662 (1,0); 2,524 (6,0); 2,520 (9,8); 2,511 (146,3); 2,507 (304,5); 2,502 (406,6); 2,498 (299,7); 2,493 (148,9); 2,435 (8,0); 2,424 (5,5); 2,375 (0,5); 2,338 (1,0); 2,333 (2,0); 2,329 (2,8); 2,324 (2,2); 2,296 (1,6); 2,267 (0,5); 2,253 (1,8); 2,180 (6,0); 2,109 (0,6); 2,091 (16,0); 1,351 (0,8); 1,259 (0,4); 1,233 (1,0); 0,008 (1,3); 0,000 (44,6); -0,008 (1,8) |
| Beispiel 15: ¹H-NMR(400,0 MHz, DMSO): |
| δ = 8,723 (10,3); 8,316 (0,5); 8,045 (9,4); 8,040 (7,0); 8,022 (16,0); 7,211 (11,1); 5,756 (2,5); 4,184 (2,2); 4,159 (7,0); 4,146 (0,8); 4,133 (7,3); 4,108 (2,5); 3,324 (132,3); 2,680 (0,4); 2,675 (0,7); 2,671 (1,1); 2,666 (0,8); 2,662 (0,3); 2,524 (2,6); 2,519 (4,1); 2,511 (58,1); 2,506 (118,2); 2,502 (156,8); 2,497 (112,2); 2,493 (52,3); 2,338 (0,4); 2,333 (0,8); 2,328 (1,1); 2,324 (0,8); 2,319 (0,3); 1,235 (0,5); 0,146 (0,7); 0,008 (5,7); 0,000 (174,4); -0,009 (5,5); -0,150 (0,7) |
| Beispiel 16: ¹H-NMR(400,0 MHz, DMSO): |
| δ = 10,306 (0,5); 8,725 (14,9); 8,597 (0,3); 8,405 (0,4); 8,316 (0,7); 8,117 (0,4); 8,087 (9,3); 8,069 (9,3); 7,945 (10,8); 7,921 (10,8); 7,698 (0,5); 7,672 (0,5); 7,214 (16,0); 5,757 (2,3); 4,184 (3,3); 4,159 (10,5); 4,134 (10,9); 4,108 (3,7); 3,970 (0,6); 3,943 (0,7); 3,918 (0,4); 3,640 (0,4); 3,326 (311,9); 3,295 (0,5); 3,256 (0,4); 3,142 (0,3); 2,675 (1,5); 2,671 (2,0); 2,666 (1,5); 2,541 (0,8); 2,524 (5,6); 2,510 (118,1); 2,506 (232,8); 2,502 (304,3); 2,497 (223,3); 2,493 (110,3); 2,333 (1,5); 2,328 (2,0); 2,324 (1,5); 1,178 (0,7); 1,160 (1,4); 1,143 (0,8); 0,008 (0,8); 0,000 (23,5); -0,009 (0,9) |
| Beispiel 17: ¹H-NMR(400,0 MHz, DMSO): |
| δ = 8,431 (5,6); 8,317 (0,7); 7,891 (3,6); 7,885 (3,8); 7,640 (1,6); 7,635 (1,6); 7,620 (2,5); 7,614 (2,4); 7,536 (3,2); 7,515 (2,2); 6,368 (4,8); 5,757 (0,3); 4,234 (0,8); 4,224 (0,5); 4,206 (0,9); 4,197 (1,3); 4,178 (0,4); 4,169 (1,3); 4,142 (0,5); 4,114 (0,4); 4,086 (1,3); 4,060 (1,4); 4,050 (0,9); 4,033 (0,5); 4,023 (0,8); 3,325 (406,6); 2,675 (2,6); 2,671 (3,5); 2,666 (2,6); 2,575 (1,8); 2,556 (4,7); 2,537 (5,8); 2,524 (9,7); 2,510 (199,9); 2,506 (396,5); 2,502 (519,5); 2,497 (385,0); 2,493 (194,5); 2,444 (16,0); 2,388 (0,7); 2,333 (2,4); 2,328 (3,3); 2,324 (2,5); 1,236 (0,6); 1,216 (6,1); 1,198 (12,8); 1,179 (5,8); 0,008 (1,6); 0,000 (41,2); -0,008 (1,5) |
| Beispiel 18: ¹H-NMR(400,0 MHz, DMSO): |
| δ = 8,668 (9,3); 8,627 (0,8); 8,323 (0,8); 8,304 (0,4); 8,148 (5,4); 8,129 (5,5); 7,768 (0,4); 7,742 (0,4); 7,608 (4,6); 7,581 (4,6); 6,793 (10,6); 4,991 (0,5); 4,966 (0,6); 4,287 (1,0); 4,252 (1,6); 4,224 (1,4); 4,196 (0,5); 4,124 (0,5); 4,098 (1,5); 4,072 (1,7); 4,062 (1,4); 4,044 (0,9); 4,035 (1,2); 4,009 (0,4); 3,330 (113,6); 2,750 (2,3); 2,679 (1,5); 2,510 (240,7); 2,488 (38,4); 2,337 (1,6); 2,003 (7,4); 1,955 (16,0); 1,907 (8,3); 1,245 (0,3); 0,008 (0,5) |
| Beispiel 19: ¹H-NMR(400,0 MHz, DMSO): |
| δ = 20,012 (0,4); 8,317 (2,9); 8,187 (2,7); 8,177 (1,9); 8,169 (2,9); 8,159 (1,7); 7,652 (2,2); 7,644 (1,4); 7,626 (2,2); 7,617 (1,4); 7,054 (3,1); 7,047 (5,1); 4,385 (0,8); 4,376 (0,4); 4,358 (0,9); 4,349 (1,0); 4,321 (1,1); 4,294 (0,4); 4,285 (0,4); 4,258 (0,4); 4,248 (0,8); 4,221 (0,8); 4,200 (0,8); 4,173 (0,9); 4,135 (0,4); 3,978 (0,4); 3,952 (1,1); 3,925 (1,1); 3,915 (0,9); 3,899 (0,5); 3,888 (1,0); 3,861 (0,3); 3,330 (1595,1); 2,676 (5,4); 2,671 (7,6); 2,667 (5,6); 2,524 (18,0); 2,511 (415,9); 2,507 (858,2); 2,502 (1139,8); 2,498 (837,1); 2,493 (412,0); 2,481 (24,3); 2,475 (14,3); 2,333 (5,5); 2,329 (7,6); 2,324 (6,3); 2,276 (9,6); 2,192 (16,0); 1,351 (1,7); 1,298 (0,6); 1,258 (1,0); 1,233 (2,6); 1,161 (0,7); 1,094 (0,4); 0,853 (0,4); 0,146 (0,6); 0,008 (4,2); 0,000 (127,6); -0,008 (4,2); -0,150 (0,6) |
| Beispiel 20: ¹H-NMR(601,6 MHz, DMSO): |
| δ = 19,975 (0,5); 9,713 (0,4); 9,694 (0,4); 9,573 (1,4); 8,411 (1,7); 8,393 (1,6); 8,316 (0,6); 8,270 (3,8); 8,267 (3,8); 8,227 (1,1); 7,931 (5,2); 7,508 (1,1); 7,358 (2,2); 7,293 (0,9); 7,133 (4,1); 7,112 (1,8); 6,375 (1,0); 4,017 (0,5); 3,999 (1,6); 3,982 (1,6); 3,965 (0,6); 3,796 (1,2); 3,778 (3,6); 3,761 (3,8); 3,744 (1,3); 3,326 (96,7); 2,614 (0,9); 2,523 (1,5); 2,520 (1,9); 2,517 (1,9); 2,508 (56,6); 2,505 (124,1); 2,502 (171,8); 2,499 (122,8); 2,496 (55,0); 2,386 (1,0); 2,370 (3,3); 2,330 (15,7); 2,289 (6,9); 2,271 (3,0); 2,178 (7,7); 2,173 (16,0); 2,000 (3,3); 1,351 (1,6); 1,336 (0,6); 1,259 (0,5); 1,249 (0,8); 1,228 (1,1); 0,000 (1,9) |
| Beispiel 21: ¹H-NMR(400,0 MHz, DMSO): |
| δ = 8,278 (5,3); 7,905 (3,6); 7,639 (3,2); 7,634 (3,4); 7,414 (2,3); 7,393 (3,1); 7,291 (2,2); 7,286 (2,1); 7,271 (1,6); 7,266 (1,6); 4,111 (1,3); 4,085 (3,9); 4,059 (4,1); 4,034 (1,4); 3,325 (43,6); 2,671 (0,6); 2,666 (0,5); 2,506 (67,0); 2,502 (88,0); 2,497 (66,3); 2,397 (16,0); 2,333 (0,5); 2,328 (0,6); 2,324 (0,4); 1,998 (13,7); 1,235 (0,5); 0,000 (9,6); -0,008 (0,4) |
| Beispiel 22: ¹H-NMR(400,0 MHz, DMSO): |
| δ = 8,618 (4,7); 7,885 (2,9); 7,867 (2,9); 7,475 (2,6); 7,448 (2,5); 6,779 (5,4); 4,062 (1,2); 4,037 (3,9); 4,011 (4,1); 3,985 (1,4); 3,322 (44,2); 2,675 (0,4); 2,671 (0,6); 2,666 (0,4); 2,524 (1,4); 2,506 (65,8); 2,502 (88,0); 2,497 (66,2); 2,435 (16,0); 2,333 (0,4); 2,328 (0,6); 2,324 (0,5); 1,997 (4,1); 1,949 (8,9); 1,900 (4,7); 0,008 (0,3); 0,000 (10,4); -0,008 (0,5) |
| Beispiel 23: ¹H-NMR(601,6 MHz, DMSO): |
| δ = 8,182 (5,5); 7,500 (5,5); 7,281 (4,3); 6,446 (5,7); 5,755 (0,4); 4,026 (0,6); 4,012 (1,7); 4,009 (1,8); 3,995 (1,8); 3,992 (1,9); 3,974 (0,7); 3,326 (57,8); 2,613 (0,5); 2,523 (0,8); 2,520 (1,0); 2,517 (1,0); 2,508 (29,7); 2,505 (65,1); 2,502 (90,5); 2,499 (65,5); 2,496 (30,2); 2,391 (0,5); 2,386 (0,5); 2,361 (15,5); 2,086 (0,7); 2,065 (0,4); 1,994 (16,0); 1,973 (0,8); 1,963 (1,3); 1,954 (0,8); 1,950 (0,8); 1,941 (0,4); 1,398 (0,8); 0,991 (3,7); 0,987 (6,0); 0,974 (3,2); 0,000 (1,0) |
| Beispiel 24: ¹H-NMR(400,0 MHz, DMSO): |
| δ = 8,323 (7,3); 8,305 (0,5); 7,838 (0,4); 7,749 (4,8); 7,736 (3,4); 7,578 (0,4); 7,436 (2,5); 7,416 (3,6); 6,374 (4,0); 6,368 (3,3); 4,269 (0,5); 4,241 (0,5); 4,232 (0,6); 4,205 (0,6); 4,151 (0,9); 4,125 (2,7); 4,098 (2,9); 4,071 (1,0); 4,025 (0,7); 3,998 (0,7); 3,988 (0,5); 3,961 (0,5); 3,321 (216,3); 2,680 (2,0); 2,675 (2,4); 2,671 (3,1); 2,594 (1,3); 2,576 (3,8); 2,558 (4,0); 2,553 (3,5); 2,538 (2,3); 2,506 (357,6); 2,501 (466,5); 2,497 (362,9); 2,418 (16,0); 2,355 (0,3); 2,328 (3,1); 2,324 (2,5); 2,294 (0,4); 2,261 (0,4); 2,139 (1,3); 2,116 (13,5); 2,110 (10,6); 1,227 (4,6); 1,222 (4,0); 1,208 (9,4); 1,204 (7,8); 1,190 (4,6); 1,185 (3,8); 0,146 (2,7); 0,008 (26,2); 0,000 (549,3); -0,060 (0,4); -0,150 (2,8) |
| Beispiel 25: ¹H-NMR(400,0 MHz, DMSO): |
| δ = 8,410 (5,2); 8,317 (0,4); 7,998 (3,4); 7,981 (3,5); 7,649 (3,2); 7,644 (3,3); 7,418 (2,2); 7,397 (3,0); 7,301 (2,2); 7,295 (2,2); 7,281 (1,6); 7,275 (1,6); 6,524 (2,8); 6,522 (3,0); 6,507 (2,8); 6,505 (2,9); 4,117 (1,3); 4,091 (4,1); 4,065 (4,3); 4,039 (1,5); 3,323 (115,5); 2,675 (1,1); 2,671 (1,6); 2,666 (1,2); 2,524 (4,1); 2,519 (6,1); 2,510 (86,0); 2,506 (177,4); 2,501 (236,9); 2,497 (175,3); 2,493 (87,2); 2,395 (16,0); 2,333 (1,1); 2,328 (1,5); 2,324 (1,1); 1,989 (0,6); 1,175 (0,3); 0,146 (0,9); 0,008 (6,7); 0,000 (210,8); -0,009 (7,6); -0,150 (0,9) |
| Beispiel 26: ¹H-NMR(400,0 MHz, DMSO): |
| δ = 8,763 (13,6); 8,719 (1,6); 8,401 (0,9); 8,382 (0,9); 8,324 (1,0); 8,227 (8,0); 8,209 (8,1); 7,782 (0,8); 7,755 (0,7); 7,617 (6,3); 7,590 (6,3); 7,222 (16,0); 5,764 (2,6); 5,022 (0,3); 4,997 (1,1); 4,972 (1,2); 4,948 (0,4); 4,323 (0,4); 4,295 (1,3); 4,260 (1,9); 4,232 (1,7); 4,206 (0,6); 4,099 (0,6); 4,071 (1,6); 4,044 (1,9); 4,008 (1,3); 3,982 (0,5); 3,331 (223,5); 2,753 (5,0); 2,683 (2,7); 2,679 (3,5); 2,675 (2,8); 2,647 (0,4); 2,514 (411,2); 2,510 (530,6); 2,506 (403,1); 2,488 (54,5); 2,341 (2,6); 2,337 (3,4); 2,332 (2,7); 1,243 (0,5); 1,223 (0,4); 1,192 (0,5); 1,182 (0,3); 0,008 (1,2) |
| Beispiel 27: ¹H-NMR(400,0 MHz, DMSO): |
| δ = 8,368 (5,6); 7,924 (3,6); 7,922 (3,7); 7,901 (3,7); 7,895 (3,9); 7,652 (1,7); 7,646 (1,6); 7,631 (2,4); 7,626 (2,4); 7,542 (3,2); 7,521 (2,2); 4,238 (0,9); 4,228 (0,5); 4,211 (1,0); 4,201 (1,4); 4,183 (0,4); 4,174 (1,3); 4,147 (0,4); 4,113 (0,4); 4,086 (1,3); 4,076 (0,3); 4,059 (1,4); 4,049 (0,9); 4,032 (0,5); 4,022 (0,9); 3,325 (76,1); 2,671 (0,6); 2,666 (0,4); 2,524 (1,4); 2,510 (27,8); 2,506 (56,4); 2,501 (76,0); 2,497 (58,1); 2,493 (30,0); 2,447 (16,0); 2,333 (0,3); 2,328 (0,5); 2,324 (0,4); 2,010 (13,7); 1,351 (0,4); 1,298 (0,6); 1,259 (0,8); 1,250 (0,5); 1,234 (1,2); 0,000 (0,5) |
| Beispiel 28: ¹H-NMR(400,0 MHz, DMSO): |
| δ = 8,498 (5,4); 8,016 (3,8); 7,999 (3,9); 7,914 (3,6); 7,909 (3,8); 7,659 (1,7); 7,653 (1,6); 7,638 (2,4); 7,633 (2,3); 7,546 (3,1); 7,525 (2,2); 6,553 (3,0); 6,551 (3,2); 6,537 (2,9); 6,534 (3,1); 4,236 (0,8); 4,226 (0,5); 4,209 (0,9); 4,199 (1,3); 4,181 (0,4); 4,172 (1,3); 4,144 (0,4); 4,119 (0,4); 4,092 (1,3); 4,082 (0,3); 4,065 (1,5); 4,055 (0,9); 4,038 (0,5); 4,028 (0,9); 3,345 (133,3); 3,341 (137,3); 3,290 (0,3); 2,671 (0,5); 2,667 (0,3); 2,525 (1,2); 2,520 (1,8); 2,511 (26,1); 2,507 (53,8); 2,502 (71,6); 2,498 (52,4); 2,493 (25,4); 2,448 (16,0); 2,333 (0,3); 2,329 (0,5); 2,324 (0,3); 0,008 (1,9); 0,000 (59,6); -0,009 (2,0) |
| Beispiel 29: ¹H-NMR(400,0 MHz, DMSO): |
| δ = 8,736 (14,4); 8,316 (2,3); 8,294 (10,4); 8,276 (10,4); 8,122 (9,5); 8,099 (9,5); 7,821 (0,4); 7,197 (16,0); 5,756 (2,4); 4,421 (0,4); 4,392 (1,1); 4,357 (1,7); 4,330 (1,5); 4,302 (0,6); 4,198 (0,9); 4,172 (2,5); 4,162 (1,1); 4,145 (2,9); 4,136 (2,2); 4,118 (1,3); 4,109 (2,0); 4,081 (0,8); 3,323 (659,7); 2,675 (3,6); 2,671 (4,8); 2,666 (3,7); 2,586 (0,4); 2,524 (12,2); 2,510 (278,7); 2,506 (554,9); 2,502 (731,6); 2,497 (544,7); 2,333 (3,6); 2,328 (4,9); 2,324 (3,7); 1,235 (0,8); 1,215 (0,4); 1,184 (0,7); 1,167 (0,9); 1,151 (0,6); 0,146 (0,7); 0,008 (5,6); 0,000 (164,4); -0,008 (7,6); -0,150 (0,8) |
| Beispiel 30: ¹H-NMR(400,0 MHz, DMSO): |
| δ = 7,631 (4,9); 7,463 (0,3); 7,346 (3,9); 7,114 (0,6); 7,005 (4,8); 4,070 (0,5); 4,056 (0,4); 4,045 (0,6); 4,031 (1,2); 4,006 (1,4); 3,983 (1,3); 3,958 (1,3); 3,944 (0,6); 3,932 (0,5); 3,918 (0,6); 3,796 (0,5); 3,769 (0,5); 3,330 (35,4); 2,675 (0,4); 2,671 (0,6); 2,666 (0,4); 2,524 (1,3); 2,520 (2,1); 2,511 (32,6); 2,506 (68,1); 2,502 (92,3); 2,497 (69,3); 2,493 (35,0); 2,375 (13,3); 2,345 (0,6); 2,330 (2,5); 2,182 (0,4); 2,158 (2,4); 2,109 (16,0); 1,985 (13,8); 0,008 (1,6); 0,000 (53,8); -0,009 (2,0) |
| Beispiel 31: ¹H-NMR(400,0 MHz, DMSO): |
| δ = 8,403 (4,2); 7,819 (2,3); 7,800 (2,3); 7,439 (2,0); 7,412 (2,0); 6,353 (4,5); 4,078 (1,0); 4,053 (3,2); 4,027 (3,3); 4,001 (1,1); 3,338 (3,0); 3,333 (9,2); 2,818 (0,4); 2,801 (1,0); 2,783 (1,4); 2,766 (1,1); 2,749 (0,4); 2,511 (3,6); 2,507 (7,4); 2,503 (9,8); 2,498 (7,3); 2,494 (3,8); 2,425 (11,8); 2,400 (0,4); 2,383 (0,7); 2,244 (0,7); 1,397 (1,1); 1,213 (16,0); 1,195 (15,5); 0,984 (0,4); 0,000 (9,2); -0,008 (0,4) |
| Beispiel 32: ¹H-NMR(400,0 MHz, DMSO): |
| δ = 8,622 (4,9); 7,887 (2,9); 7,868 (2,9); 7,475 (2,5); 7,448 (2,5); 6,982 (1,4); 6,847 (7,9); 6,712 (1,6); 5,756 (1,9); 4,056 (1,3); 4,030 (3,9); 4,005 (4,1); 3,979 (1,4); 3,323 (47,5); 2,675 (0,3); 2,671 (0,5); 2,666 (0,4); 2,524 (1,1); 2,510 (27,2); 2,506 (56,2); 2,502 (75,5); 2,497 (56,6); 2,493 (28,9); 2,457 (0,5); 2,437 (16,0); 2,333 (0,4); 2,328 (0,5); 2,324 (0,4); 2,086 (1,3); 0,008 (0,4); 0,000 (11,8); -0,008 (0,5) |
| Beispiel 33: ¹H-NMR(400,0 MHz, DMSO): |
| δ = 8,650 (2,6); 8,627 (3,9); 8,414 (0,3); 8,317 (0,7); 8,098 (0,4); 7,958 (3,9); 7,954 (5,4); 7,549 (0,4); 7,465 (2,4); 7,439 (3,5); 7,172 (6,7); 7,162 (0,4); 5,757 (1,0); 4,303 (0,5); 4,276 (0,6); 4,266 (0,7); 4,239 (0,7); 4,232 (0,3); 4,214 (0,4); 4,167 (0,6); 4,156 (0,5); 4,140 (0,7); 4,130 (1,1); 4,103 (1,2); 4,081 (1,1); 4,054 (1,2); 4,045 (0,6); 4,027 (0,5); 4,018 (0,6); 3,940 (0,6); 3,914 (0,7); 3,904 (0,6); 3,877 (0,5); 3,331 (446,6); 2,692 (0,9); 2,676 (1,5); 2,671 (2,1); 2,666 (1,5); 2,591 (1,1); 2,524 (5,0); 2,511 (114,9); 2,507 (234,5); 2,502 (309,1); 2,498 (226,9); 2,493 (111,8); 2,428 (16,0); 2,333 (1,6); 2,329 (2,2); 2,324 (1,7); 2,173 (1,0); 2,153 (12,5); 2,143 (8,7); 1,351 (0,6); 1,310 (0,4); 1,293 (0,5); 1,259 (0,4); 1,234 (0,9); 1,184 (0,4); 1,175 (0,3); 0,008 (1,1); 0,000 (36,2); -0,008 (1,4) |
| Beispiel 34: ¹H-NMR(400,0 MHz, DMSO): |
| δ = 8,703 (12,7); 8,316 (1,1); 8,100 (10,6); 8,094 (10,9); 7,905 (5,5); 7,884 (16,0); 7,866 (10,0); 7,861 (9,1); 7,845 (3,3); 7,839 (3,5); 7,137 (13,3); 4,394 (0,7); 4,367 (2,5); 4,357 (1,2); 4,340 (2,8); 4,330 (3,5); 4,313 (1,1); 4,303 (3,6); 4,276 (1,1); 4,165 (0,9); 4,139 (3,3); 4,129 (0,9); 4,112 (3,7); 4,102 (2,7); 4,085 (1,4); 4,075 (2,7); 4,048 (0,9); 3,325 (469,0); 3,303 (1,1); 2,679 (0,8); 2,675 (1,6); 2,671 (2,2); 2,666 (1,6); 2,661 (0,8); 2,524 (5,7); 2,511 (122,2); 2,506 (248,6); 2,502 (330,0); 2,497 (238,1); 2,493 (113,6); 2,337 (0,8); 2,333 (1,6); 2,328 (2,2); 2,324 (1,6); 1,235 (0,4); 0,146 (2,2); 0,026 (0,5); 0,019 (1,1); 0,008 (18,3); 0,000 (502,6); -0,009 (18,2); -0,023 (0,9); -0,032 (0,4); -0,150 (2,2) |
| Beispiel 35: ¹H-NMR(400,0 MHz, DMSO): |
| δ = 8,317 (0,7); 8,251 (4,6); 7,731 (4,5); 7,715 (3,1); 7,426 (2,2); 7,406 (3,1); 6,480 (4,1); 6,479 (4,3); 6,474 (3,3); 6,472 (3,1); 4,264 (0,4); 4,237 (0,5); 4,228 (0,6); 4,201 (0,6); 4,141 (0,7); 4,114 (2,4); 4,087 (2,5); 4,060 (0,9); 4,023 (0,6); 3,996 (0,6); 3,986 (0,5); 3,959 (0,5); 3,362 (0,5); 3,330 (358,6); 2,675 (2,0); 2,671 (2,5); 2,667 (1,6); 2,524 (4,8); 2,520 (8,0); 2,511 (118,7); 2,507 (244,5); 2,502 (323,9); 2,498 (237,1); 2,493 (116,6); 2,436 (0,6); 2,412 (16,0); 2,333 (1,6); 2,329 (2,2); 2,324 (1,7); 2,133 (0,8); 2,110 (11,5); 2,102 (8,4); 1,997 (0,8); 1,983 (1,2); 1,976 (1,2); 1,967 (0,9); 1,351 (0,6); 1,259 (0,4); 1,232 (0,9); 1,025 (0,8); 1,004 (6,4); 1,000 (5,5); 0,991 (4,8); 0,988 (4,4); 0,980 (2,8); 0,008 (1,1); 0,000 (38,4); -0,009 (1,4) |
| Beispiel 36: ¹H-NMR(400,0 MHz, DMSO): |
| δ = 8,611 (5,0); 7,663 (5,8); 7,333 (4,5); 7,177 (5,7); 7,175 (5,7); 5,757 (1,1); 4,037 (1,2); 4,011 (3,9); 3,985 (4,0); 3,959 (1,4); 3,346 (5,5); 2,525 (0,5); 2,520 (0,8); 2,511 (12,0); 2,507 (24,8); 2,502 (33,0); 2,498 (24,4); 2,493 (12,1); 2,470 (0,8); 2,411 (0,8); 2,383 (15,2); 2,329 (0,7); 2,199 (0,4); 2,038 (16,0); 1,293 (0,4); 0,000 (8,6) |
| Beispiel 37: ¹H-NMR(600,1 MHz, CD₃CN): |
| δ = 8,110 (0,3); 8,106 (0,3); 8,012 (3,8); 7,598 (2,5); 7,586 (2,5); 7,499 (0,3); 7,486 (0,3); 7,264 (2,1); 7,246 (2,1); 6,357 (4,7); 6,355 (4,5); 3,716 (0,8); 3,704 (0,6); 3,629 (1,6); 3,625 (0,4); 3,612 (4,9); 3,608 (1,0); 3,596 (5,0); 3,592 (1,0); 3,579 (1,7); 3,575 (0,4); 3,550 (0,3); 2,499 (16,0); 2,465 (0,9); 2,456 (2,3); 2,411 (0,4); 2,133 (4,3); 1,955 (0,4); 1,951 (0,6); 1,947 (4,5); 1,943 (8,1); 1,939 (11,9); 1,935 (8,0); 1,931 (4,0); 1,914 (0,4); 1,906 (0,7); 1,900 (0,8); 1,893 (1,3); 1,885 (0,8); 1,879 (0,8); 1,871 (0,4); 1,077 (0,5); 1,055 (0,6); 1,042 (1,8); 1,034 (2,2); 1,030 (3,3); 1,026 (2,6); 1,022 (2,9); 1,017 (1,3); 1,012 (0,7); 1,005 (1,7); 1,000 (2,9); 0,995 (2,2); 0,991 (1,4); 0,986 (3,1); 0,982 (1,7); 0,976 (0,6); 0,974 (0,6); 0,000 (1,2) |
| Beispiel 39: ¹H-NMR(400,0 MHz, DMSO): |
| δ = 8,250 (5,3); 7,517 (5,6); 7,293 (4,5); 6,345 (4,6); 6,343 (4,7); 5,755 (4,4); 4,048 (1,0); 4,023 (3,2); 3,997 (3,3); 3,971 (1,1); 3,321 (17,3); 2,579 (1,3); 2,561 (3,8); 2,542 (4,0); 2,523 (2,0); 2,510 (14,4); 2,506 (29,9); 2,501 (40,7); 2,497 (31,3); 2,493 (16,6); 2,370 (15,3); 2,328 (0,3); 2,001 (16,0); 1,221 (5,8); 1,202 (12,4); 1,184 (5,6); 0,008 (1,5); 0,000 (42,9); -0,008 (2,4) |
| Beispiel 40: ¹H-NMR(400,0 MHz, DMSO): |
| δ = 8,412 (8,2); 8,193 (4,1); 8,186 (4,2); 7,981 (3,7); 7,976 (3,8); 7,699 (1,7); 7,694 (1,7); 7,679 (2,3); 7,673 (2,3); 7,569 (3,1); 7,548 (2,3); 4,254 (0,9); 4,244 (0,4); 4,227 (1,0); 4,217 (1,3); 4,199 (0,4); 4,190 (1,3); 4,163 (0,4); 4,090 (0,4); 4,063 (1,2); 4,054 (0,4); 4,036 (1,4); 4,027 (0,9); 4,009 (0,5); 4,000 (0,9); 3,325 (44,5); 2,670 (0,4); 2,524 (1,3); 2,506 (50,5); 2,501 (65,8); 2,497 (48,8); 2,454 (16,0); 2,333 (0,3); 2,328 (0,4); 2,324 (0,3); 1,236 (0,4); 0,000 (5,1) |
| Beispiel 41: ¹H-NMR(400,0 MHz, DMSO): |
| δ = 8,739 (14,3); 8,316 (2,9); 8,262 (11,1); 8,243 (11,3); 8,228 (10,1); 8,205 (10,1); 7,839 (0,5); 7,814 (0,4); 7,195 (16,0); 5,756 (6,3); 4,361 (0,4); 4,340 (1,0); 4,305 (1,5); 4,279 (1,3); 4,179 (0,4); 4,154 (0,7); 4,128 (2,0); 4,101 (2,3); 4,092 (1,8); 4,074 (1,1); 4,064 (1,6); 4,038 (0,6); 3,845 (2,2); 3,324 (955,8); 2,675 (4,3); 2,671 (5,9); 2,666 (4,3); 2,662 (2,0); 2,524 (15,5); 2,511 (332,6); 2,506 (669,9); 2,502 (882,7); 2,497 (635,7); 2,493 (303,8); 2,337 (2,1); 2,333 (4,2); 2,328 (5,8); 2,324 (4,2); 2,086 (0,4); 1,257 (0,6); 1,235 (0,9); 1,215 (0,4); 1,183 (0,4); 1,166 (0,3); 0,146 (1,0); 0,008 (7,8); 0,000 (237,7); -0,009 (7,9); -0,150 (1,0) |
| Beispiel 43: ¹H-NMR(400,0 MHz, DMSO): |
| δ = 8,342 (5,7); 7,626 (3,4); 7,622 (3,5); 7,404 (2,4); 7,384 (3,2); 7,277 (2,2); 7,273 (2,1); 7,257 (1,6); 7,253 (1,6); 6,336 (5,1); 4,117 (1,2); 4,091 (3,8); 4,066 (3,9); 4,040 (1,3); 3,326 (89,7); 2,671 (0,5); 2,560 (1,5); 2,541 (4,4); 2,522 (5,9); 2,506 (62,9); 2,502 (80,4); 2,497 (60,3); 2,389 (16,0); 2,333 (0,4); 2,328 (0,5); 1,212 (5,5); 1,193 (11,4); 1,174 (5,3); 0,000 (8,5) |
| Beispiel 44: ¹H-NMR(400,0 MHz, DMSO): |
| δ = 8,360 (5,0); 7,796 (2,9); 7,778 (2,9); 7,438 (2,5); 7,411 (2,5); 6,398 (4,4); 5,757 (1,7); 4,059 (1,2); 4,033 (3,9); 4,007 (4,1); 3,981 (1,4); 3,326 (68,0); 2,675 (0,4); 2,671 (0,6); 2,666 (0,4); 2,524 (1,4); 2,511 (32,7); 2,506 (66,0); 2,502 (87,1); 2,497 (64,1); 2,493 (31,8); 2,427 (16,0); 2,378 (0,4); 2,368 (0,6); 2,333 (0,5); 2,329 (0,6); 2,324 (0,5); 2,268 (14,5); 2,066 (0,4); 0,000 (5,8) |
| Beispiel 45: ¹H-NMR(400,0 MHz, DMSO): |
| δ = 8,550 (2,5); 8,532 (3,5); 7,854 (7,4); 7,460 (2,2); 7,437 (3,2); 6,766 (4,1); 6,760 (3,1); 4,292 (0,4); 4,265 (0,5); 4,255 (0,6); 4,228 (0,6); 4,155 (0,4); 4,137 (0,5); 4,128 (1,3); 4,102 (1,7); 4,076 (1,3); 4,068 (0,5); 4,049 (0,4); 4,040 (0,3); 3,986 (0,5); 3,960 (0,6); 3,950 (0,5); 3,923 (0,5); 3,328 (8,8); 2,505 (17,6); 2,501 (23,0); 2,497 (17,5); 2,428 (16,0); 2,144 (11,4); 2,136 (8,4); 2,086 (3,4); 2,002 (3,4); 1,954 (7,2); 1,951 (6,5); 1,906 (3,7); 1,903 (3,3); 0,000 (0,9) |
| Beispiel 46: ¹H-NMR(400,0 MHz, DMSO): |
| δ = 8,428 (5,0); 8,317 (0,4); 8,020 (3,1); 8,002 (3,1); 7,568 (2,4); 7,541 (2,3); 6,422 (4,5); 4,259 (0,5); 4,251 (0,3); 4,232 (0,6); 4,223 (0,8); 4,196 (0,8); 4,127 (0,9); 4,100 (1,0); 4,091 (0,7); 4,073 (0,4); 4,064 (0,6); 3,328 (131,6); 2,729 (0,5); 2,676 (0,8); 2,671 (1,2); 2,666 (0,9); 2,662 (0,4); 2,524 (2,8); 2,520 (4,2); 2,511 (60,6); 2,506 (125,9); 2,502 (168,1); 2,497 (123,8); 2,493 (61,0); 2,468 (16,0); 2,338 (0,4); 2,333 (0,9); 2,328 (1,6); 2,280 (14,4); 2,105 (0,4); 1,234 (0,4); 0,008 (1,2); 0,000 (41,0); -0,009 (1,5) |
| Beispiel 47: ¹H-NMR(400,0 MHz, DMSO): |
| δ = 8,642 (4,8); 7,721 (3,3); 7,716 (3,4); 7,444 (2,2); 7,424 (3,3); 7,357 (2,5); 7,352 (2,4); 7,337 (1,6); 7,332 (1,6); 7,100 (5,1); 4,098 (1,2); 4,073 (4,0); 4,047 (4,1); 4,021 (1,4); 3,324 (40,9); 2,671 (0,4); 2,524 (0,9); 2,519 (1,4); 2,511 (20,0); 2,506 (41,1); 2,502 (54,6); 2,497 (39,7); 2,492 (19,0); 2,400 (16,0); 2,333 (0,6); 2,328 (0,5); 2,324 (0,3); 0,008 (1,8); 0,000 (55,9); -0,009 (1,9) |
| Beispiel 48: ¹H-NMR(400,0 MHz, DMSO): |
| δ = 8,317 (0,7); 8,166 (5,0); 8,055 (0,5); 7,718 (5,2); 7,703 (3,7); 7,623 (0,4); 7,435 (2,6); 7,415 (3,6); 4,274 (0,6); 4,247 (0,6); 4,237 (0,7); 4,210 (0,7); 4,149 (1,0); 4,140 (0,4); 4,122 (3,0); 4,112 (0,5); 4,095 (3,1); 4,068 (1,1); 4,015 (0,7); 3,988 (0,8); 3,978 (0,6); 3,951 (0,6); 3,354 (1,2); 3,328 (188,4); 2,680 (0,7); 2,676 (1,1); 2,671 (1,4); 2,666 (1,1); 2,662 (0,6); 2,574 (0,3); 2,524 (3,6); 2,520 (5,5); 2,511 (74,3); 2,506 (152,5); 2,502 (202,5); 2,497 (147,9); 2,493 (71,9); 2,415 (14,9); 2,365 (1,2); 2,353 (0,8); 2,338 (0,5); 2,333 (1,1); 2,329 (1,5); 2,324 (1,2); 2,319 (0,9); 2,307 (12,9); 2,246 (1,2); 2,149 (0,8); 2,127 (0,3); 2,099 (15,7); 2,031 (0,4); 2,007 (16,0); 1,234 (0,6); 1,031 (0,4); 1,013 (0,7); 0,995 (0,3); 0,008 (1,5); 0,000 (51,5); -0,009 (1,7) |
| Beispiel 49: ¹H-NMR(400,0 MHz, DMSO): |
| δ = 8,638 (2,3); 8,621 (3,3); 8,317 (0,7); 7,918 (4,8); 7,465 (2,1); 7,439 (3,0); 7,132 (4,6); 4,301 (0,4); 4,273 (0,5); 4,263 (0,5); 4,236 (0,5); 4,167 (0,4); 4,140 (0,5); 4,130 (1,0); 4,104 (1,0); 4,082 (0,9); 4,055 (1,0); 4,046 (0,4); 4,028 (0,4); 4,019 (0,4); 3,946 (0,5); 3,919 (0,6); 3,909 (0,4); 3,883 (0,5); 3,325 (200,0); 2,675 (1,7); 2,671 (2,3); 2,666 (1,7); 2,524 (5,6); 2,506 (258,8); 2,502 (340,1); 2,497 (251,2); 2,428 (16,0); 2,333 (1,8); 2,328 (2,4); 2,324 (1,8); 2,153 (11,1); 2,146 (8,1); 0,146 (1,2); 0,008 (8,9); 0,000 (277,2); -0,008 (10,7); -0,150 (1,3) |
| Beispiel 50: ¹H-NMR(400,0 MHz, DMSO): |
| δ = 8,700 (5,0); 8,316 (0,5); 8,011 (3,7); 8,005 (3,9); 7,698 (1,8); 7,692 (1,7); 7,677 (2,4); 7,672 (2,4); 7,570 (3,1); 7,549 (2,3); 7,114 (5,2); 5,756 (0,7); 4,259 (0,9); 4,249 (0,4); 4,232 (1,0); 4,222 (1,3); 4,205 (0,4); 4,195 (1,3); 4,168 (0,4); 4,059 (0,3); 4,031 (1,2); 4,022 (0,4); 4,004 (1,3); 3,995 (1,0); 3,977 (0,5); 3,968 (1,0); 3,941 (0,3); 3,324 (164,1); 2,732 (0,6); 2,675 (0,9); 2,671 (1,2); 2,666 (0,9); 2,524 (3,5); 2,510 (69,4); 2,506 (137,1); 2,501 (179,6); 2,497 (130,3); 2,493 (62,9); 2,455 (16,0); 2,427 (0,4); 2,333 (0,9); 2,328 (1,2); 2,324 (0,9); 2,301 (0,4); 0,008 (1,8); 0,000 (44,1); -0,009 (1,4) |
| Beispiel 51: ¹H-NMR(400,0 MHz, DMSO): |
| δ = 8,490 (4,8); 7,990 (0,4); 7,607 (5,7); 7,385 (0,3); 7,320 (4,5); 6,753 (5,3); 5,756 (0,5); 4,049 (1,2); 4,024 (3,9); 3,998 (4,0); 3,972 (1,4); 3,330 (31,0); 2,524 (0,5); 2,519 (0,8); 2,511 (11,0); 2,506 (22,7); 2,502 (30,0); 2,497 (22,2); 2,493 (11,1); 2,376 (15,2); 2,347 (0,6); 2,338 (1,2); 2,329 (0,4); 2,145 (1,1); 2,055 (0,5); 2,027 (16,0); 2,002 (4,3); 1,954 (9,8); 1,906 (4,9); 0,987 (0,5); 0,969 (0,7); 0,008 (1,7); 0,000 (48,0); -0,009 (2,0) |
| Beispiel 52: ¹H-NMR(400,0 MHz, DMSO): |
| δ = 8,460 (4,2); 8,038 (2,4); 8,020 (2,4); 7,570 (1,9); 7,543 (1,9); 6,370 5,756 (15,0); 4,262 (0,4); 4,234 (0,5); 4,225 (0,7); 4,198 (0,6); 4,131 (0,7); 4,104 (0,8); 4,095 (0,5); 4,068 (0,5); 3,330 (14,1); 2,827 (0,4); 2,810 (1,0); 2,793 (1,4); 2,776 (1,0); 2,759 (0,4); 2,524 (0,4); 2,511 (7,2); 2,507 (14,2); 2,502 (18,4); 2,498 (13,7); 2,472 (11,8); 1,216 (16,0); 1,199 (15,6); 0,000 (4,0) |
| Beispiel 53: ¹H-NMR(400,0 MHz, DMSO): |
| δ = 8,714 (3,0); 8,680 (4,0); 8,316 (0,7); 8,194 (9,3); 7,845 (3,3); 7,819 (4,4); 7,195 (4,5); 7,189 (3,5); 5,756 (0,3); 4,401 (0,6); 4,374 (0,7); 4,364 (0,7); 4,337 (0,8); 4,246 (0,7); 4,237 (0,4); 4,219 (0,8); 4,210 (1,1); 4,191 (0,4); 4,183 (1,1); 4,156 (0,4); 4,071 (1,0); 4,044 (1,1); 4,035 (0,8); 4,018 (0,6); 4,008 (0,8); 3,992 (0,7); 3,981 (0,5); 3,966 (0,8); 3,955 (0,7); 3,939 (0,3); 3,929 (0,7); 3,324 (258,8); 2,739 (0,3); 2,675 (1,2); 2,671 (1,6); 2,666 (1,2); 2,524 (4,5); 2,510 (90,1); 2,506 (181,6); 2,502 (240,3); 2,497 (174,5); 2,493 (84,2); 2,469 (16,0); 2,333 (1,7); 2,328 (2,0); 2,324 (1,3); 0,146 (1,5); 0,008 (12,7); 0,000 (335,6); -0,009 (12,4); -0,027 (0,4); -0,150 (1,5) |
| Beispiel 54: ¹H-NMR(400,0 MHz, DMSO): |
| δ = 8,663 (15,1); 8,316 (1,6); 7,888 (10,5); 7,882 (10,6); 7,732 (13,1); 7,711 (15,4); 7,479 (8,1); 7,474 (7,8); 7,458 (6,9); 7,452 (6,8); 7,137 (16,0); 4,221 (3,5); 4,196 (11,1); 4,170 (11,5); 4,145 (3,9); 3,324 (386,1); 2,675 (2,6); 2,671 (3,5); 2,666 (2,6); 2,524 (9,9); 2,510 (197,9); 2,506 (393,6); 2,502 (514,9); 2,497 (372,2); 2,493 (178,9); 2,333 (2,5); 2,328 (3,3); 2,324 (2,4); 0,146 (1,8); 0,008 (15,3); 0,000 (403,7); -0,009 (14,3); -0,150 (1,7) |
| Beispiel 55: ¹H-NMR(400,0 MHz, DMSO): |
| δ = 8,668 (4,7); 8,315 (0,4); 8,147 (3,0); 8,128 (3,1); 7,598 (2,2); 7,571 (2,2); 6,986 (1,3); 6,852 (5,3); 6,716 (1,6); 5,756 (0,3); 4,275 (0,4); 4,249 (0,5); 4,240 (0,6); 4,213 (0,6); 4,092 (0,6); 4,065 (0,7); 4,056 (0,6); 4,038 (0,3); 4,029 (0,5); 3,323 (140,2); 2,740 (0,5); 2,679 (0,4); 2,675 (0,7); 2,671 (1,0); 2,666 (0,8); 2,661 (0,4); 2,524 (2,4); 2,519 (3,7); 2,510 (55,7); 2,506 (116,1); 2,501 (156,5); 2,497 (114,8); 2,492 (56,5); 2,478 (16,0); 2,333 (0,9); 2,328 (1,1); 2,324 (0,8); 2,319 (0,4); 0,000 (0,4) |
| Beispiel 56: ¹H-NMR(400,0 MHz, DMSO): |
| δ = 8,638 (2,4); 8,621 (3,4); 7,918 (5,0); 7,464 (2,1); 7,439 (3,1); 7,131 (4,8); 4,300 (0,4); 4,273 (0,5); 4,263 (0,6); 4,236 (0,6); 4,167 (0,4); 4,157 (0,3); 4,140 (0,5); 4,130 (1,0); 4,103 (1,1); 4,082 (1,0); 4,055 (1,1); 4,045 (0,5); 4,028 (0,4); 4,018 (0,5); 3,945 (0,5); 3,918 (0,6); 3,909 (0,5); 3,882 (0,5); 3,325 (56,3); 2,675 (0,5); 2,671 (0,7); 2,666 (0,5); 2,524 (1,8); 2,510 (37,0); 2,506 (72,9); 2,502 (95,9); 2,497 (71,3); 2,493 (35,6); 2,428 (16,0); 2,333 (0,5); 2,328 (0,7); 2,324 (0,5); 2,153 (11,5); 2,145 (8,4); 0,146 (0,4); 0,008 (3,1); 0,000 (80,1); -0,008 (3,4); -0,150 (0,4) |
| Beispiel 57: ¹H-NMR(400,0 MHz, DMSO): |
| δ = 8,382 (5,0); 7,806 (2,9); 7,788 (2,9); 7,438 (2,5); 7,411 (2,5); 6,368 (4,5); 6,366 (4,6); 5,756 (1,5); 4,066 (1,2); 4,041 (3,9); 4,015 (4,1); 3,989 (1,4); 3,324 (45,7); 2,675 (0,4); 2,671 (0,5); 2,666 (0,4); 2,574 (1,3); 2,555 (4,0); 2,536 (4,2); 2,510 (27,3); 2,506 (54,9); 2,502 (73,2); 2,497 (55,5); 2,493 (29,3); 2,426 (16,0); 2,333 (0,4); 2,328 (0,5); 2,324 (0,4); 1,213 (5,8); 1,195 (12,3); 1,176 (5,7); 0,008 (1,9); 0,000 (50,4); -0,009 (2,8) |
| Beispiel 58: ¹H-NMR(400,0 MHz, DMSO): |
| δ = 8,581 (5,7); 8,316 (0,5); 8,155 (0,4); 8,146 (2,6); 8,141 (3,2); 8,133 (3,0); 8,122 (3,0); 7,889 (2,9); 7,871 (2,9); 7,555 (0,9); 7,546 (6,6); 7,540 (6,3); 7,532 (3,9); 7,529 (4,1); 7,519 (1,1); 7,505 (0,4); 7,473 (2,6); 7,447 (2,6); 7,132 (6,0); 5,756 (0,9); 4,079 (1,2); 4,053 (3,8); 4,027 (4,0); 4,001 (1,4); 3,324 (139,4); 2,675 (0,8); 2,671 (1,2); 2,666 (0,9); 2,506 (133,0); 2,502 (176,5); 2,497 (131,1); 2,446 (16,0); 2,401 (0,4); 2,333 (0,9); 2,328 (1,2); 2,324 (1,0); 0,146 (1,0); 0,008 (8,0); 0,000 (207,3); -0,008 (8,4); -0,150 (1,0) |
| Beispiel 59: ¹H-NMR(400,0 MHz, DMSO): |
| δ = 8,691 (4,6); 8,316 (0,3); 7,917 (6,2); 7,676 (5,2); 7,203 (5,0); 5,756 (0,6); 4,116 (0,8); 4,112 (0,8); 4,104 (0,3); 4,090 (2,3); 4,087 (2,3); 4,065 (2,3); 4,061 (2,3); 4,039 (0,8); 4,035 (0,8); 3,324 (85,4); 2,675 (0,6); 2,671 (0,8); 2,666 (0,6); 2,524 (2,2); 2,511 (43,3); 2,506 (85,9); 2,502 (112,3); 2,497 (81,1); 2,493 (38,9); 2,406 (16,0); 2,364 (0,6); 2,333 (0,5); 2,328 (0,7); 2,324 (0,5); 0,146 (0,4); 0,008 (3,1); 0,000 (86,6); -0,009 (3,2); -0,150 (0,4) |

### Anwendungsbeispiele:

### 1. Boophilus microplus-Injektionstest

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken (*Boophilus microplus*) injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigten z.B. die folgenden Verbindungen der Herstellungsbeispiele eine Wirkung von 100% bei einer Aufwandmenge von 20µg/Tier: 2, 4, 5, 6, 9, 22, 26, 34, 47, 50, 53, 59

### 2. Meloidogyne incognita-Test

| | | |
|---|---|---|
| Lösungsmittel: | 125,0 | Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigten z. B. die folgenden Verbindungen der Herstellungsbeispiele Wirkung von 100% bei einer Aufwandmenge von 20 ppm: 18, 21, 33, 45, 55

Bei diesem Test zeigten z. B. die folgenden Verbindungen der Herstellungsbeispiele Wirkung von 90% bei einer Aufwandmenge von 20 ppm: 9, 12, 18, 21, 26, 27, 30, 41, 55

### 3. Phaedon cochleariae - Sprühtest

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigten z. B. die folgenden Verbindungen der Herstellungsbeispiele Wirkung von 100% bei einer Aufwandmenge von 500g/ha: 2, 9, 44, 53, 55

Bei diesem Test zeigten z. B. die folgenden Verbindungen der Herstellungsbeispiele Wirkung von 83% bei einer Aufwandmenge von 500 g/ha: 18, 34, 46

### 4. Tetranychus urticae - Sprühtest, OP-resistent

| | | |
|---|---|---|
| Lösungsmittel: | 78,0 | GewichtsteileAceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben (*Phaseolus vulgaris*)*,* die von allen Stadien der Gemeinen Spinnmilbe (*Tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele eine Wirkung von 100% bei einer Aufwandmenge von 500g/ha: 1, 2, 4, 5, 6, 7, 8, 9, 10, 13, 14, 15, 16, 17, 18, 19, 20, 22, 23, 26, 29, 30, 31, 32, 33, 34, 36, 37, 39, 40, 41, 44, 45, 46, 47, 50, 51, 52, 53, 54, 55, 57, 59

Bei diesem Test zeigten z. B. die folgenden Verbindungen der Herstellungsbeispiele eine Wirkung von 90% bei einer Aufwandmenge von 500g/ha: 12, 24, 28, 35, 43, 48

### 5. Meloidogyne incognita-Test

| | | |
|---|---|---|
| Lösungsmittel: | 7 | Gewichtsteile Dimethylformamid |
| Emulgator: | 2,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration, wobei das Erdvolumen in das gedrencht wird, mitberücksichtigt werden muss. Es ist darauf zu achten, dass in der Erde eine Konzentration von 20 ppm Emulgator nicht überschritten wird. Zur Herstellung weiterer Testkonzentrationen wird mit Wasser verdünnt.

Mit Erde (lehmiger Sand) gefüllte Töpfe werden mit der Wirkstofflösung angegossen. Eine Ei-Larven-Suspension des südlichen Wurzelgallenälchens (*Meloidogyne incognita*) wird hinzugegeben, die Erdoberfläche mit Salatsamen bestreut und mit Quarzsand abgedeckt. Die Salatsamen keimen und die Pflänzchen entwickeln sich. An den Wurzeln entwickeln sich die Gallen.

Nach 21 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigten z. B. die folgenden Verbindungen der Herstellungsbeispiele Wirkung von 85% bei einer Aufwandmenge von 8 ppm: 2

### 6. Tetranychus urticae- Sprühtest; OP-resistent

| | |
|---|---|
| Lösungsmittel: | 7 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Bohnenpflanzen (*Phaseolus vulgaris*)*,* die stark von allen Stadien der Gemeinen Spinnmilbe (*Tetranychus urticae*) befallen sind, werden durch Spritzen mit der Wirkstoffzubereitung in der gewünschten Konzentration behandelt.

Nach 7 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele Wirkung von 100% bei einer Aufwandmenge von 20 ppm: 49, 56

### Vergleichsbeispiele:

### 1. Phaedon cochleariae - Sprühtest (PHAECO)

| | | |
|---|---|---|
| Lösungsmittel: | 78,0 | Gewichtsteile Aceton |
| | 1,5 | Gewichtsteile Dimethylformamid |
| Emulgator: | Alky larylpolygl ykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigten z. B. die folgenden Verbindungen der Herstellungsbeispiele überlegene Wirksamkeit gegenüber 4-[6-Oxo-4-(trifluoromethyl)pyrimidin-1-yl]-2-(2,2,2-trifluoroethylsulfinyl)benzonitril und 5-Fluoro-4-[6-oxo-4-(trifluoromethyl)pyrimidin-1-yl]-2-(2,2,2-trifluoroethylsulfinyl)benzonitril: siehe nachstehende Tabelle 5

### 2. Tetranychus urticae - Sprühtest ; OP-resistent (TETRUR)

| | | |
|---|---|---|
| Lösungsmittel: | 78,0 | Gewichtsteile Aceton |
| | 1,5 | Gewichtsteile Dimethylformamid |
| Emulgator: | Alky larylpolygl ykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben (*Phaseolus vulgaris*)*,* die von allen Stadien der Gemeinen Spinnmilbe (*Tetranychus urticae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele überlegene Wirksamkeit gegenüber 4-[6-Oxo-4-(trifluoromethyl)pyrimidin-1-yl]-2-(2,2,2-trifluoroethylsulfinyl)benzonitril und 5-Fluoro-4-[6-oxo-4-(trifluoromethyl)pyrimidin-1-yl]-2-(2,2,2-trifluoroethylsulfinyl)benzonitril: siehe nachstehende Tabelle 5

**Tabelle 5:**

| Substanz | Struktur | Tierart | Konzentration | % dat | Mortalität |
|---|---|---|---|---|---|
| 4-[6-Oxo-4-(trifluoromethyl)pyrimidin-1-yl]-2-(2,2,2-trifluoroethylsulfinyl)benzonitril | 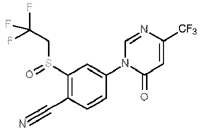 | PHAECO | 500 g/ha | 0 | 7dat |
| | | TETRUR | 20 g/ha | 0 | 6dat |
| Beispiel 2 erfindungsgemäß | 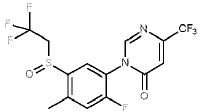 | PHAECO | 500 g/ha | 100 | 7dat |
| Beispiel 6 erfindungsgemäß | 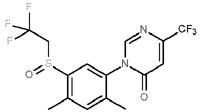 | TETRUR | 20 g/ha | 100 | 6dat |
| 5-Fluoro-4-[6-oxo-4-(trifluoromethyl)pyrimidin-1-yl]-2-(2,2,2-trifluoroethylsulfinyl)benzonitril | 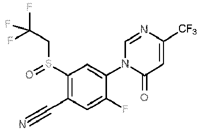 | PHAECO | 500 g/ha | 50 | 7dat |

## Patentansprüche

1. Verbindung der Formel (I) 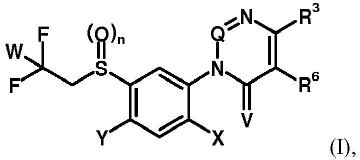 worin
V für Sauerstoff steht;
Q für C-R⁵ oder Stickstoff steht, wobei
R⁵ für Wasserstoff, Halogen, Amino, Cyano, Nitro, (C₁-C₆)Alkyl, (C₁-C₆)Halogenalkyl, (C₁-C₆)Alkylsulfanyl, (C₁-C₆)Alkylsulfinyl, (C₁-C₆)Alkylsulfonyl, (C₁-C₆)Halogenalkylsulfanyl, (C₁-C₆)Halogenalkylsulfinyl, (C₁-C₆)Halogenalkylsulfonyl, (C₃-C₆)Cycloalkyl oder (C₁-C₆)Alkoxy steht;
R³ und R⁶ unabhängig voneinander
für Wasserstoff, Halogen, Cyano oder Nitro stehen; oder
für (C₁-C₆)Alkyl, (C₁-C₆)Halogenalkyl, (C₂-C₆)Alkenyl, (C₂-C₆)Alkinyl, (C₁-C₆)Alkoxy, (C₁-C₆)Halogenalkoxy, (C₁-C₆)Halogenalkylsulfanyl, (C₁-C₆)Halogenalkylsulfinyl, (C₁-C₆)Halogenalkylsulfonyl, (C₁-C₆)Alkylsulfanyl, (C₁-C₆)Alkylsulfinyl, (C₁-C₆)Alkylsulfonyl, (C₁-C₅)Alkylcarbonyl, (C₁-C₅)Halogenalkylcarbonyl oder (C₂-C₆)Alkoxycarbonyl stehen; oder
für (C₃-C₆)Cycloalkyl, (C₃-C₆)Cycloalkyl(C₁-C₃)alkyl oder (C₃-C₆)Cycloalkenyl stehen, wobei die vorgenannten Reste gegebenenfalls durch Halogen, Alkyl, Cyano, Nitro, Alkoxy, Halogenalkyl oder Halogenalkoxy substituiert sein können; oder
für Phenyl, Heteroaryl, Phenyl(C₁-C₃)alkyl oder Heteroaryl(C₁-C₃)alkyl stehen, wobei die vorgenannten Reste gegebenenfalls durch Halogen, Alkyl, Cyano, Nitro, Alkoxy, Halogenalkyl oder Halogenalkoxy substituiert sein können; oder
für NR'R" stehen,
wobei R' und R" unabhängig voneinander für Wasserstoff, Cyano, (C₁-C₄)Alkyl, (C₁-C₄)Halogenalkyl, (C₁-C₄)Cyanoalkyl, (C₁-C₄)Hydroxyalkyl, (C₁-C₄)Alkoxy(C₁-C₄)alkyl, (C₁-C₄)Alkylsulfanyl(C₁-C₄)alkyl, (C₁-C₄)Alkylsulfinyl(C₁-C₄)alkyl, (C₁-C₄)Alkylsulfonyl(C₁-C₄)alkyl, (C₃-C₄)Alkenyl, (C₃-C₄)Halogenalkenyl, (C₂-C₄)Cyanoalkenyl, (C₃-C₄)Alkinyl, (C₃-C₄)Halogenalkinyl, (C₃-C₄)Cyanoalkinyl, (C₁-C₄)Alkylcarbonyl oder (C₁-C₄)Alkoxycarbonyl stehen;
oder
wobei R' und R" gemeinsam mit dem N-Atom, an das sie gebunden sind, einen gegebenenfalls durch Halogen, Alkyl, Cyano, Nitro, Alkoxy, Halogenalkyl oder Halogenalkoxy substituierten und gegebenenfalls durch ein oder mehrere Heteroatome, welche unabhängig ausgewählt werden aus der Gruppe bestehend aus O, S und N mit der Maßgabe, dass zwei Sauerstoffatome nicht unmittelbar benachbart zueinander stehen, unterbrochenen gesättigten oder ungesättigten drei- bis achtgliedrigen Ring bilden können;
W für Wasserstoff oder Halogen steht;
n für die Zahl 0 oder 1 steht;
Y für Wasserstoff, Halogen, (C₁-C₄)Alkyl, Trifluoromethyl, (C₂-C₄)Halogenalkyl, (C₁-C₄)Alkoxy, (C₁-C₄)Halogenalkoxy oder Amino steht; oder
für NR"'R"" steht,
wobei R"' und R"" unabhängig voneinander für Wasserstoff, (C₁-C₄)Alkyl, oder (C₂-C₄)Halogenalkyl stehen;
X für Wasserstoff, Halogen, Cyano, (C₁-C₄)Alkyl, (C₁-C₄)Halogenalkyl oder (C₁-C₄)Alkoxy steht.

2. Verbindung nach Anspruch 1, worin
V für Sauerstoff steht;
Q für C-R⁵ oder Stickstoff steht, wobei
R⁵ für Wasserstoff, Halogen, Amino, Cyano, Nitro, (C₁-C₄)Alkyl, (C₁-C₄)Halogenalkyl, (C₃-C₆)Cycloalkyl oder (C₁-C₄)Alkoxy steht;
R³ und R⁶ unabhängig voneinander
für Wasserstoff oder Halogen; oder
für (C₁-C₄)Alkyl, (C₁-C₄)Halogenalkyl, (C₁-C₄)Alkoxy, (C₁-C₄)Halogenalkoxy; oder
für (C₃-C₆)Cycloalkyl oder (C₃-C₆)Cycloalkyl(C₁-C₂)alkyl stehen, wobei die vorgenannten Reste gegebenenfalls durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituiert sein können; oder
für Phenyl oder Phenyl(C₁-C₃)alkyl stehen, wobei die vorgenannten Reste gegebenenfalls einfach bis zweifach durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituiert sein können; oder
für Heteroaryl ausgewählt aus der Gruppe bestehend aus Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Indolyl, Isoindolyl und Indazolyl, stehen, wobei die vorgenannten Reste gegebenenfalls einfach bis zweifach durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituiert sein können; oder
für NR'R" stehen,
wobei R' und R" unabhängig voneinander für Wasserstoff, (C₁-C₄)Alkyl, (C₁-C₄)Halogenalkyl, (C₁-C₄)Cyanoalkyl, (C₁-C₄)Alkoxy(C₁-C₄)alkyl, (C₁-C₄)Alkylsulfanyl(C₁-C₄)alkyl, (C₁-C₄)Alkylsulfinyl(C₁-C₄)alkyl, (C₁-C₄)Alkylsulfonyl(C₁-C₄)alkyl, (C₁-C₄)Alkylcarbonyl oder (C₁-C₄)Alkoxycarbonyl stehen;
oder
wobei R' und R" gemeinsam mit dem N-Atom, an das sie gebunden sind, einen gegebenenfalls einfach bis zweifach durch Fluor, Chlor, Brom, Iod, (C₁-C₃)Alkyl, Cyano, Nitro, (C₁-C₃)Alkoxy, (C₁-C₃)Halogenalkyl oder (C₁-C₃)Halogenalkoxy substituierten und gegebenenfalls durch ein Heteroatom, welches ausgewählt ist aus der Gruppe bestehend aus O, S und N, unterbrochenen gesättigten vier- bis sechsgliedrigen Ring bilden können;
W für Wasserstoff oder Fluor steht;
n für die Zahl 0 oder 1 steht;
Y für Wasserstoff, Fluor, Chlor, Brom, Methyl, Ethyl, Trifluoromethyl, Methoxy, Ethoxy, Difluoromethoxy, Trifluoromethoxy, Amino, Methylamino oder Dimethylamino steht;
X für Wasserstoff, Fluor, Chlor, Brom, Cyano, Methyl, Ethyl, Trifluoromethyl, Methoxy oder Ethoxy steht.

3. Verbindung nach Anspruch 1 oder 2, worin
V für Sauerstoff steht;
Q für C-R⁵ steht, wobei
R⁵ für Wasserstoff, Methyl, Ethyl, Methoxy, Cyclopropyl oder Trifluoromethyl steht;
R³ und R⁶ unabhängig voneinander für Wasserstoff, Fluor, Methyl, Ethyl, Isopropyl, Trifluoromethyl, Pentafluoroethyl, Difluoromethyl, 1,1-Difluoroethyl, Difluorchlormethyl, Cyclopropyl oder Phenyl stehen;
W für Wasserstoff oder Fluor steht;
n für die Zahl 0 oder 1 steht;
Y für Fluor, Chlor, Brom, Methyl, Trifluoromethyl oder Methoxy steht;
X für Wasserstoff, Chlor, Fluor oder Methyl steht;
insbesondere wobei X und Y für folgende Kombinationen (Y,X) stehen: (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F).

4. Verbindung nach einem der Ansprüche 1 bis 3, worin
V für Sauerstoff steht;
Q für C-R⁵ steht, wobei
R⁵ für Wasserstoff oder Methyl steht;
R³ für Wasserstoff, Methyl, Ethyl, Cyclopropyl, Isopropyl, Difluoromethyl, Trifluoromethyl, Pentafluoroethyl, 1,1-Difluoroethyl oder Phenyl steht;
R⁶ für Wasserstoff, Fluor oder Methyl steht;
W für Fluor steht;
N für die Zahl 0 oder 1 steht;
Y für Brom, Chlor oder Methyl steht;
X für Wasserstoff, Chlor, Fluor oder Methyl steht;
insbesondere wobei X und Y für folgende Kombinationen (Y,X) stehen: (Me,H), (Me,Cl), (Me, F), (Me,Me), (Cl,Cl), (Cl,F), (Br,F).

5. Verbindung nach einem der Ansprüche 1 bis 4, in der Q für C-R⁵ steht und R⁵, R³, R⁶, W, n, Y und X die in Anspruch 1 definierten Bedeutungen oder die in Anspruch 2 definierten Bedeutungen oder die in Anspruch 3 definierten Bedeutungen oder die in Anspruch 4 definierten Bedeutungen haben.

6. Verbindung der Formel (IIa) 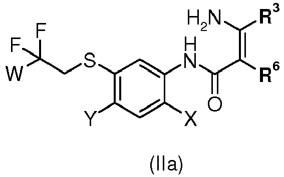 worin R³, R⁶, W, X und Y die in Anspruch 1 definierten Bedeutungen oder die in Anspruch 1 definierten Bedeutungen oder die in Anspruch 2 definierten Bedeutungen oder die in Anspruch 3 definierten Bedeutungen oder die in Anspruch 4 definierten Bedeutungen haben.

7. Verbindung der Formel (XXXI) 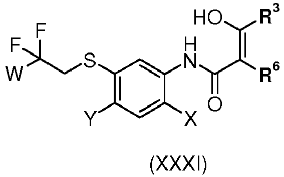 worin R³, R⁶, W, X und Y die in Anspruch 1 definierten Bedeutungen oder die in Anspruch 1 definierten Bedeutungen oder die in Anspruch 2 definierten Bedeutungen oder die in Anspruch 3 definierten Bedeutungen oder die in Anspruch 4 definierten Bedeutungen haben.

8. Formulierung, insbesondere agrochemische Formulierung, umfassend mindestens eine Verbindung der Formel (I) gemäß einem der Ansprüche 1 bis 5.

9. Formulierung nach Anspruch 8 ferner umfassend mindestens ein Streckmittel und/oder mindestens eine oberflächenaktive Substanz.

10. Formulierung nach Anspruch 8 oder 9, **dadurch gekennzeichnet, dass** die Verbindung der Formel (I) in Mischung mit mindestens einem weiteren Wirkstoff vorliegt.

11. Verfahren zur Bekämpfung von Schädlingen, insbesondere tierischen Schädlingen, **dadurch gekennzeichnet, dass** man eine Verbindung der Formel (I) gemäß einem der Ansprüche 1 bis 5 oder eine Formulierung gemäß einem der Ansprüche 8 bis 10 auf die Schädlinge und/oder ihren Lebensraum einwirken lässt, wobei Verfahren zur chirurgischen oder therapeutischen Behandlung des menschlichen oder tierischen Körpers und Diagnostizierverfahren, die am menschlichen oder tierischen Körper vorgenommen werden, ausgenommen sind.

12. Verfahren nach Anspruch 11, **dadurch gekennzeichnet, dass** der Schädling ein tierischer Schädling ist und ein Insekt, einen Akariden oder einen Nematoden umfasst, oder dass der Schädling ein Insekt, ein Akaride oder ein Nematode ist.

13. Verwendung einer Verbindung der Formel (I) gemäß einem der Ansprüche 1 bis 5 oder einer Formulierung gemäß einem der Ansprüche 8 bis 10 zur Bekämpfung von tierischen Schädlingen, wobei Verwendungen in Verfahren zur chirurgischen oder therapeutischen Behandlung des menschlichen oder tierischen Körpers und Diagnostizierverfahren, die am menschlichen oder tierischen Körper vorgenommen werden, ausgenommen sind.

14. Verwendung nach Anspruch 13, **dadurch gekennzeichnet, dass** der tierische Schädling ein Insekt, einen Akariden oder einen Nematoden umfasst, oder dass der tierische Schädling ein Insekt, ein Akaride oder ein Nematode ist.

15. Verwendung nach Anspruch 13 oder 14 im Pflanzenschutz.

16. Verwendung nach Anspruch 13 oder 14 auf dem Gebiet der Tiergesundheit.

17. Verfahren zum Schutz eines Saatgutes oder einer keimenden Pflanze vor Schädlingen, insbesondere tierischen Schädlingen, umfassend einen Verfahrensschritt, in welchem das Saatgut in Kontakt mit einer Verbindung der Formel (I) gemäß einem der Ansprüche 1 bis 5 oder mit einer Formulierung gemäß einem der Ansprüche 8 bis 10 gebracht wird.

18. Saatgut, erhalten durch ein Verfahren gemäß Anspruch 17.

## Claims

1. Compound of the formula (I) 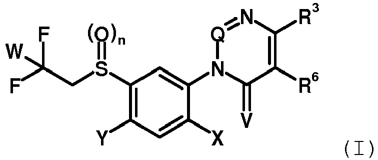 in which
V represents oxygen;
Q represents C-R⁵ or nitrogen, where
R⁵ represents hydrogen, halogen, amino, cyano, nitro, (C₁-C₆) -alkyl, (C₁-C₆)-haloalkyl, (C₁-C₆) -alkylsulphanyl, (C₁-C₆)-alkylsulphinyl, (C₁-C₆)-alkylsulphonyl, (C₁-C₆)-haloalkylsulphanyl, (C₁-C₆)-haloalkylsulphinyl, (C₁-C₆)-haloalkylsulphonyl, (C₃-C₆)-cycloalkyl or (C₁-C₆)-alkoxy;
R³ and R⁶ independently of one another
represent hydrogen, halogen, cyano or nitro; or
represent (C₁-C₆)-alkyl, (C₁-C₆)-haloalkyl, (C₂-C₆)-alkenyl, (C₂-C₆)-alkynyl, (C₁-C₆)-alkoxy, (C₁-C₆)-haloalkoxy, (C₁-C₆)-haloalkylsulphanyl, (C₁-C₆)-haloalkylsulphinyl, (C₁-C₆)-haloalkylsulphonyl, (C₁-C₆)-alkylsulphanyl, (C₁-C₆)-alkylsulphinyl, (C₁-C₆)-alkylsulphonyl, (C₁-C₅)-alkylcarbonyl, (C₁-C₅)-haloalkylcarbonyl or (C₂-C₆)-alkoxycarbonyl; or
represent (C₃-C₆)-cycloalkyl, (C₃-C₆)-cycloalkyl-(C₁-C₃)-alkyl or (C₃-C₆)-cycloalkenyl, where the radicals mentioned above may optionally be substituted by halogen, alkyl, cyano, nitro, alkoxy, haloalkyl or haloalkoxy; or
represent phenyl, heteroaryl, phenyl-(C₁-C₃)-alkyl or heteroaryl-(C₁-C₃)-alkyl, where the radicals mentioned above may optionally be substituted by halogen, alkyl, cyano, nitro, alkoxy, haloalkyl or haloalkoxy; or
represent NR'R",
where R' and R" independently of one another represent hydrogen, cyano, (C₁-C₄) -alkyl, (C₁-C₄)-haloalkyl, (C₁-C₄)-cyanoalkyl, (C₁-C₄)-hydroxyalkyl, (C₁-C₄)-alkoxy-(C₁-C₄)-alkyl, (C₁-C₄)-alkylsulphanyl-(C₁-C₄)-alkyl, (C₁-C₄)-alkylsulphinyl-(C₁-C₄)-alkyl, (C₁-C₄)-alkylsulphonyl-(C₁-C₄)-alkyl, (C₃-C₄) -alkenyl, (C₃-C₄) -haloalkenyl, (C₂-C₄)-cyanoalkenyl, (C₃-C₄)-alkynyl, (C₃-C₄)-haloalkynyl, (C₃-C₄)-cyanoalkynyl, (C₁-C₄)-alkylcarbonyl or (C₁-C₄)-alkoxycarbonyl;
or
where R' and R" together with the nitrogen atom to which they are attached may form an optionally halogen-, alkyl-, cyano-, nitro-, alkoxy-, haloalkyl- or haloalkoxy-substituted, saturated or unsaturated three- to eight-membered ring optionally interrupted by one or more heteroatoms independently selected from the group consisting of O, S and N, with the proviso that two oxygen atoms must not be directly adjacent to one another; or
W represents hydrogen or halogen;
n represents the number 0 or 1;
Y represents hydrogen, halogen, (C₁-C₄)-alkyl, trifluoromethyl, (C₂-C₄)-haloalkyl, (C₁-C₄)-alkoxy, (C₁-C₄)-haloalkoxy or amino; or
represents NR'''R'''',
where R''' and R'''' independently of one another represent hydrogen, (C₁-C₄)-alkyl or (C₂-C₄)-haloalkyl;
X represents hydrogen, halogen, cyano, (C₁-C₄)-alkyl, (C₁-C₄)-haloalkyl or (C₁-C₄)-alkoxy.

2. Compound according to Claim 1, in which
V represents oxygen;
Q represents C-R⁵ or nitrogen, where
R⁵ represents hydrogen, halogen, amino, cyano, nitro, (C₁-C₄)-alkyl, (C₁-C₄)-haloalkyl, (C₃-C₆)-cycloalkyl or (C₁-C₄)-alkoxy;
R³ and R⁶ independently of one another
represent hydrogen or halogen; or
represent (C₁-C₄)-alkyl, (C₁-C₄)-haloalkyl, (C₁-C₄)-alkoxy or (C₁-C₄)-haloalkoxy; or
represent (C₃-C₆) -cycloalkyl or (C₃-C₆)-cycloalkyl-(C₁-C₂)-alkyl, where the radicals mentioned above may optionally be substituted by fluorine, chlorine, bromine, iodine, (C₁-C₃)-alkyl, cyano, nitro, (C₁-C₃)-alkoxy, (C₁-C₃)-haloalkyl or (C₁-C₃)-haloalkoxy; or
represent phenyl or phenyl-(C₁-C₃)-alkyl, where the radicals mentioned above may optionally be mono- or disubstituted by fluorine, chlorine, bromine, iodine, (C₁-C₃)-alkyl, cyano, nitro, (C₁-C₃)-alkoxy, (C₁-C₃)-haloalkyl or (C₁-C₃)-haloalkoxy; or
represent heteroaryl selected from the group consisting of furyl, thienyl, pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, indolyl, isoindolyl and indazolyl, where the radicals mentioned above may optionally be mono- or disubstituted by fluorine, chlorine, bromine, iodine, (C₁-C₃)-alkyl, cyano, nitro, (C₁-C₃)-alkoxy, (C₁-C₃)-haloalkyl or (C₁-C₃)-haloalkoxy; or
represent NR'R",
where R' and R" independently of one another represent hydrogen, (C₁-C₄)-alkyl, (C₁-C₄)-haloalkyl, (C₁-C₄)-cyanoalkyl, (C₁-C₄)-alkoxy-(C₁-C₄)-alkyl, (C₁-C₄)-alkylsulphanyl-(C₁-C₄)-alkyl, (C₁-C₄)-alkylsulphinyl-(C₁-C₄)-alkyl, (C₁-C₄)-alkylsulphonyl-(C₁-C₄)-alkyl, (C₁-C₄)-alkylcarbonyl or (C₁-C₄)-alkoxycarbonyl;
or
where R' and R" together with the nitrogen atom to which they are attached may form a saturated four- to six-membered ring which is optionally mono- or disubstituted by fluorine, chlorine, bromine, iodine, (C₁-C₃)-alkyl, cyano, nitro, (C₁-C₃)-alkoxy, (C₁-C₃)-haloalkyl or (C₁-C₃)-haloalkoxy and optionally interrupted by a heteroatom selected from the group consisting of O, S and N;
W represents hydrogen or fluorine;
n represents the number 0 or 1;
Y represents hydrogen, fluorine, chlorine, bromine, methyl, ethyl, trifluoromethyl, methoxy, ethoxy, difluoromethoxy, trifluoromethoxy, amino, methylamino or dimethylamino;
X represents hydrogen, fluorine, chlorine, bromine, cyano, methyl, ethyl, trifluoromethyl, methoxy or ethoxy.

3. Compound according to Claim 1 or 2, in which
V represents oxygen;
Q represents C-R⁵, where
R⁵ represents hydrogen, methyl, ethyl, methoxy, cyclopropyl or trifluoromethyl;
R³ and R⁶ independently of one another represent hydrogen, fluorine, methyl, ethyl, isopropyl, trifluoromethyl, pentafluoroethyl, difluoromethyl, 1,1-difluoroethyl, difluorochloromethyl, cyclopropyl or phenyl;
W represents hydrogen or fluorine;
n represents the number 0 or 1;
Y represents fluorine, chlorine, bromine, methyl, trifluoromethyl or methoxy;
X represents hydrogen, chlorine, fluorine or methyl;
where X and Y represent in particular the following combinations (Y,X): (Me,F), (Me,H), (Me,Cl), (Me,Me), (Cl,Cl), (Cl,F), (MeO,F), (MeO,H), (Cl,H), (Br,H), (Br,F), (F,F), (CF₃, H), (CF₃, F).

4. Compound according to any of Claims 1 to 3, in which
V represents oxygen;
Q represents C-R⁵, where
R⁵ represents hydrogen or methyl;
R³ represents hydrogen, methyl, ethyl, cyclopropyl, isopropyl, difluoromethyl, trifluoromethyl, pentafluoroethyl, 1,1-difluoroethyl or phenyl;
R⁶ represents hydrogen, fluorine or methyl;
W represents fluorine;
n represents the number 0 or 1;
Y represents bromine, chlorine or methyl;
X represents hydrogen, chlorine, fluorine or methyl;
where X and Y represent in particular the following combinations (Y,X): (Me,H), (Me,Cl), (Me,F), (Me,Me), (Cl,Cl), (Cl,F), (Br,F).

5. Compound according to any of Claims 1 to 4, in which Q represents C-R⁵ and R⁵, R³, R⁶, W, n, Y and X have the meanings defined in Claim 1 or the meanings defined in Claim 2 or the meanings defined in Claim 3 or the meanings defined in Claim 4.

6. Compound of the formula (IIa), 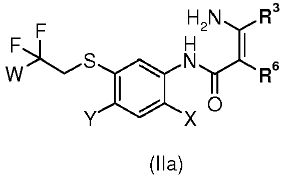 in which R³, R⁶, W, X and Y have the meanings defined in Claim 1 or the meanings defined in Claim 1 or the meanings defined in Claim 2 or the meanings defined in Claim 3 or the meanings defined in Claim 4.

7. Compound of the formula (XXXI), 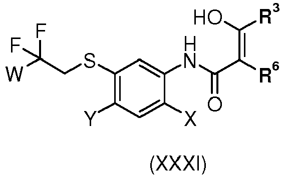 in which R³, R⁶, W, X and Y have the meanings defined in Claim 1 or the meanings defined in Claim 1 or the meanings defined in Claim 2 or the meanings defined in Claim 3 or the meanings defined in Claim 4.

8. Formulation, especially agrochemical formulation, comprising at least one compound of the formula (I) according to any of Claims 1 to 5.

9. Formulation according to Claim 8, further comprising at least one extender and/or at least one surface-active substance.

10. Formulation according to Claim 8 or 9, **characterized in that** the compound of the formula (I) is in a mixture with at least one further active compound.

11. Method for controlling pests, especially animal pests, **characterized in that** a compound of the formula (I) according to any of Claims 1 to 5 or a formulation according to any of Claims 8 to 10 is allowed to act on the pests and/or their habitat, wherein methods for the surgical or therapeutic treatment of the human or animal body and diagnostic methods carried out on the human or animal body are excluded.

12. Method according to Claim 11, **characterized in that** the pest is an animal pest and comprises an insect, an acarid or a nematode, or **in that** the pest is an insect, an acarid or a nematode.

13. Use of a compound of the formula (I) according to any of Claims 1 to 5 or of a formulation according to any of Claims 8 to 10 for controlling animal pests, wherein uses in methods for the surgical or therapeutic treatment of the human or animal body and diagnostic methods carried out on the human or animal body are excluded.

14. Method according to Claim 13, **characterized in that** the animal pest comprises an insect, an acarid or a nematode, or **in that** the animal pest is an insect, an acarid or a nematode.

15. Use according to Claim 13 or 14 in crop protection.

16. Use according to Claim 13 or 14 in the field of animal health.

17. Method for protecting seed or a germinating plant from pests, especially animal pests, comprising a method step in which the seed is contacted with a compound of the formula (I) according to any of Claims 1 to 5 or with a formulation according to any of Claims 8 to 10.

18. Seed obtained by a method according to Claim 17.

## Revendications

1. Composé de formule (I) 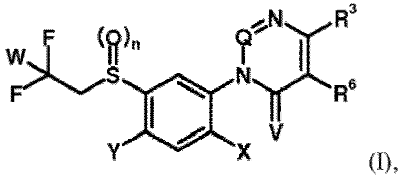 dans laquelle
V représente oxygène,
Q représente C-R⁵ ou azote,
R⁵ représentant hydrogène, halogène, amino, cyano, nitro, alkyle en (C₁-C₆), halogénoalkyle en (C₁-C₆), alkylsulfanyle en (C₁-C₆), alkylsulfinyle en (C₁-C₆), alkylsulfonyle en (C₁-C₆), halogénoalkylsulfanyle en (C₁-C₆), halogénoalkylsulfinyle en (C₁-C₆), halogéno-alkylsulfonyle en (C₁-C₆), cycloalkyle en (C₃-C₆) ou alcoxy en (C₁-C₆) ;
R³ et R⁶ représentent indépendamment l'un de l'autre
hydrogène, halogène, cyano ou nitro ; ou
alkyle en (C₁-C₆), halogénoalkyle en (C₁-C₆), alcényle en (C₂-C₆), alcynyle en (C₂-C₆), alcoxy en (C₁-C₆), halogénoalcoxy en (C₁-C₆), halogénoalkylsulfanyle en (C₁-C₆), halogénoalkylsulfinyle en (C₁-C₆), halogéno-alkylsulfonyle en (C₁-C₆), alkylsulfanyle en (C₁-C₆), alkylsulfinyle en (C₁-C₆), alkylsulfonyle en (C₁-C₆), alkylcarbonyle en (C₁-C₅), halogénoalkylcarbonyle en (C₁-C₅) ou alcoxycarbonyle en (C₂-C₆) ; ou
cycloalkyle en (C₃-C₆), cycloalkyle en (C₃-C₆)-alkyle en (C₁-C₃) ou cycloalcényle en (C₃-C₆), les radicaux susmentionnés pouvant éventuellement être substitués par halogène, alkyle, cyano, nitro, alcoxy, halogénoalkyle ou halogénoalcoxy ; ou
phényle, hétéroaryle, phényl-alkyle en (C₁-C₃) ou hétéroaryl-alkyle en (C₁-C₃), les radicaux susmentionnés pouvant éventuellement être substitués par halogène, alkyle, cyano, nitro, alcoxy, halogénoalkyle ou halogénoalcoxy ; ou
NR'R",
R' et R" représentant indépendamment l'un de l'autre hydrogène, cyano, alkyle en (C₁-C₄), halogénoalkyle en (C₁-C₄), cyanoalkyle en (C₁-C₄), hydroxyalkyle en (C₁-C₄), alcoxy en (C₁-C₄)-alkyle en (C₁-C₄), alkylsulfanyle en (C₁-C₄)-alkyle en (C₁-C₄), alkylsulfinyle en (C₁-C₄) -alkyle en (C₁-C₄), alkylsulfonyle en (C₁-C₄)-alkyle en (C₁-C₄), alcényle en (C₃-C₄), halogénoalcényle en (C₃-C₄), cyanoalcényle en (C₂-C₄), alcynyle en (C₃-C₄), halogénoalcynyle en (C₃-C₄), cyanoalcynyle en (C₃-C₄), alkylcarbonyle en (C₁-C₄) ou alcoxycarbonyle en (C₁-C₄) ; ou
R' et R" pouvant former ensemble avec l'atome N auquel ils sont reliés un cycle saturé ou insaturé de trois à huit chaînons, éventuellement substitué par halogène, alkyle, cyano, nitro, alcoxy, halogénoalkyle ou halogénoalcoxy, et éventuellement interrompu par un ou plusieurs hétéroatomes, qui sont choisis indépendamment les uns des autres dans le groupe constitué par 0, S et N, à condition que deux atomes d'oxygène ne soient pas directement voisins ;
W représente hydrogène ou halogène ;
n représente le nombre 0 ou 1 ;
Y représente hydrogène, halogène, alkyle en (C₁-C₄), trifluorométhyle, halogénoalkyle en (C₂-C₄), alcoxy en (C₁-C₄), halogénoalcoxy en (C₁-C₄) ou amino ; ou
NR'''R'''',
R''' et R'''' représentant indépendamment l'un de l'autre hydrogène, alkyle en (C₁-C₄) ou halogénoalkyle en (C₂-C₄) ; X représente hydrogène, halogène, cyano, alkyle en (C₁-C₄), halogénoalkyle en (C₁-C₄) ou alcoxy en (C₁-C₄) .

2. Composé selon la revendication 1, dans lequel
V représente oxygène,
Q représente C-R⁵ ou azote,
R⁵ représentant hydrogène, halogène, amino, cyano, nitro, alkyle en (C₁-C₄), halogénoalkyle en (C₁-C₄), cycloalkyle en (C₃-C₆) ou alcoxy en (C₁-C₄) ;
R³ et R⁶ représentent indépendamment l'un de l'autre
hydrogène ou halogène ; ou
alkyle en (C₁-C₄), halogénoalkyle en (C₁-C₄), alcoxy en (C₁-C₄), halogénoalcoxy en (C₁-C₄) ; ou
cycloalkyle en (C₃-C₆) ou cycloalkyle en (C₃-C₆)-alkyle en (C₁-C₂), les radicaux susmentionnés pouvant éventuellement être substitués par fluor, chlore, brome, iode, alkyle en (C₁-C₃), cyano, nitro, alcoxy en (C₁-C₃), halogénoalkyle en (C₁-C₃) ou halogénoalcoxy en (C₁-C₃) ; ou
phényle ou phényl-alkyle en (C₁-C₃), les radicaux susmentionnés pouvant éventuellement être substitués une fois ou deux fois par fluor, chlore, brome, iode, alkyle en (C₁-C₃), cyano, nitro, alcoxy en (C₁-C₃), halogénoalkyle en (C₁-C₃) ou halogénoalcoxy en (C₁-C₃) ; ou
hétéroaryle choisi dans le groupe constitué par furyle, thiényle, pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, indolyle, isoindolyle et indazolyle, les radicaux susmentionnés pouvant éventuellement être substitués une fois à deux fois par fluor, chlore, brome, iode, alkyle en (C₁-C₃), cyano, nitro, alcoxy en (C₁-C₃), halogénoalkyle en (C₁-C₃) ou halogénoalcoxy en (C₁-C₃) ; ou
NR' R",
R' et R" représentant indépendamment l'un de l'autre hydrogène, alkyle en (C₁-C₄), halogénoalkyle en (C₁-C₄), cyanoalkyle en (C₁-C₄), alcoxy en (C₁-C₄) -alkyle en (C₁-C₄), alkylsulfanyle en (C₁-C₄)-alkyle en (C₁-C₄), alkylsulfinyle en (C₁-C₄)-alkyle en (C₁-C₄), alkylsulfonyle en (C₁-C₄)-alkyle en (C₁-C₄), alkylcarbonyle en (C₁-C₄) ou alcoxycarbonyle en (C₁-C₄) ; ou
R' et R" pouvant former ensemble avec l'atome N auquel ils sont reliés un cycle saturé de quatre à six chaînons, éventuellement substitué une fois à deux fois par fluor, chlore, brome, iode, alkyle en (C₁-C₃), cyano, nitro, alcoxy en (C₁-C₃), halogénoalkyle en (C₁-C₃) ou halogénoalcoxy en (C₁-C₃), et éventuellement interrompu par un hétéroatome, qui est choisi dans le groupe constitué par 0, S et N ;
W représente hydrogène ou fluor ;
n représente le nombre 0 ou 1 ;
Y représente hydrogène, fluor, chlore, brome, méthyle, éthyle, trifluorométhyle, méthoxy, éthoxy, difluorométhoxy, trifluorométhoxy, amino, méthylamino ou diméthylamino ;
X représente hydrogène, fluor, chlore, brome, cyano, méthyle, éthyle, trifluorométhyle, méthoxy ou éthoxy.

3. Composé selon la revendication 1 ou 2, dans lequel
V représente oxygène,
Q représente C-R⁵,
R⁵ représentant hydrogène, méthyle, éthyle, méthoxy, cyclopropyle ou trifluorométhyle ;
R³ et R⁶ représentent indépendamment l'un de l'autre hydrogène, fluor, méthyle, éthyle, isopropyle, trifluorométhyle, pentafluoroéthyle, difluorométhyle, 1,1-difluoroéthyle, difluorochlorométhyle, cyclopropyle ou phényle ;
W représente hydrogène ou fluor ;
n représente le nombre 0 ou 1 ;
Y représente fluor, chlore, brome, méthyle, trifluorométhyle ou méthoxy ;
X représente hydrogène, chlore, fluor ou méthyle ; X et Y représentant notamment les combinaisons (Y,
X) suivantes : (Me, F), (Me, H), (Me, Cl), (Me, Me), (Cl, Cl), (Cl, F), (MeO, F), (MeO, H), (Cl, H), (Br, H), (Br, F), (F, F), (CF₃, H), (CF₃, F).

4. Composé selon l'une quelconque des revendications 1 à 3, dans lequel
V représente oxygène,
Q représente C-R⁵,
R⁵ représentant hydrogène ou méthyle ;
R³ représente hydrogène, méthyle, éthyle, cyclopropyle, isopropyle, difluorométhyle, trifluorométhyle, pentafluoroéthyle, 1,1-difluoroéthyle ou phényle ;
R⁶ représente hydrogène, fluor ou méthyle ;
W représente fluor ;
N représente le nombre 0 ou 1 ;
Y représente brome, chlore ou méthyle ;
X représente hydrogène, chlore, fluor ou méthyle ; X et Y représentant notamment les combinaisons (Y,
X) suivantes : (Me, H), (Me, Cl), (Me, F), (Me, Me), (Cl, Cl), (Cl, F), (Br, F).

5. Composé selon l'une quelconque des revendications 1 à 4, dans lequel Q représente C-R⁵, et R⁵, R³, R⁶, W, n, Y et X ont les significations définies dans la revendication 1 ou les significations définies dans la revendication 2 ou les significations définies dans la revendication 3 ou les significations définies dans la revendication 4.

6. Composé de formule (IIa) 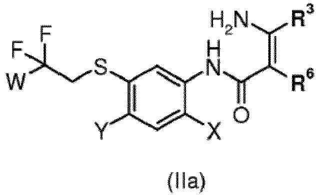 dans laquelle R³, R⁶, W, X et Y ont les significations définies dans la revendication 1 ou les significations définies dans la revendication les significations définies dans la revendication 2 ou les significations définies dans la revendication 3 ou les significations définies dans la revendication 4.

7. Composé de formule (XXXI) 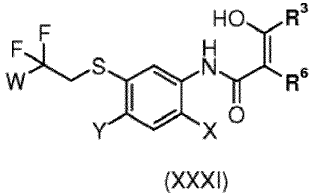 dans laquelle R³, R⁶, W, X et Y ont les significations définies dans la revendication 1 ou les significations définies dans la revendication les significations définies dans la revendication 2 ou les significations définies dans la revendication 3 ou les significations définies dans la revendication 4.

8. Formulation, notamment formulation agrochimique, comprenant au moins un composé de formule (I) selon l'une quelconque des revendications 1 à 5.

9. Formulation selon la revendication 8, comprenant en outre au moins un extendeur et/ou au moins une substance tensioactive.

10. Formulation selon la revendication 8 ou 9, **caractérisée en ce que** le composé de formule (I) se présente en mélange avec au moins un autre agent actif.

11. Procédé de lutte contre des nuisibles, notamment des animaux nuisibles, **caractérisé en ce qu'**un composé de formule (I) selon l'une quelconque des revendications 1 à 5 ou une formulation selon l'une quelconque des revendications 8 à 10 est laissé agir sur les nuisibles et/ou leur habitat, les procédés de traitement chirurgical ou thérapeutique du corps humain ou animal et les procédés de diagnostic qui sont réalisés sur le corps humain ou animal étant exclus.

12. Procédé selon la revendication 11, **caractérisé en ce que** le nuisible est un animal nuisible, et comprend un insecte, un acarien ou un nématode, ou **en ce que** le nuisible est un insecte, un acarien ou un nématode.

13. Utilisation d'un composé de formule (I) selon l'une quelconque des revendications 1 à 5 ou d'une formulation selon l'une quelconque des revendications 8 à 10 pour lutter contre des animaux nuisibles, les utilisations dans des procédés de traitement chirurgical ou thérapeutique du corps humain ou animal et des procédés de diagnostic qui sont réalisés sur le corps humain ou animal étant exclues.

14. Utilisation selon la revendication 13, **caractérisée en ce que** l'animal nuisible comprend un insecte, un acarien ou un nématode, ou **en ce que** l'animal nuisible est un insecte, un acarien ou un nématode.

15. Utilisation selon la revendication 13 ou 14, dans la protection des plantes.

16. Utilisation selon la revendication 13 ou 14, dans le domaine de la santé animale.

17. Procédé de protection d'une graine ou d'une plante germée contre des nuisibles, notamment des animaux nuisibles, comprenant une étape de procédé lors de laquelle la graine est mise en contact avec un composé de formule (I) selon l'une quelconque des revendications 1 à 5 ou une formulation selon l'une quelconque des revendications 8 à 10.

18. Graine, obtenue par un procédé selon la revendication 17.
